(19) Europäisches Patentamt
European Patent Office
Office européen des brevets

(11) **EP 4 681 729 A1**

(12) **EUROPEAN PATENT APPLICATION**
published in accordance with Art. 153(4) EPC

(43) Date of publication:
**21.01.2026  Bulletin 2026/04**

(21) Application number: **24770021.4**

(22) Date of filing: **15.03.2024**

(51) International Patent Classification (IPC):
*A61K 39/395* (2006.01)     *C07K 16/28* (2006.01)
*C07K 19/00* (2006.01)     *C12N 15/13* (2006.01)
*C12N 15/62* (2006.01)     *A61P 35/00* (2006.01)

(52) Cooperative Patent Classification (CPC):
**A61K 39/395; A61P 35/00; C07K 16/00;
C07K 16/28; C07K 19/00; C12N 15/62;** Y02A 50/30

(86) International application number:
**PCT/CN2024/081815**

(87) International publication number:
**WO 2024/188331 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **15.03.2023   CN 202310250609**

(71) Applicant: **Shanghai Mabgen Biotech Ltd.
Shanghai 201206 (CN)**

(72) Inventors:
 • **YAN, Yijie
   Shanghai 201206 (CN)**
 • **WU, Hui
   Shanghai 201206 (CN)**
 • **ZHAO, Zhilong
   Shanghai 201206 (CN)**
 • **LI, Lei
   Shanghai 201206 (CN)**

(74) Representative: **Dragotti & Associati S.P.A.
Via Nino Bixio, 7
20129 Milano (IT)**

Remarks:
The complete document including Reference
Table(s) and the Sequence Listing(s) can be
downloaded from the EPO website

(54) **PHARMACEUTICAL COMPOSITION CONTAINING PD-1/PVRIG/TIGIT BINDING PROTEIN AND MEDICAL USE THEREOF**

(57)     The present disclosure relates to a pharmaceutical composition containing a PD-1/PVRIG/TIGIT binding protein and a medical use thereof. Specifically, the pharmaceutical composition involved in the present disclosure contains a PD-1/PVRIG/TIGIT binding protein and a buffering agent. The pharmaceutical composition of the present disclosure has good biological activity and stability.

EP 4 681 729 A1

## Description

The present application claims priority to Chinese Patent Application No. 202310250609.3 filed on March 15, 2023.

## TECHNICAL FIELD

The present disclosure relates to the field of pharmaceutical formulations, in particular to a pharmaceutical composition comprising a PD-1/PVRIG/TIGIT-binding protein and pharmaceutical use thereof.

## BACKGROUND

Immunotherapy is a popular topic in the area of cancer treatment. Anti-PD-1 antibodies, as representative immunotherapy drugs, have been clinically shown to be highly effective and highly safe.

PD-1 (programmed cell death-1), an immunosuppressive receptor, belongs to the CD28 receptor family (Riley et al., 2009, Immunol. Rev. 29:114-25). This family also includes CD28, CTLA-4, ICOS, PD-1, and BTLA. PD-1 is a type I transmembrane protein and is structurally similar to CTLA-4, but PD-1 lacks the MYPPPY sequence that binds to B7-1 and B7-2. PD-1 is expressed mainly by activated B cells, T cells, and bone marrow cells (Chen et al., 2013, Nat. Rev. Immunol. 13:227-42).

There are two cell surface glycoprotein ligands for PD-1, which are PD ligand 1 (PD-L1, also known as CD274, B7-H1) and PD ligand 2 (PD-L2, also known as B7-DC). Neither PD-L1 nor PD-L2 binds to other members of the CD28 receptor family. PD-L1 is widely expressed by lymphocytes (e.g., $CD4^+$ T cells, $CD8^+$ T cells, and macrophages), peripheral tissues, various tumor cells, virus-infected cells, etc. PD-L2 is expressed mainly by activated dendritic cells and macrophages (Dong et al., 1999, Nat. Med. 5:1365-9). Upon binding to its ligand PD-L1 or PD-L2, PD-1 down-regulates T cell function, including reducing T cell activation, differentiation, proliferation, cytokine secretion, etc.

PVRIG (poliovirus receptor-related Ig domain containing protein), also known as CD112R, belongs to the B7/CD28 superfamily as TIGIT (T cell immunoglobulin and ITIM domain), CD96, and CD226 do and plays an important role in the immune system. When PVRIG's ligand PVRL2 (also known as CD112) binds to PVRIG, it activates the ITIM domain in the intracellular region of PVRIG, causing PVRIG to play an immunosuppressive role. PVRIG is expressed mainly on the surfaces of $CD4^+$ T cells, $CD8^+$ T cells, and NK cells. PVRIG and its ligand PVRL2 are highly expressed in many solid tumors, including lung cancer, breast cancer, ovarian cancer, renal cancer, gastric cancer, endometrial cancer, head and neck cancer, and the like. The expression of PVRIG in these cancers is highly correlated with TIGIT and PD-1. Similar to PD-1 and TIGIT, PVRIG-positive T cells are also Eomes-positive and Tbet-negative, indicating that PVRIG is associated with T cell depletion. Thus, PVRIG may represent a new immune checkpoint other than PD-1 and TIGIT and plays the role of redundancy. *In vitro* cell experiments and mouse model experiments show that knocking out or inhibiting mouse PVRIG can effectively inhibit cancer growth and act synergistically with PD-1 and TIGIT inhibitors. TIGIT is highly expressed on lymphocytes, including tumor-infiltrating lymphocytes (TILs) and Tregs that infiltrate different types of cancer. TIGIT binding to its cognate ligand PVR (also known as CD155) has been shown to directly suppress the cytotoxicity of NK cells through its cytoplasmic ITIM domain. PVR is also widely expressed in cancer, suggesting that the TIGIT-PVR signaling axis may be a major immune escape mechanism in cancer. The inhibiting receptors TIGIT and PVRIG bind to the ligands CD155 and CD112 as the activating receptor DNAM-1 does, but the affinities of the inhibiting receptors are higher (Y. Zhu et al., 2016, J Exp Med. 213:167-176). In patients with tumors, DNAM-1 expression is down-regulated in isolated NK cells, making NK cells more susceptible to inhibition by TIGIT or PVRIG. Blocking the binding of TIGIT and PVRIG can activate NK cells' cell killing function (L. Martinet et al., 2015, Cell Reports. 11:85-97).

PD-1/PD-L1 antibodies are currently the most clinically successful immune checkpoint inhibitor mAbs; however, up-regulation of the expression of other immune checkpoints on the surfaces of T cells can limit their efficacy. TIGIT and PVRIG also regulate NK cell activity in addition to T cell function, suggesting a possible synergy with the function of PD-1/PD-L1 in NK cells. There are reports that the high expression of CD155 and CD112, the ligands for TIGIT and PVRIG, in lung cancer, ovarian cancer, colorectal cancer, and melanoma is significantly correlated with poor prognoses following PD-1/PD-L1 treatment. For example, patients with high CD155 expression respond poorly to anti-PD-1 antibodies, whereas patients with low CD155 expression respond well to PD-1 (S. Whelan et al., 2019, Cancer Immunol Res. 7:257-268; A. Lepletier et al., 2020, Clin Cancer Res. 26:3671-3681).

There has not been any PVRIG antibody or anti-PVRIG/TIGIT bispecific antibody drug on the market. COM701 of Compugen is the world's first anti-PVRIG humanized antibody approved by the FDA to enter clinical trials, and it is currently in phase I clinical trials for treating cancer. Surface Oncology is also developing an anti-PVRIG antibody, which is SRF-813. Anti-TIGIT antibodies include tiragolumab of Genentech, BMS-986207 developed by Ono Pharmaceutical in cooperation with BMS, MK-7684 of MSD, EOS-884448 of iTeos Therapeutics, and AB-154 of Arcus Biosciences, all of which are in phase II clinical trials.

WO2023040945 provides an anti-PD-1 antibody of a new structure and a related anti-PD-1/PVRIG/TIGIT trispecific antibody, which are expected to enhance T cell and NK cell activation, overcome the drug resistance of PD-1/PD-L1, expand the population of responsive patients, and thereby improve the effect of cancer immunotherapy. Moreover, the anti-PD-1/PVRIG/TIGIT trispecific antibody can bind to multiple immune checkpoints on the same cell surface simultaneously and has a potential avidity effect, and therefore has the potential to provide better clinical efficacy than the existing PD-1, TIGIT, and PVRIG antibody drugs or their combinations.

Due to their great molecular weights and complex structures, antibody drugs are prone to degradation, aggregation, unwanted chemical modifications, etc., which make them unstable. It is particularly important to develop stable formulations of antibody drugs that make the antibodies more suitable for administration, keep the antibodies stable during storage and subsequent use, and make the antibodies produce better effects. For a novel PD-1/PVRIG/TIGIT-binding protein, there is still a need to develop a suitable pharmaceutical composition.

SUMMARY

The present disclosure provides a pharmaceutical composition comprising a PD-1/PVRIG/TIGIT-binding protein, and the composition has excellent stability.

The present disclosure provides a pharmaceutical composition comprising a PD-1/PVRIG/TIGIT-binding protein and a buffer, wherein the buffer is selected from the group consisting of a citrate buffer and a histidine salt buffer. In some embodiments, the buffer is a citric acid-sodium citrate buffer. In some embodiments, the buffer is a citric acid-disodium citrate buffer. In some embodiments, the buffer is a histidine salt buffer. In some embodiments, the buffer is a histidine-hydrochloride buffer. In some embodiments, the buffer is a histidine-acetate buffer. In some embodiments, the buffer is a histidine-histidine hydrochloride buffer. In some embodiments, the buffer is a histidine-acetic acid buffer.

In some embodiments, the PD-1/PVRIG/TIGIT-binding protein comprises a first antigen-binding domain that specifically binds to PD-1, a second antigen-binding domain that specifically binds to PVRIG, and a third antigen-binding domain that specifically binds to TIGIT, which is capable of specifically binding to PD-1, PVRIG, and TIGIT simultaneously or separately.

In some embodiments, the aforementioned PD-1/PVRIG/TIGIT-binding protein comprises or is an anti-PD-1/PVRIG/TIGIT trispecific antibody.

In some embodiments, each of the antigen-binding domains in the aforementioned PD-1/PVRIG/TIGIT-binding protein is selected from the group consisting of a Fab, an Fv, an sFv, a Fab', a F(ab')$_2$, a linear antibody, a single-chain antibody, an scFv, an sdAb, an sdFv, a nanobody, a peptibody, a domain antibody, and any combination thereof. In a specific embodiment, the first antigen-binding domain that specifically binds to PD-1 comprises or is a VHH, the second antigen-binding domain that specifically binds to PVRIG comprises or is a VHH, and the third antigen-binding domain that specifically binds to TIGIT is a Fab or F(ab')$_2$.

In some embodiments, the aforementioned PD-1/PVRIG/TIGIT-binding protein is modified by humanization, affinity maturation, T cell epitope removal, reduction of antibody deamidation, and/or reduction of antibody isomerization.

In some embodiments, the aforementioned PD-1/PVRIG/TIGIT-binding protein is a recombinant antibody, a camelid antibody, a chimeric antibody, a humanized antibody, a fully human antibody, or an antigen-binding fragment thereof.

In some embodiments, in the aforementioned PD-1/PVRIG/TIGIT-binding protein, the first antigen-binding domain that specifically binds to PD-1 comprises an (at least one) immunoglobulin single variable domain, and the immunoglobulin single variable domain comprises:

a CDR1, a CDR2, and a CDR3 from the amino acid sequence set forth in any one of SEQ ID NOs: 2, 8-10, and 11-29, wherein the CDR1, CDR2, and CDR3 are defined according to the Kabat, IMGT, Chothia, AbM, or Contact numbering scheme.

In some embodiments, according to the Kabat numbering scheme, the amino acid sequences of the CDR1, CDR2, and CDR3 of the immunoglobulin single variable domain are set forth in: SEQ ID NOs: 3, 30, and 31, respectively.

In some specific embodiments, the amino acid sequence of the CDR1 of the immunoglobulin single variable domain is set forth in SEQ ID NO: 3, the amino acid sequence of the CDR2 is set forth in any one of SEQ ID NOs: 4, 32-34, and 79, and the amino acid sequence of the CDR3 is set forth in any one of SEQ ID NOs: 5, 35, and 36. In some specific embodiments, the amino acid sequences of the CDR1, CDR2, and CDR3 of the immunoglobulin single variable domain are set forth in SEQ ID NOs: 3, 32, and 35, respectively.

In some embodiments, in the aforementioned PD-1/PVRIG/TIGIT-binding protein, the second antigen-binding domain that specifically binds to PVRIG comprises an (at least one) immunoglobulin single variable domain, and the immunoglobulin single variable domain comprises:

a CDR1, a CDR2, and a CDR3 from the amino acid sequence set forth in any one of SEQ ID NOs: 38, 46-50, and 74-75.

In some specific embodiments, according to the Kabat numbering scheme, the amino acid sequences of the CDR1, CDR2, and CDR3 of the immunoglobulin single variable domain are set forth in SEQ ID NOs: 39, 40, and 41, respectively.

In some embodiments, in the aforementioned PD-1/PVRIG/TIGIT-binding protein, the third antigen-binding domain that specifically binds to TIGIT comprises a heavy chain variable region (VH) and a light chain variable region (VL), wherein:

the heavy chain variable region comprises a HCDR1, a HCDR2, and a HCDR3, and the amino acid sequences of the HCDR1, HCDR2, and HCDR3 are set forth in SEQ ID NOs: 57, 58, and 59, respectively; the light chain variable region comprises a LCDR1, a LCDR2, and a LCDR3, and the amino acid sequences of LCDR1, LCDR2, and LCDR3 are set forth in SEQ ID NOs: 60, 61, and 62, respectively.

In some embodiments, in the aforementioned PD-1/PVRIG/TIGIT-binding protein, the first antigen-binding domain that specifically binds to PD-1 comprises:

the amino acid sequence set forth in any one of SEQ ID NOs: 2, 8-10, and 11-29 or an amino acid sequence having at least 80% or at least 90% sequence identity thereto.

In some embodiments, in the aforementioned PD-1/PVRIG/TIGIT-binding protein, the second antigen-binding domain that specifically binds to PVRIG comprises the amino acid sequence set forth in any one of SEQ ID NOs: 38, 46-50, and 74-75 or an amino acid sequence having at least 80% or at least 90% sequence identity thereto.

In some embodiments, in the aforementioned PD-1/PVRIG/TIGIT-binding protein, the third antigen-binding domain that specifically binds to TIGIT comprises a heavy chain variable region (VH) and a light chain variable region (VL), wherein:

the heavy chain variable region comprises the amino acid sequence set forth in any one of SEQ ID NOs: 63-65 or an amino acid sequence having at least 80% or at least 90% identity thereto;

the light chain variable region comprises the amino acid sequence set forth in SEQ ID NO: 66 or 67 or an amino acid sequence having at least 80% or at least 90% identity thereto.

In some embodiments, in the aforementioned PD-1/PVRIG/TIGIT-binding protein, the third antigen-binding domain that specifically binds to TIGIT comprises a heavy chain variable region (VH) and a light chain variable region (VL), wherein:

the heavy chain variable region comprises the amino acid sequence set forth in SEQ ID NO: 63 or an amino acid sequence having at least 80% or at least 90% identity thereto;

the light chain variable region comprises the amino acid sequence set forth in SEQ ID NO: 67 or an amino acid sequence having at least 80% or at least 90% identity thereto.

In some specific embodiments, the third antigen-binding domain that specifically binds to TIGIT comprises a full-length heavy chain (HC) and a full-length light chain (LC).

For example, the full-length heavy chain is of the IgG1, IgG2, or IgG4 isotype, and the full-length light chain is of the Kappa isotype;

For example, the heavy chain sequence is the amino acid sequence set forth in SEQ ID NO: 51 or has at least 80% or at least 90% sequence identity thereto, and the light chain sequence is the amino acid sequence set forth in SEQ ID NO: 52 or has at least 80% or at least 90% sequence identity thereto.

In some embodiments, in the aforementioned PD-1/PVRIG/TIGIT-binding protein, the first antigen-binding domain that specifically binds to PD-1, the second antigen-binding domain that specifically binds to PVRIG, and the third antigen-binding domain that specifically binds to TIGIT are linked, either directly or by a linker. For example, the linker is an amino acid sequence represented by $(G_4S)_x$, wherein x is independently selected from the group consisting of integers of 1-20; for example, the linker is an amino acid sequence represented by $(G_4S)_2$, $(G_4S)_3$, or $(G_4S)_4$.

In some embodiments, in the aforementioned PD-1/PVRIG/TIGIT-binding protein, the antigen-binding domain that specifically binds to PD-1 is located at the N-terminus or C-terminus of the antigen-binding domain that specifically binds to PVRIG.

In some embodiments, in the aforementioned PD-1/PVRIG/TIGIT-binding protein, the antigen-binding domain that specifically binds to PD-1 is located at the N-terminus or C-terminus of the antigen-binding domain that specifically binds to TIGIT.

In some embodiments, in the aforementioned PD-1/PVRIG/TIGIT-binding protein, the antigen-binding domain that specifically binds to PVRIG is located at the N-terminus or C-terminus of the antigen-binding domain that specifically binds to TIGIT.

In some embodiments, the first antigen-binding domain, the second antigen-binding domain, and the third antigen-binding domain are on the same polypeptide chain or not. In some embodiments, the aforementioned PD-1/PVRIG/TIGIT-binding protein comprises a first polypeptide chain and a second polypeptide chain represented by structures selected from the group consisting of the following:

(I) a first polypeptide chain: [the first antigen-binding domain that specifically binds to PD-1]-[linker 1]a-[the second antigen-binding domain that specifically binds to PVRIG]-[linker 2]b-[the VH of the third antigen-binding domain that specifically binds to TIGIT]-CH1-Fc, and a second polypeptide chain: [the VL of the third antigen-binding domain that specifically binds to TIGIT]-Cκ; or

(II) a first polypeptide chain: [the second antigen-binding domain that specifically binds to PVRIG]-[linker 2]b-[the VH of the third antigen-binding domain that specifically binds to TIGIT]-CH1-Fc, and a second polypeptide chain: [the first antigen-binding domain that specifically binds to PD-1]-[linker 3]c-[the VL of the third antigen-binding domain that specifically binds to TIGIT]-Cκ; or

(III) a first polypeptide chain: [the second antigen-binding domain that specifically binds to PVRIG]-[linker 2]b-[the VH of the third antigen-binding domain that specifically binds to TIGIT]-CH1-Fc-[linker 4]d-[the first antigen-binding domain that specifically binds to PD-1], and a second polypeptide chain: [the VL of the third antigen-binding domain that specifically binds to TIGIT]-Cκ; or

(IV) a first polypeptide chain: [the second antigen-binding domain that specifically binds to PVRIG]-[linker 2]b-[the VH of the third antigen-binding domain that specifically binds to TIGIT]-CH1-Fc, and a second polypeptide chain: [the VL of the third antigen-binding domain that specifically binds to TIGIT]-Cκ-[linker 5]e-[the first antigen-binding domain that specifically binds to PD-1].

The above first and second polypeptide chains are shown in order from N-terminus to C-terminus.

- represents a peptide bond; the linkers are polypeptides capable of fulfilling the function of linking; linker 1, linker 2, linker 3, linker 4, and linker 5 may be identical or different; a. b, c, d, and e may optionally independently be 0 or 1.

For example, each of the linkers is independently a linker of EPKSS or $(G_xS)_y$, wherein x is selected from the group consisting of integers of 1-5 (e.g., 1, 2, 3, 4, or 5), and y is selected from the group consisting of integers of 1-6 (e.g., 1, 2, 3, 4, 5, or 6).

When the linker is a linker of $(G_xS)_y$, it is a linker represented by, for example, any one of $(G_4S)_2$, $(G_4S)_3$, and $(G_4S)_4$.

In some embodiments, provided is a PD-1/PVRIG/TIGIT-binding protein, comprising a first polypeptide chain and a second polypeptide chain, wherein:

the first and second polypeptide chains comprise the amino acid sequences set forth in SEQ ID NOs: 70 and 52, respectively;
the first and second polypeptide chains comprise the amino acid sequences set forth in SEQ ID NOs: 68 and 71, respectively;
the first and second polypeptide chains comprise the amino acid sequences set forth in SEQ ID NOs: 72 and 52, respectively;
the first and second polypeptide chains comprise the amino acid sequences set forth in SEQ ID NOs: 68 and 73, respectively;
the first and second polypeptide chains comprise the amino acid sequences set forth in SEQ ID NOs: 76 and 52, respectively;
the first and second polypeptide chains comprise the amino acid sequences set forth in SEQ ID NOs: 77 and 52, respectively;
or a combination of amino acid sequences having at least 80% or at least 90% sequence identity to any one of the aforementioned first and second polypeptide chains.

In some embodiments, the aforementioned PD-1/PVRIG/TIGIT-binding protein of the present disclosure comprises two identical or different first polypeptide chains and two identical or different second polypeptide chains.

In some specific embodiments, the PD-1/PVRIG/TIGIT-binding protein comprises two identical first polypeptide chains and two identical second polypeptide chains.

In some embodiments, comprising the antigen-binding domain of the present disclosure that specifically binds to PD-1, the PD-1/PVRIG/TIGIT-binding protein of the present disclosure has all or any of its properties and functions.

Comprising the PVRIG/TIGIT-binding domains, the PD-1/PVRIG/TIGIT-binding protein further has at least one of the following characteristics:

(a) binding to human PVRIG with a $K_D$ value of less than $1 \times 10^{-7}$ M;

(b) blocking the interaction of PVRIG with its ligand (e.g., PVRL2);

(c) eliminating the inhibition of T cells by dendritic cells (DCs) and activating T cells; and

(d) eliminating the inhibition of NK cells by tumor cells.

In some embodiments, the aforementioned PD-1/PVRIG/TIGIT-binding protein of the present disclosure is capable of inhibiting tumor growth by at least about 10%, e.g., at least about 20%, about 30%, about 40%, about 50%, about 60%, about 70%, about 80%, or about 90%.

In some embodiments, the aforementioned PD-1/PVRIG/TIGIT-binding protein of the present disclosure encompasses a variant, wherein the variant comprises one or more (e.g., 1, 2, 3, 4, 5, 6, 7, 8, 9, or 10) amino acid mutations as compared to any one of the combinations of first and second polypeptide chains; the amino acid mutations may be conservative replacements, substitutions, or modifications, and/or deletions or additions that do not affect function. The mutations may occur in the antigen-binding domain that specifically binds to PD-1, the antigen-binding domain that specifically binds to PVRIG, or the antigen-binding domain that specifically binds to TIGIT (e.g., the CDRs and/or FRs).

In some embodiments, provided is a PD-1/PVRIG/TIGIT-binding protein that binds to or competes for binding to the same PD-1, PVRIG, and/or TIGIT or epitopes thereof with the aforementioned PD-1/PVRIG/TIGIT-binding protein of the present disclosure; or blocks the binding of the aforementioned PD-1/PVRIG/TIGIT-binding protein of the present disclosure to PD-1, PVRIG, and/or TIGIT; or whose binding to PD-1, PVRIG, and/or TIGIT is blocked by the aforementioned PD-1/PVRIG/TIGIT-binding protein of the present disclosure.

In the present disclosure, when referring to a PVRIG-binding protein or an antigen-binding domain that specifically binds to PVRIG, it may comprise or be selected from the group consisting of PVRIG antibodies from WO2016134333, WO2016134335, WO2018033798, WO2018220446, WO2019079777, WO2019232484, WO2020018879, WO2021021837, WO2021097294, WO2021091605, and WO2021113831. For example, the PVRIG antibody may be any one of CPA.7.002, CPA.7.005, CPA.7.021, and CPA.7.050 (see WO2016134333). The above patents are incorporated herein by reference in their entirety.

In the present disclosure, when referring to a TIGIT-binding protein or an antigen-binding domain that specifically binds to TIGIT, it may comprise or be selected from the group consisting of TIGIT antibodies from WO2019062832, WO2009126688, WO2014089113, WO2015009856, WO2015143343, WO2015174439, WO2016028656, WO2016106302, WO2017053748, WO2017030823, US20160176963, US20130251720, WO2019232484, and WO2019062832. For example, the TIGIT antibody may be any one of CPA.9.083.H4(S241P), CPA.9.086.H4(S241P), CHA.9.547.7.H4(S241P), and CHA.9.547.13.H4(S241P) (see WO2019232484). The above patents are incorporated herein by reference in their entirety.

In some embodiments, the PD-1/PVRIG/TIGIT-binding protein in the pharmaceutical composition is at a concentration of 0.01 mg/mL-500 mg/mL, e.g., 0.05 mg/mL-450 mg/mL, 0.05 mg/mL-400 mg/mL, 0.05 mg/mL-350 mg/mL, 0.05 mg/mL-300 mg/mL, 0.05 mg/mL-250 mg/mL, 0.05 mg/mL-200 mg/mL, 0.05 mg/mL-150 mg/mL, 0.05 mg/mL-140 mg/mL, 0.05 mg/mL-130 mg/mL, 0.05 mg/mL-120 mg/mL, 0.05 mg/mL-110 mg/mL, 0.05 mg/mL-100 mg/mL, 0.1 mg/mL-400 mg/mL, 0.1 mg/mL-350 mg/mL, 0.1 mg/mL-300 mg/mL, 0.1 mg/mL-250 mg/mL, 0.1 mg/mL-200 mg/mL, 0.1 mg/mL-150 mg/mL, 0.1 mg/mL-140 mg/mL, 0.1 mg/mL-130 mg/mL, 0.1 mg/mL-120 mg/mL, 0.1 mg/mL-110 mg/mL, 0.1 mg/mL-100 mg/mL, 0.5 mg/mL-350 mg/mL, 0.5 mg/mL-300 mg/mL, 0.5 mg/mL-250 mg/mL, 0.5 mg/mL-200 mg/mL, 0.5 mg/mL-150 mg/mL, 0.5 mg/mL-140 mg/mL, 0.5 mg/mL-130 mg/mL, 0.5 mg/mL-120 mg/mL, 0.5 mg/mL-110 mg/mL, 0.5 mg/mL-100 mg/mL, 1 mg/mL-300 mg/mL, 1 mg/mL-250 mg/mL, 1 mg/mL-200 mg/mL, 1 mg/mL-150 mg/mL, 1 mg/mL-140 mg/mL, 1 mg/mL-130 mg/mL, 1 mg/mL-120 mg/mL, 1 mg/mL-110 mg/mL, 1 mg/mL-100 mg/mL, 1 mg/mL-95 mg/mL, 1 mg/mL-90 mg/mL, 1 mg/mL-85 mg/mL, 1 mg/mL-80 mg/mL, 1 mg/mL-75 mg/mL, 1 mg/mL-70 mg/mL, 1 mg/mL-65 mg/mL, 1 mg/mL-60 mg/mL, 1 mg/mL-55 mg/mL, 1 mg/mL-50 mg/mL, 1 mg/mL-45 mg/mL, 1 mg/mL-40 mg/mL, 1 mg/mL-35 mg/mL, 1 mg/mL-30 mg/mL, 1 mg/mL-25 mg/mL, 1 mg/mL-20 mg/mL, 1 mg/mL-15 mg/mL, 5 mg/mL-90 mg/mL, 5 mg/mL-85 mg/mL, 5 mg/mL-80 mg/mL, 5 mg/mL-75 mg/mL, 5 mg/mL-70 mg/mL, 5 mg/mL-65 mg/mL, 5 mg/mL-60 mg/mL, 5 mg/mL-55 mg/mL, 5 mg/mL-50 mg/mL, 5 mg/mL-45 mg/mL, 5 mg/mL-40 mg/mL, 5 mg/mL-35 mg/mL, 5 mg/mL-30 mg/mL, 5 mg/mL-25 mg/mL, 5 mg/mL-20 mg/mL, 5 mg/mL-15 mg/mL, 8 mg/mL-12 mg/mL, 30 mg/mL-250 mg/mL, 30 mg/mL-200 mg/mL, 30 mg/mL-190 mg/mL, 30 mg/mL-180 mg/mL, 30 mg/mL-170 mg/mL, 30 mg/mL-160 mg/mL, 30 mg/mL-150 mg/mL, 30 mg/mL-140 mg/mL, 30 mg/mL-130 mg/mL, 30 mg/mL-120 mg/mL, 30 mg/mL-110 mg/mL, 30 mg/mL-100 mg/mL, 50 mg/mL-200 mg/mL, 50 mg/mL-190 mg/mL, 50 mg/mL-180 mg/mL, 50 mg/mL-170 mg/mL, 50 mg/mL-160 mg/mL, 50 mg/mL-150 mg/mL, 50 mg/mL-140 mg/mL, 50 mg/mL-130 mg/mL, 50 mg/mL-120 mg/mL, 50 mg/mL-110 mg/mL, 50 mg/mL-100 mg/mL, 70 mg/mL-180 mg/mL, 70 mg/mL-170 mg/mL, 70 mg/mL-160 mg/mL, 70 mg/mL-150 mg/mL, 70 mg/mL-140 mg/mL, 70 mg/mL-130 mg/mL, 70 mg/mL-120 mg/mL, 70 mg/mL-110 mg/mL, 70 mg/mL-100 mg/mL, 90 mg/mL-110 mg/mL, or 95 mg/mL-105 mg/mL. In some embodiments, the PD-1/PVRIG/TIGIT-binding protein in the pharmaceutical composition is at a concentration of 0.1 mg/mL-400 mg/mL. In some embodiments, the PD-1/PVRIG/TIGIT-binding protein in the pharmaceutical composition is at a concentration of 0.5 mg/mL-200 mg/mL. In some embodiments, the PD-1/PVRIG/TIGIT-binding protein in the pharmaceutical composition is at a concentration of 1 mg/mL-150 mg/mL. In some embodiments, the PD-1/PVRIG/TIGIT-binding protein in the pharmaceutical composition is at a concentration of 5 mg/mL-45 mg/mL. In some embodiments, the PD-1/PVRIG/TIGIT-binding protein in the pharmaceutical composition is at a concentration of 50 mg/mL-110 mg/mL. In some embodiments, the PD-1/PVRIG/TIGIT-binding protein in the pharmaceutical composition is at a concentration of about 1 mg/mL, about 5 mg/mL, about 10 mg/mL, about 15 mg/mL, about 20 mg/mL, about 25 mg/mL, about 30 mg/mL, about 40 mg/mL, about 45 mg/mL, about 50 mg/mL, about 55 mg/mL, about 60 mg/mL, about 70 mg/mL, about 75 mg/mL, about 80 mg/mL, about 85 mg/mL, about 90 mg/mL, about 95 mg/mL, about 100 mg/mL, about 110 mg/mL, about 120 mg/mL, about 130 mg/mL, or about 150 mg/mL. In some embodiments, the PD-1/PVRIG/TIGIT-binding protein in the pharmaceutical composition is at a concentration of about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL. In some embodiments, the PD-1/PVRIG/TIGIT-binding protein in the pharmaceutical composition is at a concentration of 10 mg/mL, about 20 mg/mL, about 25 mg/mL, about 50 mg/mL, about 80 mg/mL, or about 100 mg/mL. In some embodiments, the PD-1/PVRIG/TIGIT-binding protein in the pharmaceutical composition is at a concentration of about 120 mg/mL, about 130 mg/mL, or about 150 mg/mL. In some embodiments, the PD-1/PVRIG/TIGIT-binding protein in the pharmaceutical composition is at a concentration of at least 50 mg/mL, at least 100 mg/mL, at least 200 mg/mL, at least 400 mg/mL, or at least 500 mg/mL.

In some embodiments, the buffer in the pharmaceutical composition is at a concentration of 0.1 mM-50 mM, e.g., 0.1 mM-45 mM, 0.1 mM-40 mM, 0.1 mM-35 mM, 0.1 mM-30 mM, 0.1 mM-25 mM, 0.1 mM-20 mM, 0.1 mM-15 mM, 0.1 mM-10 mM, 0.5 mM-45 mM, 0.5 mM-40 mM, 0.5 mM-35 mM, 0.5 mM-30 mM, 0.5 mM-25 mM, 0.5 mM-20 mM, 0.5 mM-15 mM, 0.5 mM-10 mM, 1 mM-40 mM, 1 mM-35 mM, 1 mM-30 mM, 1 mM-25 mM, 1 mM-20 mM, 1 mM-15 mM, 1 mM-10 mM, 5 mM-35 mM, 5 mM-30 mM, 5 mM-25 mM, 5 mM-20 mM, 5 mM-15 mM, 5 mM-10 mM, 8 mM-30 mM, 8 mM-25 mM, 8 mM-20 mM, 8 mM-15 mM, or 8 mM-12 mM. In some embodiments, the buffer in the pharmaceutical composition is at a concentration of 0.5 mM-40 mM. In some embodiments, the buffer in the pharmaceutical composition is at a concentration of 1 mM-30 mM. In some embodiments, the buffer in the pharmaceutical composition is at a concentration of 5 mM-20 mM. In some embodiments, the buffer in the pharmaceutical composition is at a concentration of about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM, or about 30 mM. In some embodiments, the buffer in the pharmaceutical composition is at a concentration of about 10 mM. In some embodiments, the buffer in the pharmaceutical composition is at a concentration of at least 10 mM, at least 20 mM, at least 30 mM, at least 40 mM, or at least 50 mM.

In some embodiments, the pharmaceutical composition further comprises a surfactant. In some embodiments, the surfactant is an ionic or nonionic surfactant. In some embodiments, the surfactant is one or more surfactants selected from the group consisting of polysorbate, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocamidopropyl-betaine, linoleamidopropyl-betaine, myristamidopropyl-betaine, palmidopropyl-betaine, isostearamidopropyl-betaine, myristamidopropyl-dimethylamine, palmidopropyl-dimethylamine, isostearamidopropyl-dimethylamine, sodium methyl cocoyl, sodium methyl oleyl taurate, polyethylene glycol, polypropylene glycol, and copolymers of ethylene and propylene glycol. In some embodiments, the surfactant is selected from the group consisting of polysorbate. In some embodiments, the surfactant is selected from the group consisting of polysorbate 20 and/or polysorbate 80. In some embodiments, the surfactant is polysorbate 80.

In some embodiments, the surfactant in the pharmaceutical composition is at a concentration of 0.01 mg/mL-10 mg/mL, e.g., 0.01 mg/mL-9 mg/mL, 0.01 mg/mL-8 mg/mL, 0.01 mg/mL-7 mg/mL, 0.01 mg/mL-6 mg/mL, 0.01 mg/mL-5 mg/mL, 0.01 mg/mL-4 mg/mL, 0.01 mg/mL-3 mg/mL, 0.01 mg/mL-2 mg/mL, 0.01 mg/mL-1 mg/mL, 0.01 mg/mL-0.5 mg/mL, 0.05 mg/mL-8 mg/mL, 0.05 mg/mL-7 mg/mL, 0.05 mg/mL-6 mg/mL, 0.05 mg/mL-5 mg/mL, 0.05 mg/mL-4 mg/mL, 0.05 mg/mL-3 mg/mL, 0.05 mg/mL-2 mg/mL, 0.05 mg/mL-1 mg/mL, 0.05 mg/mL-0.5 mg/mL, 0.1 mg/mL-6 mg/mL, 0.1 mg/mL-5 mg/mL, 0.1 mg/mL-4 mg/mL, 0.1 mg/mL-3 mg/mL, 0.1 mg/mL-2 mg/mL, 0.1 mg/mL-1 mg/mL, 0.1 mg/mL-0.5 mg/mL, 0.2 mg/mL-5 mg/mL, 0.2 mg/mL-4.5 mg/mL, 0.2 mg/mL-4 mg/mL, 0.2 mg/mL-3.5 mg/mL, 0.2 mg/mL-3 mg/mL, 0.2 mg/mL-2.5 mg/mL, 0.2 mg/mL-2 mg/mL, 0.2 mg/mL-1.5 mg/mL, 0.2 mg/mL-1 mg/mL, 0.2 mg/mL-0.5 mg/mL, 0.3 mg/mL-4.5 mg/mL, 0.3 mg/mL-4 mg/mL, 0.3 mg/mL-3.5 mg/mL, 0.3 mg/mL-3 mg/mL, 0.3 mg/mL-2.5 mg/mL, 0.3 mg/mL-2 mg/mL, 0.3 mg/mL-1.5 mg/mL, 0.3 mg/mL-1 mg/mL, 0.3 mg/mL-0.5 mg/mL, 0.7 mg/mL-3.5 mg/mL, 0.7 mg/mL-3 mg/mL, 0.7 mg/mL-2.5 mg/mL, 0.7 mg/mL-2 mg/mL, 0.7 mg/mL-1.5 mg/mL, 0.7 mg/mL-1 mg/mL, or 0.7 mg/mL-0.9 mg/mL. In some embodiments, the surfactant in the pharmaceutical composition is at a concentration of 0.05 mg/mL-5 mg/mL. In some embodiments, the surfactant in the pharmaceutical composition is at a concentration of 0.1 mg/mL-3 mg/mL. In some embodiments, the surfactant in the pharmaceutical composition is at a concentration of 0.2 mg/mL-2 mg/mL. In some embodiments, the surfactant in the pharmaceutical composition is at a concentration of about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, about 1 mg/mL, about 1.1 mg/mL, about 1.2 mg/mL, about 1.3 mg/mL, about 1.4 mg/mL, about 1.5 mg/mL, about 1.6 mg/mL, about 1.7 mg/mL, about 1.8 mg/mL, about 1.9 mg/mL, or about 2 mg/mL. In some embodiments, the surfactant in the pharmaceutical composition is at a concentration of about 0.2 mg/mL, about 0.4 mg/mL, about 0.6 mg/mL, or about 0.8 mg/mL. In some embodiments, the surfactant in the pharmaceutical composition is at a concentration of about 0.4 mg/mL or about 0.8 mg/mL. In some embodiments, the surfactant in the pharmaceutical composition is at a concentration of at least 0.4 mg/mL, at least 0.8 mg/mL, at least 2 mg/mL, at least 3 mg/mL, at least 5 mg/mL, or at least 10 mg/mL.

In some embodiments, the pharmaceutical composition further comprises a sugar. In some embodiments, the sugar is one or more sugars selected from the group consisting of sucrose, glucose, trehalose, and maltose. In some embodiments, the sugar is sucrose. In some embodiments, the sugar in the pharmaceutical composition is at a concentration of 0.1% w/v-20% w/v, e.g., 0.1% w/v-15% w/v, 0.1% w/v-14% w/v, 0.1% w/v-13% w/v, 0.1% w/v-12% w/v, 0.1% w/v-11% w/v, 0.1% w/v-10% w/v, 0.1% w/v-9.5% w/v, 0.1% w/v-9% w/v, 0.1% w/v-8.5% w/v, 0.1% w/v-8% w/v, 1% w/v-15% w/v, 1% w/v-14% w/v, 1% w/v-13% w/v, 1% w/v-12% w/v, 1% w/v-11% w/v, 1% w/v-10% w/v, 1% w/v-9.5% w/v, 1% w/v-9% w/v, 1% w/v-8.5% w/v, 1% w/v-8% w/v, 2% w/v-13% w/v, 2% w/v-12% w/v, 2% w/v-11% w/v, 2% w/v-10% w/v, 2% w/v-9.5% w/v, 2% w/v-9% w/v, 2% w/v-8.5% w/v, 2% w/v-8% w/v, 3% w/v-12% w/v, 3% w/v-11% w/v, 3% w/v-10% w/v, 3% w/v-9.5% w/v, 3% w/v-9% w/v, 3% w/v-8.5% w/v, 3% w/v-8% w/v, 5% w/v-11% w/v, 5% w/v-10% w/v, 5% w/v-9.5% w/v, 5% w/v-9% w/v, 5% w/v-8.5% w/v, 5% w/v-8% w/v, 6% w/v-10% w/v, 6% w/v-9.5% w/v, 6% w/v-9% w/v, 6% w/v-8.5% w/v, 6% w/v-8% w/v, 7% w/v-9.5% w/v, 7% w/v-9% w/v, 7% w/v-8.5% w/v, or 7% w/v-8% w/v. In some embodiments, the sugar in the pharmaceutical composition is at a concentration of 1% w/v-15% w/v. In some embodiments, the sugar in the pharmaceutical composition is at a concentration of 3% w/v-12% w/v. In some embodiments, the sugar in the pharmaceutical composition is at a concentration of 5% w/v-10% w/v. In some embodiments, the sugar in the pharmaceutical composition is at a concentration of about 3% w/v, about 3.5% w/v, about 4% w/v, about 4.5% w/v, about 5% w/v, about 5.5% w/v, about 6% w/v, about 6.5% w/v, about 7% w/v, about 7.5% w/v, about 8% w/v, about 8.5% w/v, about 9% w/v, about 9.5% w/v, about 10% w/v, about 10.5% w/v, about 11% w/v, about 11.5% w/v, or about 12% w/v. In some embodiments, the sugar in the pharmaceutical composition is at a concentration of about 8% w/v. In some embodiments, the sugar in the pharmaceutical composition is at a concentration of at least 8% w/v, at least 10% w/v, at least 12% w/v, at least 15% w/v, or at least 20% w/v.

In some embodiments, the pharmaceutical composition further comprises an additional amino acid or a pharmaceutically acceptable salt thereof. In some embodiments, the additional amino acid or the pharmaceutically acceptable salt thereof is arginine or a pharmaceutically acceptable salt thereof. In some embodiments, the additional amino acid or the pharmaceutically acceptable salt thereof is arginine-hydrochloric acid.

In some embodiments, the additional amino acid or the pharmaceutically acceptable salt thereof in the pharmaceutical composition is at a concentration of 1 mM-200 mM, e.g., 1 mM-190 mM, 1 mM-180 mM, 1 mM-170 mM, 1 mM-160 mM, 1 mM-150 mM, 1 mM-140 mM, 1 mM-130 mM, 1 mM-120 mM, 1 mM-110 mM, 1 mM-100 mM, 1 mM-90 mM, 1 mM-80 mM, 1 mM-70 mM, 1 mM-60 mM, 1 mM-50 mM, 1 mM-40 mM, 1 mM-30 mM, 1 mM-20 mM, 5 mM-180 mM, 5 mM-170 mM, 5 mM-160 mM, 5 mM-150 mM, 5 mM-140 mM, 5 mM-130 mM, 5 mM-120 mM, 5 mM-110 mM, 5 mM-100 mM, 5 mM-90 mM, 5 mM-80 mM, 5 mM-70 mM, 5 mM-60 mM, 5 mM-50 mM, 5 mM-40 mM, 5 mM-30 mM, 5 mM-20 mM, 10 mM-170 mM, 10 mM-160 mM, 10 mM-150 mM, 10 mM-140 mM, 10 mM-130 mM, 10 mM-120 mM, 10 mM-110 mM, 10 mM-100 mM, 10 mM-90 mM, 10 mM-80 mM, 10 mM-70 mM, 10 mM-60 mM, 10 mM-50 mM, 10 mM-40 mM, 10 mM-30 mM, 10 mM-20 mM, 15 mM-160 mM, 15 mM-150 mM, 15 mM-140 mM, 15 mM-130 mM, 15 mM-120 mM, 15 mM-110 mM, 15 mM-100 mM, 15 mM-90 mM, 15 mM-80 mM, 15 mM-70 mM, 15 mM-60 mM, 15 mM-50 mM, 15 mM-40 mM, 15 mM-30 mM, 15 mM-20 mM, 20 mM-150 mM, 20 mM-140 mM, 20 mM-130 mM, 20 mM-120 mM, 20 mM-110 mM, 20 mM-100 mM, 20 mM-90 mM, 20 mM-80 mM, 20 mM-70 mM, 20 mM-60 mM, 20 mM-50 mM, 20 mM-40 mM, 20 mM-30 mM, 25 mM-140 mM, 25 mM-130 mM, 25 mM-120 mM, 25 mM-110 mM, 25 mM-100 mM, 25 mM-90 mM, 25 mM-80 mM, 25 mM-70 mM, 25 mM-60 mM, 25 mM-50 mM, 25 mM-40 mM, 30 mM-140 mM, 30 mM-130 mM, 30 mM-120 mM, 30 mM-110 mM, 30 mM-100 mM, 30 mM-90 mM, 30 mM-80 mM, 30 mM-70 mM, 30 mM-60 mM, 30 mM-50 mM, 30 mM-40 mM, 40 mM-120 mM, 40 mM-110 mM, 40 mM-100 mM, 40 mM-90 mM, 40 mM-80 mM, 40 mM-70 mM, 40 mM-60 mM, 40 mM-50 mM, 50 mM-110 mM, 50 mM-100 mM, 50 mM-90 mM, 50 mM-80 mM, 50 mM-70 mM, or 50 mM-60 mM. In some embodiments, the additional amino acid or the pharmaceutically acceptable salt thereof in the pharmaceutical composition is at a concentration of 10 mM-80 mM. In some embodiments, the additional amino acid or the pharmaceutically acceptable salt thereof in the pharmaceutical composition is at a concentration of 20 mM-70 mM. In some embodiments, the additional amino acid or the pharmaceutically acceptable salt thereof in the pharmaceutical composition is at a concentration of 40 mM-60 mM. In some embodiments, the additional amino acid or the pharmaceutically acceptable salt thereof in the pharmaceutical composition is at a concentration of about 20 mM, about 25 mM, about 30 mM, about 35 mM, about 40 mM, about 45 mM, about 50 mM, about 55 mM, about 60 mM, about 65 mM, or about 70 mM. In some embodiments, the buffer in the pharmaceutical composition is at a concentration of about 50 mM. In some embodiments, the additional amino acid or the pharmaceutically acceptable salt thereof in the pharmaceutical composition is at a concentration of 5 mM-170 mM. In some embodiments, the additional amino acid or the pharmaceutically acceptable salt thereof in the pharmaceutical composition is at a concentration of 10 mM-150 mM. In some embodiments, the additional amino acid or the pharmaceutically acceptable salt thereof in the pharmaceutical composition is at a concentration of 15 mM-120 mM. In some embodiments, the additional amino acid or the pharmaceutically acceptable salt thereof in the pharmaceutical composition is at a concentration of about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, about 100 mM, about 105 mM, about 110 mM, about 115 mM, or about 120 mM. In some embodiments, the additional amino acid or the pharmaceutically acceptable salt thereof in the pharmaceutical composition is at a concentration of about 20 mM, about 50 mM, or about 100 mM.

In some embodiments, the buffer in the pharmaceutical composition has a pH of 3.5-9, e.g., 3.5-8.5, 3.5-8, 3.5-7.5, 3.5-7, 3.5-6.9, 3.5-6.8, 3.5-6.7, 3.5-6.6, 3.5-6.5, 3.5-6.4, 3.5-6.3, 3.5-6.2, 3.5-6.1, 3.5-6, 3.5-5.9, 3.5-5.8, 3.5-5.7, 3.5-5.6, 3.5-5.5, 4-5.4, 4-5.3, 4-5.2, 4-5.1, 4-5.0, 4-8.5, 4-8, 4-7.5, 4-7, 4-6.9, 4-6.8, 4-6.7, 4-6.6, 4-6.5, 4-6.4, 4-6.3, 4-6.2, 4-6.1, 4-6, 4-5.9, 4-5.8, 4-5.7, 4-5.6, 4-5.5, 4-5.4, 4-5.3, 4-5.2, 4-5.1, 4-5.0, 4.2-8, 4.2-7.5, 4.2-7, 4.2-6.9, 4.2-6.8, 4.2-6.7, 4.2-6.6, 4.2-6.5, 4.2-6.4, 4.2-6.3, 4.2-6.2, 4.2-6.1, 4.2-6, 4.2-5.9, 4.2-5.8, 4.2-5.7, 4.2-5.6, 4.2-5.5, 4.2-5.4, 4.2-5.3, 4.2-5.2, 4.2-5.1, 4.2-5.0, 4.5-8, 4.5-7.5, 4.5-7, 4.5-6.9, 4.5-6.8, 4.5-6.7, 4.5-6.6, 4.5-6.5, 4.5-6.4, 4.5-6.3, 4.5-6.2, 4.5-6.1, 4.5-6, 4.5-5.9, 4.5-5.8, 4.5-5.7, 4.5-5.6, 4.5-5.5, 4.5-5.4, 4.5-5.3, 4.5-5.2, 4.5-5.1, 4.5-5.0, 4.6-7.5, 4.6-7, 4.6-6.9, 4.6-6.8, 4.6-6.7, 4.6-6.6, 4.6-6.5, 4.6-6.4, 4.6-6.3, 4.6-6.2, 4.6-6.1, 4.6-6, 4.6-5.9, 4.6-5.8, 4.6-5.7, 4.6-5.6, 4.6-5.5, 4.6-5.4, 4.6-5.3, 4.6-5.2, 4.6-5.1, 4.6-5.0, 4.8-7, 4.8-6.9, 4.8-6.8, 4.8-6.7, 4.8-6.6, 4.8-6.5, 4.8-6.4, 4.8-6.3, 4.8-6.2, 4.8-6.1, 4.8-6, 4.8-5.9, 4.8-5.8, 4.8-5.7, 4.8-5.6, 4.8-5.5, 5-6.5, 5-6.4, 5-6.3, 5-6.2, 5-6.1, 5-6, 5-5.9, 5-5.8, 5-5.7, 5-5.6, or 5-5.5. In some embodiments, the buffer in the pharmaceutical composition has a pH of 3.5-7. In some embodiments, the buffer in the pharmaceutical composition has a pH of 4.0-6.5. In some embodiments, the buffer in the pharmaceutical composition has a pH of 4.2-6.2. In some embodiments, the buffer in the pharmaceutical composition has a pH of 4.5-6.0. In some embodiments, the buffer in the pharmaceutical composition has a pH of about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, or about 6.5. In some embodiments, the buffer in the pharmaceutical composition has a pH of about 5.0, about 5.2, about 5.3, about 5.5, about 5.6, about 5.8, or about 6.0. In some embodiments, the buffer in the pharmaceutical composition has a pH of about 5.0 or about 5.5. In some embodiments, the buffer in the pharmaceutical composition has a pH of at least 5.0, at least 5.5, at least 6.0, at least 6.5, at least 7, or at least 9.

In some embodiments, the pharmaceutical composition further comprises a pH adjuster, such as sodium hydroxide and/or hydrochloric acid.

In some embodiments, there is a difference of no more than ±0.5 between the pH of the pharmaceutical composition and the pH of the buffer that the pharmaceutical composition comprises. In some embodiments, the pharmaceutical composition has a pH of 3.5-9, e.g., 3.5-8.5, 3.5-8, 3.5-7.5, 3.5-7, 3.5-6.9, 3.5-6.8, 3.5-6.7, 3.5-6.6, 3.5-6.5, 3.5-6.4, 3.5-6.3, 3.5-6.2, 3.5-6.1, 3.5-6, 3.5-5.9, 3.5-5.8, 3.5-5.7, 3.5-5.6, 3.5-5.5, 4-5.4, 4-5.3, 4-5.2, 4-5.1, 4-5.0, 4-8.5, 4-8, 4-7.5, 4-7, 4-6.9, 4-6.8, 4-6.7, 4-6.6, 4-6.5, 4-6.4, 4-6.3, 4-6.2, 4-6.1, 4-6, 4-5.9, 4-5.8, 4-5.7, 4-5.6, 4-5.5, 4-5.4, 4-5.3, 4-5.2, 4-5.1, 4-5.0, 4.2-8, 4.2-7.5, 4.2-7, 4.2-6.9, 4.2-6.8, 4.2-6.7, 4.2-6.6, 4.2-6.5, 4.2-6.4, 4.2-6.3, 4.2-6.2, 4.2-6.1, 4.2-6, 4.2-5.9, 4.2-5.8, 4.2-5.7, 4.2-5.6, 4.2-5.5, 4.2-5.4, 4.2-5.3, 4.2-5.2, 4.2-5.1, 4.2-5.0, 4.5-8, 4.5-7.5, 4.5-7, 4.5-6.9, 4.5-6.8, 4.5-6.7, 4.5-6.6, 4.5-6.5, 4.5-6.4, 4.5-6.3, 4.5-6.2, 4.5-6.1, 4.5-6, 4.5-5.9, 4.5-5.8, 4.5-5.7, 4.5-5.6, 4.5-5.5, 4.5-5.4, 4.5-5.3, 4.5-5.2, 4.5-5.1, 4.5-5.0, 4.6-7.5, 4.6-7, 4.6-6.9, 4.6-6.8, 4.6-6.7, 4.6-6.6, 4.6-6.5, 4.6-6.4, 4.6-6.3, 4.6-6.2, 4.6-6.1, 4.6-6, 4.6-5.9, 4.6-5.8, 4.6-5.7, 4.6-5.6, 4.6-5.5, 4.6-5.4, 4.6-5.3, 4.6-5.2, 4.6-5.1, 4.6-5.0, 4.8-7, 4.8-6.9, 4.8-6.8, 4.8-6.7, 4.8-6.6, 4.8-6.5, 4.8-6.4, 4.8-6.3, 4.8-6.2, 4.8-6.1, 4.8-6, 4.8-5.9, 4.8-5.8, 4.8-5.7, 4.8-5.6, 4.8-5.5, 5-6.5, 5-6.4, 5-6.3, 5-6.2, 5-6.1, 5-6, 5-5.9, 5-5.8, 5-5.7, 5-5.6, or 5-5.5. In some embodiments, the pharmaceutical composition has a pH of 3.5-7. In some embodiments, the pharmaceutical composition has a pH of 4.0-6.5. In some embodiments, the pharmaceutical composition has a pH of 4.2-6.2. In some embodiments, the pharmaceutical composition has a pH of 4.5-6.0. In some embodiments, the pharmaceutical composition has a pH of about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, or about 6.5. In some embodiments, the pharmaceutical composition has a pH of about 5.0, about 5.2, about 5.3, about 5.5, about 5.6, about 5.8, or about 6.0. In some embodiments, the pharmaceutical composition has a pH of about 5.0 or about 5.5. In some embodiments, the pharmaceutical composition has a pH of at least 5.0, at least 5.5, at least 6.0, at least 6.5, at least 7, or at least 9.

In some embodiments, the present disclosure provides a pharmaceutical composition comprising a PD-1/PVRIG/TIGIT-binding protein (e.g., A17m0902-1708-151H7-01-C with first polypeptide chain and second polypeptide chain sequences set forth in SEQ ID NOs: 76 and 52, respectively, or A17m0902-1708-151H7-C with first polypeptide chain and second polypeptide chain sequences set forth in SEQ ID NOs: 72 and 52, respectively), and the pharmaceutical composition comprises any one of the following groups 1)-6):

1) a PD-1/PVRIG/TIGIT-binding protein;
a histidine salt buffer, e.g., a histidine hydrochloride buffer or a histidine acetate buffer, a histidine-histidine hydrochloride buffer, or a histidine-acetic acid buffer;

2) a PD-1/PVRIG/TIGIT-binding protein;

a histidine salt buffer;
polysorbate;
sucrose;
wherein optionally, the composition further comprises arginine or a pharmaceutically acceptable salt thereof (e.g., arginine hydrochloride);

3) a PD-1/PVRIG/TIGIT-binding protein;

a histidine hydrochloride buffer, e.g., a histidine-histidine hydrochloride buffer;
polysorbate;
sucrose;
wherein optionally, the composition further comprises arginine or a pharmaceutically acceptable salt thereof (e.g., arginine hydrochloride);

4) a PD-1/PVRIG/TIGIT-binding protein;

a histidine acetate buffer, e.g., a histidine-acetic acid buffer;
polysorbate;
sucrose;
wherein optionally, the composition further comprises arginine or a pharmaceutically acceptable salt thereof (e.g., arginine hydrochloride);

5) a PD-1/PVRIG/TIGIT-binding protein;
a citrate buffer, e.g., a citric acid-sodium citrate buffer or a citric acid-disodium citrate buffer;

6) a PD-1/PVRIG/TIGIT-binding protein;

a citrate buffer, e.g., a citric acid-disodium citrate buffer;
polysorbate;
sucrose;
wherein optionally, the composition further comprises arginine or a pharmaceutically acceptable salt thereof (e.g., arginine hydrochloride).

In some embodiments, the present disclosure provides a pharmaceutical composition comprising a PD-1/PVRIG/TIGIT-binding protein (e.g., A17m0902-1708-151H7-01-C with first polypeptide chain and second polypeptide chain sequences set forth in SEQ ID NOs: 76 and 52, respectively, or A17m0902-1708-151H7-C with first polypeptide chain and second polypeptide chain sequences set forth in SEQ ID NOs: 72 and 52, respectively), and the pharmaceutical composition comprises or is any one of the following groups 1)-6):

1) a PD-1/PVRIG/TIGIT-binding protein; a histidine salt buffer; polysorbate; sucrose; water for injection, optionally arginine or a pharmaceutically acceptable salt thereof (e.g., arginine hydrochloride), and optionally sodium hydroxide and/or hydrochloric acid;
2) a PD-1/PVRIG/TIGIT-binding protein; a histidine hydrochloride buffer; polysorbate; sucrose; water for injection, optionally arginine or a pharmaceutically acceptable salt thereof (e.g., arginine hydrochloride), and optionally sodium hydroxide and/or hydrochloric acid;
3) a PD-1/PVRIG/TIGIT-binding protein; a histidine-histidine hydrochloride buffer; polysorbate; sucrose; water for injection, optionally arginine or a pharmaceutically acceptable salt thereof (e.g., arginine hydrochloride), and optionally sodium hydroxide and/or hydrochloric acid;
4) a PD-1/PVRIG/TIGIT-binding protein; a histidine-acetic acid buffer; polysorbate; sucrose; water for injection, optionally arginine or a pharmaceutically acceptable salt thereof (e.g., arginine hydrochloride), and optionally sodium hydroxide and/or hydrochloric acid;
5) a PD-1/PVRIG/TIGIT-binding protein; a citrate buffer; polysorbate; sucrose; water for injection, optionally arginine or a pharmaceutically acceptable salt thereof (e.g., arginine hydrochloride), and optionally sodium hydroxide and/or hydrochloric acid;
6) a PD-1/PVRIG/TIGIT-binding protein; a citric acid-disodium citrate buffer; polysorbate; sucrose; water for injection, optionally arginine or a pharmaceutically acceptable salt thereof (e.g., arginine hydrochloride), and optionally sodium hydroxide and/or hydrochloric acid.

In some embodiments, the present disclosure provides a pharmaceutical composition comprising a PD-1/PVRIG/TIGIT-binding protein (e.g., A17m0902-1708-151H7-01-C with first polypeptide chain and second polypeptide chain sequences set forth in SEQ ID NOs: 76 and 52, respectively, or A17m0902-1708-151H7-C with first polypeptide chain and second polypeptide chain sequences set forth in SEQ ID NOs: 72 and 52, respectively), and the pharmaceutical composition comprises any one of the following groups 1)-10):

1) 0.01 mg/mL-500 mg/mL PD-1/PVRIG/TIGIT-binding protein;

0.1 mM-50 mM histidine hydrochloride buffer, e.g., a histidine hydrochloride buffer or a histidine acetate buffer, a histidine-histidine hydrochloride buffer, or a histidine-acetic acid buffer;
0.01 mg/mL-10 mg/mL polysorbate;
0.1% w/v-20% w/v sucrose;
wherein optionally, the pharmaceutical composition further comprises 1 mM-200 mM arginine or a pharmaceutically acceptable salt thereof (e.g., arginine hydrochloride);
and the pharmaceutical composition has a pH of 3.5-7;

2) 0.1 mg/mL-400 mg/mL PD-1/PVRIG/TIGIT-binding protein;

0.5 mM-40 mM histidine hydrochloride buffer, e.g., a histidine hydrochloride buffer or a histidine acetate buffer, a histidine-histidine hydrochloride buffer, or a histidine-acetic acid buffer;
0.05 mg/mL-5 mg/mL polysorbate;
1% w/v-15% w/v sucrose;
wherein optionally, the pharmaceutical composition further comprises 5 mM-170 mM arginine or a pharmaceutically acceptable salt thereof (e.g., arginine hydrochloride);
and the pharmaceutical composition has a pH of 4-6.5;

3) 0.5 mg/mL-200 mg/mL PD-1/PVRIG/TIGIT-binding protein;

1 mM-30 mM histidine hydrochloride buffer, e.g., a histidine hydrochloride buffer or a histidine acetate buffer, a histidine-histidine hydrochloride buffer, or a histidine-acetic acid buffer;
0.1 mg/mL-3 mg/mL polysorbate;
3% w/v-12% w/v sucrose;
wherein optionally, the pharmaceutical composition further comprises 10 mM-150 mM arginine or a pharmaceutically acceptable salt thereof (e.g., arginine hydrochloride);
and the pharmaceutical composition has a pH of 4.2-6.2;

4) 1 mg/mL-150 mg/mL PD-1/PVRIG/TIGIT-binding protein;

5 mM-20 mM histidine hydrochloride buffer, e.g., a histidine hydrochloride buffer or a histidine acetate buffer, a histidine-histidine hydrochloride buffer, or a histidine-acetic acid buffer;
0.2 mg/mL-2 mg/mL polysorbate;
5% w/v-10% w/v sucrose;
wherein optionally, the pharmaceutical composition further comprises 15 mM-120 mM arginine or a pharmaceutically acceptable salt thereof (e.g., arginine hydrochloride);
wherein the pharmaceutical composition has a pH of 4.5-6;

5) 0.01 mg/mL-500 mg/mL PD-1/PVRIG/TIGIT-binding protein;

0.1 mM-50 mM citrate buffer, e.g., a citric acid-disodium citrate buffer;
0.01 mg/mL-10 mg/mL polysorbate;
0.1% w/v-20% w/v sucrose;
wherein optionally, the pharmaceutical composition further comprises 1 mM-200 mM arginine or a pharmaceutically acceptable salt thereof (e.g., arginine hydrochloride);
and the pharmaceutical composition has a pH of 3.5-7;

6) 0.1 mg/mL-400 mg/mL PD-1/PVRIG/TIGIT-binding protein;

0.5 mM-40 mM citrate buffer, e.g., a citric acid-disodium citrate buffer;
0.05 mg/mL-5 mg/mL polysorbate;
1% w/v-15% w/v sucrose;
wherein optionally, the pharmaceutical composition further comprises 5 mM-170 mM arginine or a pharmaceutically acceptable salt thereof (e.g., arginine hydrochloride);
and the pharmaceutical composition has a pH of 4-6.5;

7) 0.5 mg/mL-200 mg/mL PD-1/PVRIG/TIGIT-binding protein;

1 mM-30 mM citrate buffer, e.g., a citric acid-disodium citrate buffer;
0.1 mg/mL-3 mg/mL polysorbate;
3% w/v-12% w/v sucrose;
wherein optionally, the pharmaceutical composition further comprises 10 mM-150 mM arginine or a pharmaceutically acceptable salt thereof (e.g., arginine hydrochloride);
and the pharmaceutical composition has a pH of 4.2-6.2;

8) 1 mg/mL-150 mg/mL PD-1/PVRIG/TIGIT-binding protein;

5 mM-20 mM citrate buffer, e.g., a citric acid-disodium citrate buffer;
0.2 mg/mL-2 mg/mL polysorbate;
5% w/v-10% w/v sucrose;
wherein optionally, the pharmaceutical composition further comprises 15 mM-120 mM arginine or a pharmaceutically acceptable salt thereof (e.g., arginine hydrochloride);
wherein the pharmaceutical composition has a pH of 4.5-6;

9) any one of the pharmaceutical compositions in 1)-8), wherein the polysorbate is polysorbate 80;
10) any one of the pharmaceutical compositions in 1)-9), wherein the pharmaceutical composition further comprises water for injection.

In some embodiments, the present disclosure provides a pharmaceutical composition comprising a PD-1/PVRIG/TIGIT-binding protein (e.g., A17m0902-1708-151H7-01-C with first polypeptide chain and second polypeptide chain sequences set forth in SEQ ID NOs: 76 and 52, respectively, or A17m0902-1708-151H7-C with first polypeptide chain and second polypeptide chain sequences set forth in SEQ ID NOs: 72 and 52, respectively), and the pharmaceutical composition comprises or is any one of the following groups 1)-16):

1) 1-150 mg/mL PD-1/PVRIG/TIGIT-binding protein;

about 10 mM histidine hydrochloride buffer or histidine acetate buffer, e.g., a histidine-histidine hydrochloride buffer or a histidine-acetic acid buffer;
0.2 mg/mL-2 mg/mL polysorbate 80;
about 8% w/v sucrose;
wherein the pharmaceutical composition has a pH of 4.5-6;

2) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM histidine-hydrochloride buffer, e.g., a histidine-histidine hydrochloride buffer;
about 0.4 mg/mL polysorbate 80;
about 8% w/v sucrose;
wherein the pharmaceutical composition has a pH of about 5;

3) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM histidine-hydrochloride buffer, e.g., a histidine-histidine hydrochloride buffer;
about 0.4 mg/mL polysorbate 80;

about 8% w/v sucrose;
wherein the pharmaceutical composition has a pH of about 5.5;

4) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM histidine-hydrochloride buffer, e.g., a histidine-histidine hydrochloride buffer;
about 0.8 mg/mL polysorbate 80;
about 8% w/v sucrose;
wherein the pharmaceutical composition has a pH of about 5;

5) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM histidine-hydrochloride buffer, e.g., a histidine-histidine hydrochloride buffer;
about 0.8 mg/mL polysorbate 80;
about 8% w/v sucrose;
wherein the pharmaceutical composition has a pH of about 5.5;

6) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM histidine-acetic acid buffer;
about 0.4 mg/mL polysorbate 80;
about 8% w/v sucrose;
wherein the pharmaceutical composition has a pH of about 5;

7) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM histidine-acetic acid buffer;
about 0.4 mg/mL polysorbate 80;
about 8% w/v sucrose;
wherein the pharmaceutical composition has a pH of about 5.5;

8) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM histidine-acetic acid buffer;
about 0.8 mg/mL polysorbate 80;
about 8% w/v sucrose;
wherein the pharmaceutical composition has a pH of about 5;

9) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM histidine-acetic acid buffer;
about 0.8 mg/mL polysorbate 80;
about 8% w/v sucrose;
wherein the pharmaceutical composition has a pH of about 5.5;

10) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM citrate buffer, e.g., a citric acid-disodium citrate buffer;
about 0.4 mg/mL polysorbate 80;
about 8% w/v sucrose;
wherein the pharmaceutical composition has a pH of about 5;

11) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM citrate buffer, e.g., a citric acid-disodium citrate buffer;
about 0.4 mg/mL polysorbate 80;
about 8% w/v sucrose;
wherein the pharmaceutical composition has a pH of about 5.5;

12) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM citrate buffer, e.g., a citric acid-disodium citrate buffer;
about 0.8 mg/mL polysorbate 80;
about 8% w/v sucrose;
wherein the pharmaceutical composition has a pH of about 5;

13) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL PD-1/PVRIG/TIGIT-binding protein, e.g., about 10 mg/mL, about 50 mg/mL, about 100 mg/mL, about 130 mg/mL, or about 150 mg/mL PD-1/PVRIG/TIGIT-binding protein;

about 10 mM citrate buffer, e.g., a citric acid-disodium citrate buffer;
about 0.8 mg/mL polysorbate 80;
about 8% w/v sucrose;
wherein the pharmaceutical composition has a pH of about 5.5;

14) the pharmaceutical composition in 1)-13), wherein the pharmaceutical composition further comprises 20 mM, 50 mM, or 100 mM arginine or a hydrochloride thereof;

15) the pharmaceutical composition in 1)-14), wherein the pharmaceutical composition further comprises hydrochloric acid and/or sodium hydroxide;

16) the pharmaceutical compositions in 1)-15) with a final volume of 1 mL, the volume being brought to 1 mL by using water for injection when required.

The pharmaceutical composition of the present disclosure already has sufficient stability for being prepared into a drug and can be stable after long-term storage.

In some embodiments, the pharmaceutical composition remains stable at 2-8 °C for at least 3 months, at least 6 months, at least 12 months, at least 18 months, at least 24 months, or at least 36 months. In some embodiments, the pharmaceutical composition remains stable at 25 °C for at least 3 months, at least 6 months, at least 12 months, at least 18 months, or at least 24 months. In some embodiments, the pharmaceutical composition remains stable at 40 °C for at least 7 days, at least 14 days, at least 28 days, at least 1 month, at least 3 months, at least 6 months, at least 12 months, at least 18 months, or at least 24 months.

The present disclosure provides a method for preparing the aforementioned pharmaceutical composition, which comprises a step of dissolving the PD-1/PVRIG/TIGIT-binding protein.

The pharmaceutical composition of the present disclosure may be further prepared into a freeze-dried formulation for convenience of drug delivery.

In certain embodiments, provided is the pharmaceutical composition according to any one of the above, wherein the pharmaceutical composition is a liquid formulation. In some embodiments, the solvent of the liquid formulation is water, normal saline, or glucose solution.

The present disclosure further provides a freeze-dried formulation, wherein the freeze-dried formulation is capable of forming the pharmaceutical composition according to any one of the above upon reconstitution.

The present disclosure further provides a freeze-dried formulation, wherein the freeze-dried formulation is obtained by lyophilizing the pharmaceutical composition according to any one of the above.

The present disclosure provides a reconstituted solution, wherein the reconstituted solution is prepared by reconstituting the aforementioned freeze-dried formulation. In certain embodiments, the reconstituted solution is selected from the group consisting of, but not limited to, water for injection, normal saline, or glucose solution.

The present disclosure further provides an article of manufacture, which comprises a container containing the aforementioned pharmaceutical composition, the aforementioned freeze-dried formulation, or the aforementioned reconstituted solution. In certain embodiments, the container is a tubular injection vial made of neutral borosilicate glass. In certain embodiments, the article of manufacture comprises a package insert.

The present disclosure further provides the pharmaceutical composition, the freeze-dried formulation, or the reconstituted solution of the freeze-dried formulation, for use in medicaments for treating or alleviating diseases or disorders.

Method for Treating Disease and Pharmaceutical Use

The present disclosure further provides the aforementioned pharmaceutical composition, freeze-dried formulation, or reconstituted solution of the freeze-dried formulation, for use in methods for treating, alleviating, preventing, or diagnosing diseases or disorders.

In some embodiments, provided is a method for ameliorating, alleviating, treating, or preventing a disease, which comprises administering to a subject an amount of the aforementioned pharmaceutical composition, freeze-dried formulation, or reconstituted solution of the freeze-dried formulation that is effective in ameliorating, alleviating, treating, or preventing the disease.

In some embodiments, provided is use of the aforementioned pharmaceutical composition, freeze-dried formulation, or reconstituted solution of the freeze-dried formulation of the present disclosure in the manufacture of a medicament for ameliorating, alleviating, treating, or preventing a disease.

In some embodiments, the aforementioned disease is a proliferative disease or any other disease or disorder characterized by uncontrolled cell growth (e.g., cancer; in the present disclosure, cancer and tumor are used interchangeably), e.g., a disease associated with overexpression of PD-L1 or abnormal expression of PVRIG and TIGIT (e.g., cancer).

In some embodiments, the aforementioned cancer is a solid tumor or hematological tumor.

In some embodiments, the aforementioned cancer is advanced or metastatic.

In some embodiments, the aforementioned cancer is selected from the group consisting of the following or a combination thereof: prostate cancer, liver cancer (HCC), colorectal cancer, ovarian cancer, endometrial cancer, breast cancer, triple-negative breast cancer, pancreatic cancer, stomach/gastric cancer, cervical cancer, head and neck cancer, thyroid cancer, testicular cancer, urothelial cancer, lung cancer (small cell lung cancer or non-small cell lung cancer), melanoma, non-melanoma skin cancer (squamous and basal cell carcinoma), neuroglioma, renal cancer (RCC), lymphoma (NHL or HL), acute myeloid leukemia (AML), T-cell acute lymphoblastic leukemia (T-ALL), diffuse large B-cell lymphoma, testis germ cell tumor, mesothelioma, esophageal cancer, Merkel cell cancer, MSI-high cancer, KRAS-mutant tumor, adult T-cell leukemia/lymphoma, and myelodysplastic syndrome (MDS). For example, the aforementioned cancer is selected from the group consisting of lung cancer, prostate cancer, breast cancer, head and neck cancer, esophageal cancer, gastric cancer, colon cancer, colorectal cancer, bladder cancer, cervical cancer, uterine cancer, ovarian cancer, liver cancer, melanoma, renal cancer, squamous cell carcinoma, hematological cancer, and any other diseases or disorders characterized by uncontrolled cell growth.

In some embodiments, the aforementioned subject has a condition associated with PVRIG and/or TIGIT. In some embodiments, the condition of the subject includes a cancer that expresses or does not express PVRIG and further includes non-metastatic or non-infiltrative and infiltrative or metastatic cancer, wherein PVRIG expression of immune cells, stromal cells, or diseased cells inhibits an anti-tumor response and an anti-infiltration immune response. The methods of the present disclosure are suitable for treating vascularized cancers, for example.

In some embodiments, provided is a method for treating or preventing an infection or sepsis in a subject, which comprises administering to the subject a therapeutically or prophylactically effective amount of the aforementioned pharmaceutical composition, freeze-dried formulation, or reconstituted solution of the freeze-dried formulation. In some embodiments, the infection is a pathogen infection characterized by different degrees of dysfunction of a virus-specific T cell response, such as HIV, HCV, or HBV. In some embodiments, the sepsis includes severe sepsis, septic shock, systemic inflammatory response syndrome (SIRS), bacteremia, septicemia, toxemia and septic syndrome.

In some embodiments, provided are a method for activating cytotoxic T lymphocytes (CTLs) in a subject, a method for activating NK cells in a subject, a method for activating γδ T cells in a subject, a method for activating Th1 cells in a subject, a method for activating, reducing, or eliminating cell number and/or activity of at least one of regulatory T cells (Tregs) in a subject, a method for increasing IFN-γ production and/or proinflammatory cytokine secretion in a subject, a method for inhibiting the interaction between PVRIG and PVRL2 in a subject, and a method for blocking or inhibiting the interaction between PD-1 and PD-L1/PD-L2 in a subject, all of which comprise administering to the subject an effective amount of the aforementioned pharmaceutical composition, freeze-dried formulation, or reconstituted solution of the freeze-dried formulation.

The pharmaceutical composition comprising the PD-1/PVRIG/TIGIT-binding protein of the present disclosure can be used for treating patients in need of such treatment by parenteral administration. For the parenteral routes of administration, subcutaneous injection, intramuscular injection, or intravenous injection may be selected.

## BRIEF DESCRIPTION OF THE DRAWINGS

FIGs. 1A to 1B show the activity of anti-PD-1 nanobodies as measured by a PD-1/PD-L1 NFAT reporter gene system. Pembrolizumab is used as a positive control and hIgG4 as a negative control; FIG. 1A shows the results for A6_IgG4 and A17_IgG4. FIG. 1B shows the results for A17h1_IgG4, A17m09_hIgG4, A17m0901_hIgG4, A17m0902_hIgG4, A17m0903_hIgG4, and A17m0905_hIgG4.

FIG. 2 shows the measurement, through the release of cytokines in a DC:T cell mix lymphocyte reaction, of the extent to which A17m09_hIgG4 activates T cells, wherein pembrolizumab is used as a positive control and hIgG4 as a negative control.

FIGs. 3A to 3B show the *in vivo* efficacy of A17m09_hIgG4 in inhibiting the growth of mouse MC38 colon cancer tumors; FIG. 3A is a graph showing the mouse tumor volume; FIG. 3B is a graph showing the mouse body weight; wherein pembrolizumab is used as a positive control and PBS as a negative control.

FIG. 4 shows the results of a mixed lymphocyte reaction (MLR) experiment for anti-PVRIG nanobodies 30 and 151, wherein Tab5 is used as a positive control and hIgG4 as a negative control.

FIG. 5 shows the results of an MLR experiment for the anti-PVRIG/TIGIT bispecific antibody 1708-151H8, wherein 151H8, 1708, hIgG4, Tab5, and pembrolizumab are used as controls.

FIGs. 6A to 6B show the anti-tumor effects of the anti-PVRIG/TIGIT bispecific antibody 1708-151 and a combination of 1708 and 151 on a mouse subcutaneous xenograft tumor model of human melanoma A375 mixed with human PBMCs. FIG. 6B is a corresponding graph showing the mouse body weight.

FIGs. 7A to 7B show the anti-tumor effects of the anti-PVRIG/TIGIT bispecific antibodies 1708-151H7 and 1708-30H2 on a mouse subcutaneous xenograft tumor model of human melanoma A375 mixed with human PBMCs. FIG. 7B is a corresponding graph showing the mouse body weight.

FIG. 8 is a schematic diagram of 4 antibody configurations of anti-PD-1/PVRIG/TIGIT trispecific antibodies.

FIG. 9 shows the results of measuring the binding of A17h1-1708-151H7 antibodies of 4 configurations to TIGIT antigen.

FIG. 10 shows the results of measuring the binding of A17h1-1708-151H7 antibodies of 4 configurations to PVRIG antigen.

FIGs. 11A, 11B, and 11C show the abilities of anti-PD-1/PVRIG/TIGIT trispecific antibodies with optimized PVRIG sequences to bind to cells overexpressing PVRIG, TIGIT, and PD-1 antigens, respectively.

FIG. 12 shows the blocking of PD-L1/PD-1 binding in a PD-1/PD-L1 NFAT reporter gene system by A 17h1-1708-151H7 antibodies of 4 configurations.

FIG. 13 shows the MLR test results for A17h1-1708-151H7 antibodies of 4 configurations.

FIG. 14 shows the results of NK cell killing function experiments for A17h1-1708-151H7 antibodies of 4 configurations.

FIGs. 15A to 15B show an evaluation of the anti-tumor effect of an anti-PD-1/PVRIG/TIGIT trispecific antibody on a mouse subcutaneous xenograft tumor model of human melanoma A375 mixed with human PBMCs responsive to the PD-1 mAb.

FIGs. 16A to 16B show an evaluation of the anti-tumor effect of an anti-PD-1/PVRIG/TIGIT trispecific antibody on a mouse subcutaneous xenograft tumor model of human melanoma A375 mixed with human PBMCs non-responsive to the PD-1 mAb.

## DETAILED DESCRIPTION

I. Terminology

In order to facilitate the understanding of the present disclosure, certain technical and scientific terms are specifically defined below. Unless otherwise explicitly defined herein, all other technical and scientific terms used herein have the meanings generally understood by those of ordinary skill in the art to which the present disclosure belongs. The three-letter and single-letter codes for amino acids used in the present disclosure are as described in J. biol. chem., 243, p3558 (1968).

"Programmed death-1", "programmed cell death-1", "protein PD-1", "PD-1", "PDCD1" and "hPD-1" are used interchangeably and include variants, isoforms, and species homologs of human PD-1, and analogs having at least one common epitope with PD-1. The complete PD-1 sequence can be found under GenBank accession No. U64863. "Programmed death ligand-1 (PD-L1)" is one of two cell surface glycoprotein ligands of PD-1 (the other being PD-L2) that down-regulates T cell activation and cytokine secretion upon binding to PD-1. "PD-L1" as used herein includes human PD-L1 (hPD-L1), variants, isoforms, and interspecies homologs of hPD-L1, and analogs sharing at least one common epitope with hPD-L1. The complete hPD-L1 sequence can be found under GenBank accession No. Q9NZQ7.

"PVRIG", "PVRIG protein" or "PVRIG polypeptide" may optionally include any such protein or a variant, a conjugate or a fragment thereof, including but not limited to known or wild-type PVRIG described herein, as well as any naturally occurring splice variant, amino acid variant or isoform, in particular a soluble extracellular domain (ECD) fragment of PVRIG. ECD is defined herein as described in patent WO2016134333. The complete human PVRIG sequence can be found under GenBank accession No. AAH73861.1.

"TIGIT", "TIGIT protein" or "TIGIT polypeptide" may optionally include any such protein or a variant, a conjugate or a fragment thereof, including (but not limited to) known or wild-type TIGIT described herein, as well as any naturally occurring splice variant, amino acid variant or isoform. The complete TIGIT sequence can be found under GenBank accession No. AAI01289.1.

"Binding to PD-1" refers to the ability to interact with PD-1 or an epitope thereof, wherein the PD-1 or the epitope thereof may be derived from humans. "Binding to PVRIG" refers to the ability to interact with PVRIG or an epitope thereof, wherein the PVRIG or the epitope thereof may be derived from humans. "Binding to TIGIT" refers to the ability to interact with TIGIT or an epitope thereof, wherein the TIGIT or the epitope thereof may be derived from humans. "Antigen-binding site" refers to a discontinuous three-dimensional spatial site on an antigen that is recognized by the antibody or the antigen-binding fragment of the present disclosure.

"PD-1-binding protein" encompasses any protein capable of specifically binding to PD-1 or any molecule capable of specifically binding to its epitopes. The PD-1-binding protein may include an antibody against PD-1 defined in the present disclosure, an antigen-binding fragment thereof, or a conjugate thereof. The PD-1-binding protein also encompasses immunoglobulin superfamily antibodies (IgSF) or CDR-grafted molecules. The "PD-1-binding protein" of the present disclosure may comprise at least one immunoglobulin single variable domain (such as a VHH) that binds to PD-1. In some embodiments, the "PD-1-binding protein" may comprise 2, 3, 4 or more immunoglobulin single variable domains (such as VHHs) that bind to PD-1. The PD-1-binding protein of the present disclosure may also comprise, in addition to the immunoglobulin single variable domain of PD-1, a linker and/or a moiety with effector function, such as a half-life extending moiety (e.g., an immunoglobulin single variable domain that binds to serum albumin), and/or a fusion partner (such as serum albumin) and/or a conjugated polymer (such as PEG) and/or an Fc region. In some embodiments, the "PD-1-binding protein" of the present disclosure also encompasses bispecific/multispecific antibodies comprising immunoglobulins that bind to different antigens (e.g., a first antibody that binds to a first antigen (e.g., PD-1) and a second antibody that binds to a second antigen (e.g., PVRIG), optionally a third specific antibody that binds to a third antigen (e.g., TIGIT), and further optionally a fourth antibody that binds to a fourth antigen). "PD-1-binding protein" encompasses "PD-1/PVRIG-binding protein", "PD-1/TIGIT-binding protein", and "PD-1/PVRIG/TIGIT-binding protein".

"PD-1-binding protein" or "anti-PD-1 antibody" of the present disclosure may comprise one or more effector molecules, for example, in a conjugated manner. The "effector molecule" includes, for example, antineoplastic agents, drugs, toxins, biologically active proteins such as enzymes, other antibody or antibody fragments, synthetic or naturally occurring polymers, nucleic acids, and fragments thereof (such as DNA, RNA, and fragments thereof), radionuclides, particularly radioiodide, radioisotopes, chelated metals, nanoparticles and reporter groups such as fluorescent compounds or compounds which may be detected by NMR or ESR spectroscopy. When the effector molecule is a polymer, it may generally be a synthetic or naturally occurring polymer, for example, an optionally substituted linear or branched polyalkylene, polyalkenylene or polyoxyalkylene polymer or a branched or unbranched polysaccharide, such as a homo-polysaccharide or a hetero-polysaccharide. Specific optional substituents that may be present on the synthetic polymers described above include one or more hydroxy, methyl, or methoxy groups. Specific examples of synthetic polymers include optionally substituted linear or branched poly(ethylene glycol), poly(propylene glycol), poly(vinyl alcohol) or derivatives thereof, in particular optionally substituted poly(ethylene glycol), such as methoxy poly(ethylene glycol) or derivatives thereof. Specific naturally occurring polymers include lactose, amylose, dextran or glycogen or a derivative thereof. In one embodiment, the polymer is albumin or a fragment thereof, e.g., human serum albumin or a fragment thereof. Conjugation of the polymer to the PD-1-binding protein or anti-PD-1 antibody can be achieved by conventional methods.

"Cytokine" is a general term for proteins that are released by a cell population to act on other cells as intercellular mediators. Examples of such cytokines include lymphokines, monokines, chemokines, and traditional polypeptide hormones. Exemplary cytokines include: human IL-2, IFN-y, IL-6, TNF$\alpha$, IL-17 and IL-5.

"Antibody" encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) as long as they exhibit the desired antigen-binding activity. An antibody may refer to an immunoglobulin, which is of a four-peptide-chain structure formed by two heavy chains and two light chains linked by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA, and IgE, with their corresponding heavy chains being $\mu$ chain, $\delta$ chain, $\gamma$ chain, $\alpha$ chain, and $\varepsilon$ chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into $\kappa$ or $\lambda$ chains according to differences in the constant regions. Each of the five classes of Ig may have a $\kappa$ chain or $\lambda$ chain.

In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3.

"Antigen-binding fragment" encompasses a single-chain antibody (i.e., full-length heavy and light chains); a Fab, a modified Fab, a Fab', a modified Fab', a F(ab')2, an Fv, a Fab-Fv, a Fab-dsFv, a single-domain antibody (e.g., VH or VL or VHH), an scFv, a bivalent or trivalent or tetravalent antibody, a Bis-scFv, a diabody, a tribody, a triabody, a tetrabody, and an epitope-binding fragment of any one of the above (see, e.g., Holliger and Hudson, 2005, Nature Biotech. 23(9):1126-1136; Adair and Lawson, 2005, Drug Design Reviews-Online 2(3), 209-217).

Methods for producing and preparing such antigen-binding fragments are well known in the art (see, e.g., Verma et al., 1998, Journal of Immunological Methods, 216, 165-181). Fab-Fv was first disclosed in WO2009/040562, and its disulfide-stabilized form Fab-dsFv was first disclosed in WO2010/035012. The antigen-binding fragment of the present disclosure also includes Fab and Fab' fragments described in WO2005/003169, WO2005/003170, and WO2005/003171. Multivalent antibodies may comprise multiple specificities (e.g., bispecificities) or may be monospecific (see, e.g., WO92/22583 and WO05/113605), and an example of the latter is Tri-Fab (or TFM) described in WO 92/22583.

"Bispecific antibody" encompasses antibodies (including antibodies or antigen-binding fragments thereof, such as single-chain antibodies) capable of specifically binding to two different antigens, or two or at least two different epitopes of the same antigen. Bispecific antibodies of various structures have been disclosed in the prior art. The bispecific antibodies can be classified into IgG-like bispecific antibodies and antibody-fragment-type bispecific antibodies according to the integrity of IgG molecules. The bispecific antibodies can be classified into bivalent, trivalent, tetravalent, or higher-valent bispecific antibodies according to the number of the antigen-binding regions. The bispecific antibodies can be classified into symmetric bispecific antibodies and asymmetric bispecific antibodies according to whether their structures are horizontally symmetric or asymmetric. Among them, bispecific antibodies based on antibody fragments, such as Fab fragments lacking Fc fragments, formed by linking 2 or more Fab fragments in one molecule, have relatively low immunogenicity, small molecular weight, and relatively high tumor tissue permeability, and typical antibody structures of this type include such bispecific antibodies as F(ab)2, scFv-Fab, and (scFv)2-Fab. The IgG-like bispecific antibodies (e.g., antibodies having Fc fragments) have large relative molecular weight, and the Fc fragments facilitate the later purification of the antibodies and increase their solubility and stability, and the Fc portions may further bind to the receptor FcRn and increase the serum half-life of the antibodies. Typical structural models of bispecific antibodies include bispecific antibodies such as KiH, CrossMAb, Triomab quadroma, Fc$\Delta$Adp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, F(ab)$_2$-CrossMAb, IgG-(scFv)$_2$, Bs4Ab, DVD-Ig, Tetravalent-DART-Fc, (scFv)4-Fc, CODV-Ig, mAb2, and F(ab)$_4$-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

"Trispecific antibody" refers to an antibody capable of specifically binding to three or at least three different epitopes, which may be epitopes of different antigens or different epitopes of the same antigen.

For the determination or definition of "CDRs", the deterministic depiction of CDRs and identification of residues of antigen-binding sites can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). Other CDR boundary definitions may not strictly follow one of the above methods, but still overlap with at least a portion of the Kabat CDRs. The boundaries of CDRs may be shortened or lengthened based on predictions or experimental results that a particular residue or group of residues does not significantly affect the antigen binding. As used in the present disclosure, a CDR may refer to a CDR defined by any method known in the art, including combinations of methods. The correspondence between the various numbering schemes is well known to those skilled in the art, and examples are shown in Table 1 below.

Table 1. The relationships between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues of the VL and VH regions of the antibody of the present disclosure accord with the known Kabat numbering scheme in terms of number and positions.

The antibodies of the present disclosure may be polyclonal, monoclonal, xenogenic, allogeneic, syngeneic, or modified forms thereof, with the monoclonal antibodies being especially applicable in various examples. Generally, the antibodies of the present disclosure are recombinant antibodies.

The "recombinant" used herein generally refers to such products as a cell, a nucleic acid, a protein, or a vector, and indicates that the cell, the nucleic acid, the protein or the vector has been modified by the introduction of a heterologous nucleic acid or protein or the alteration of a native nucleic acid or protein. For example, recombinant cells express genes that are not found within the native (non-recombinant) cellular form or express native genes that are abnormally expressed, lowly expressed, or not expressed at all.

"Domain" of a polypeptide or protein refers to a folded protein structure that is capable of maintaining its tertiary structure independently of the rest of the protein. In general, a domain is responsible for a single functional property of a protein, and in many cases may be added, deleted, or transferred to other proteins without loss of functions of the rest of the protein and/or the domain.

"Immunoglobulin domain" refers to a globular region of an antibody chain. Immunoglobulin domains are characterized by their maintenance of the folding feature of the antibody molecule.

"Immunoglobulin variable domain" refers to an immunoglobulin domain substantially consisting of four "framework regions", i.e., "framework region 1" or "FR1", "framework region 2" or "FR2", "framework region 3" or "FR3", and "framework region 4" or "FR4", and three "complementarity determining regions" or "CDRs", i.e., "complementarity determining region 1" or "CDR1", "complementarity determining region 2" or "CDR2", and "complementarity determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Antibody framework (FR)" refers to a portion of a variable domain, which serves as a framework for the antigen-binding loops (CDRs) of the variable domain.

"Immunoglobulin single variable domain" is generally used to refer to an immunoglobulin variable domain (which may be a heavy or light chain domain, including a VH, VHH or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including a VHH, humanized VHH and/or camelized VH, e.g. a camelized human VH), IgNAR, domains, (single-domain) antibodies as VH domains or derived from VH domains (such as dAbs™) and (single-domain) antibodies as VL domains or derived from VL domains (such as dAbs™). Immunoglobulin single variable domains based on and/or derived from heavy chain variable domains (such as VH or VHH domains) are generally preferred. A specific example of an immunoglobulin single variable domain is a "VHH domain" (or "VHH" for short) as defined below.

"VHH domain", also known as heavy chain single-domain antibody, VHH, $V_HH$ domain, VHH antibody fragment, VHH antibody or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). The term "VHH domain" is used to distinguish said VHH from the VHs and VLs present in antibodies of the conventional four-peptide-chain structure. VHH domains specifically bind to epitopes without the need for additional antigen-binding domains (however, in antibodies of the conventional four-peptide-chain structure, epitopes are recognized by the VL domains together with the VH domains). A VHH domain is a small, stable, and efficient antigen recognition unit formed by a single immunoglobulin domain. The terms "heavy-chain single-domain antibody", "VHH domain", "VHH", "$V_{HH}$ domain", "VHH antibody fragment", "VHH antibody", "Nanobody®", and "Nanobody® domain" ("Nanobody" is a trademark of Ablynx N.V., Ghent, Belgium) are used interchangeably. "VHH domains" include, but are not limited to, natural antibodies produced by camelids, antibodies produced by camelids and then humanized, or antibodies obtained by screening with phage display techniques. The total number of amino acid residues in a VHH domain will usually be in the range of 110 to 120, often between 112 and 115. However, it should be noted that smaller and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240 (2000) 185-195; Li et al., J Biol Chem., 287 (2012) 13713-13721; Deffar et al., African Journal of Biotechnology Vol. 8 (12), pp.2645-2652, 17 June, 2009 and WO94/04678.

As is well known in the art for VH domains and for VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes for VHHs include Chothia, IMGT, and AbM.

"Humanized antibody", also known as CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of VHH domains derived from the family Camelidae by replacing one or more amino acid residues in the amino acid sequence of the original VHH sequence with one or more amino acid residues present at the corresponding positions in a VH domain of an antibody of the conventional four-peptide-chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences. In some specific embodiments, the human framework region sequence of IGHV3 may be contained. Another example of "humanization" includes an antibody produced by grafting mouse CDR sequences into a human antibody variable region framework, i.e., a different type of human germline antibody framework sequence. Therefore, the strong antibody variable antibody reaction induced by a large amount of heterologous protein components contained in the chimeric antibody can be overcome. Methods for humanization include, e.g., protein surface amino acid humanization (resurfacing) and universal framework grafting method for antibody humanization (CDR grafting to a universal framework), i.e., "grafting" CDRs onto other "frameworks" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the "VBase" human germline sequence database, as well as in Kabat, E. A. et al., 1991 Sequences of Proteins of Immunological Interest, 5th edition. The humanized antibody of the present disclosure also includes humanized antibodies formed by further affinity maturation of the CDRs by phage display. Additionally, in order to avoid the decrease in activity caused by the decrease in immunogenicity, the framework region sequence in the human antibody variable region can be subjected to minimum reverse mutation or back mutation to maintain activity.

"Affinity-matured" antibody is an antibody that has one or more changes in one or more hypervariable regions (HVRs) that result in an improvement in the affinity of the antibody for the antigen as compared to the parent antibody. For example, "affinity-matured" PD-1-binding protein or PD-1 antibody has one or more changes in one or more CDRs that result in an increase in the affinity for the antigen as compared to its parent antibody. Affinity-matured antibodies can be prepared, for example, by methods known in the art as described below: Marks et al., 1992, Biotechnology 10:779-783 or Barbas et al., 1994, Proc. Nat. Acad. Sci, USA 91:3809-3813; Shier et al., 1995, Gene 169:147-155; Yelton et al., 1995, Immunol. 155:1994-2004; Jackson et al., 1995, J. Immunol. 154(7):3310-9; Hawkins et al., 1992, J. Mol. Biol. 226(3):889896; KS Johnson and RE Hawkins, "Affinity maturation of antibodies using phage display", Oxford University Press 1996.

"Fully human antibody" includes antibodies having variable and constant regions derived from human germline immunoglobulin sequences. The fully human antibody of the present disclosure may include amino acid residues not encoded by human germline immunoglobulin sequences (e.g., mutations introduced by random or site-specific mutagenesis *in vitro* or by somatic mutations *in vivo*). "Fully human antibody" does not include "humanized antibodies".

Generally, "specific binding" or "selective binding" refers to the binding of a binding protein to an epitope on an antigen. The PD-1-binding protein, PD-1/PVRIG-binding protein, PD-1/TIGIT-binding protein, or PD-1/PVRIG/TIGIT-binding protein of the present disclosure will bind to the antigen to be bound (i.e., PD-1, PVRIG, and/or TIGIT) or an epitope thereof with a dissociation constant ($K_D$) of preferably $10^{-7}$ to $10^{-10}$ mol/L (M), more preferably $10^{-8}$ to $10^{-10}$ mol/L, and even more preferably $10^{-9}$ to $10^{-10}$ or less, and/or with an association constant (KA) of at least $10^{-7}$ M, preferably at least $10^{-8}$ M, more preferably at least $10^{-9}$ M, and more preferably at least $10^{-10}$ M, as measured in a Biacore, KinExA, or Fortibio assay. Any $K_D$ value greater than $10^{-4}$ M is generally considered to indicate non-specific binding. Specific binding of a binding protein to an antigen or epitope can be measured in any suitable manner known, including, for example, surface plasmon resonance (SPR) assay, Scatchard assay, and/or competitive binding assay (e.g., radioimmunoassay (RIA), enzyme immunoassay (EIA), and sandwich competitive assay) described in the present disclosure.

"Epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a unique spatial conformation. Methods for determining the binding of an epitope to a given antibody are well known in the art and include an immunoblotting assay, an immunoprecipitation assay, and the like. Methods for determining the spatial conformation of an epitope include techniques in the art and techniques described in the present disclosure, such as X-ray crystallography and two-dimensional nuclear magnetic resonance.

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant ($K_D$). $K_D$ can be determined by measuring the kinetics of complex formation and dissociation by using, e.g., the surface plasmon resonance (SPR) method (Biacore). The rate constants corresponding to the association and dissociation of a monovalent complex are referred to as the association rate constant ka (or kon) and the dissociation rate constant kd (or koff), respectively. $K_D$ is related to ka and kd by the equation $K_D = kd/ka$. The value of the dissociation constant can be determined directly by well-known methods, and calculations can even be performed for complex mixtures by, for example, those methods described in Caceci et al (1984, Byte 9:340-362). For example, $K_D$ can be determined by using a dual filtration nitrocellulose filter binding assay such as that disclosed by Wong & Lohman (1993, Proc. Natl. Acad. Sci. USA 90:5428-5432). Other standard assays for evaluating the ability of an antibody to bind to a target antigen are known in the art and include, for example, ELISA, western blot, RIA, and flow cytometry, as well as other assays exemplified in the present disclosure. The binding kinetics and binding affinity of the antibody can also be evaluated by standard assays known in the art, such as surface plasmon resonance (SPR), e.g., by using the Biacore™ system or KinExA. The binding affinities associated with different molecular interactions, e.g., the binding affinities of different antibodies for a given antigen, can be compared by comparing the $K_D$ values of antibody/antigen complexes. Similarly, the specificity of an interaction can be evaluated by determining and comparing the $K_D$ value for the interaction of interest (e.g., a specific interaction between an antibody and an antigen) with the $K_D$ value for an interaction not of interest (e.g., a control antibody known not to bind to PD-1).

"Conservative replacement" refers to a replacement with another amino acid residue having similar properties to the original amino acid residue. For example, lysine, arginine, and histidine have similar properties in that they have basic side chains, and aspartic acid and glutamic acid have similar properties in that they have acidic side chains. In addition, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not

EP 4 681 729 A1

exhibit a particular change in properties.

"Homology", "identity", or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The homology percentage between two sequences is a function of the number of matching or homologous positions shared by the two sequences divided by the number of positions compared $\times$ 100%. For example, if 6 out of 10 positions are matched or homologous when two sequences are optimally aligned, the two sequences are 60% homologous. In general, when two sequences are aligned, a comparison is performed to obtain the maximum homology percentage.

"Inhibition" and "blocking" are used interchangeably and encompass both partial and complete inhibition/blocking. "Inhibition of growth" (e.g., involving cells) is intended to include any measurable reduction in cell growth.

"Arresting the growth of" or "growth inhibition" refers to inhibiting cell growth or proliferation.

"Proliferative disease" refers to a disorder associated with a certain degree of abnormal cell proliferation. In one embodiment, the proliferative disorder is cancer.

"Tumor" refers to all neoplastic cell growth and proliferation, whether malignant or benign, and all pre-cancerous and cancerous cells and tissues. "Cancer", "cancerous", "proliferative disorder", and "tumor" are not mutually exclusive when referred to in the present disclosure.

"Preventing a cancer" refers to delaying, inhibiting, or preventing the onset of the cancer in a subject in which the onset of oncogenesis or tumorigenesis has not been evidenced but a predisposition toward the cancer has been identified, as determined, for example, by genetic screening or otherwise. The term also encompasses treating a subject having a premalignant disorder to stop the progression of, or cause regression of, the premalignant disorder towards malignancy.

"Optional" or "optionally" means that the event or circumstance subsequently described may, but does not necessarily, occur, and this description includes instances where the event or circumstance occurs or does not occur. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to".

The term "about" or "approximately" as used herein means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Alternatively, "about" or "substantially comprise" may mean a range of up to $\pm$30%; for example, a pH of about 5.5 means a pH of 5.5$\pm$1.65. Furthermore, particularly for biological systems or processes, the term may mean up to an order of magnitude or up to 5-fold of a numerical value. Unless otherwise stated, when a specific value is provided in the present application and claims, the meaning of "about" or "substantially comprise" should be assumed to be within an acceptable error range for that specific value.

"Buffer" refers to a buffer that resists changes in pH by the action of its acid-base conjugate components. Examples of buffers that control the pH in an appropriate range include tris(hydroxymethyl)aminomethane (Tris), acetate, succinate, gluconate, histidine salt, oxalate, lactate, phosphate, citrate, tartrate, fumarate, glycylglycine, and other organic acid buffers.

"Histidine salt buffer" is a buffer comprising histidine ions. Examples of histidine salt buffers include a histidine-hydrochloride buffer, a histidine-acetate buffer, a histidine-phosphate buffer, a histidine-sulfate buffer, and the like, and the histidine-hydrochloride buffer or histidine-acetate buffer is preferred. The histidine-acetate buffer is prepared from histidine and acetic acid, and the histidine-hydrochloride buffer is prepared from histidine and histidine hydrochloride, or histidine and hydrochloric acid.

"Citrate buffer" is a buffer comprising citrate ions. Examples of citrate buffers include citric acid-sodium citrate, citric acid-potassium citrate, citric acid-calcium citrate, citric acid-magnesium citrate, and the like. A preferred citrate buffer is a citric acid-sodium citrate buffer.

"Pharmaceutical composition" refers to a mixture comprising one or more of the antibodies described herein and other chemical components, for example, physiological/pharmaceutically acceptable carriers and excipients. The purpose of the pharmaceutical composition is to maintain the stability of the active ingredient and promote the administration to an organism, which facilitates the absorption of the active ingredient, thereby exerting biological activity.

In the present disclosure, "pharmaceutical composition" and "formulation" are not mutually exclusive.

Unless otherwise specified, the solvent in the pharmaceutical composition described in the present disclosure in solution form is water.

"Freeze-dried formulation" refers to a formulation or a pharmaceutical composition obtained by lyophilizing a pharmaceutical composition or a formulation in liquid or solution form *in vacuo.*

The pharmaceutical composition described in the present disclosure can achieve a stable effect, that is, the PD-1/PVRIG/TIGIT-binding protein in the pharmaceutical composition substantially retains its physical and/or chemical stability and/or biological activity after storage; for example, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical antibody formulation is one in which no significant change is observed under the following conditions: storage at refrigeration temperature (2-8 °C) for at least 3 months, at least 6 months, at least 1 year, at least 2 years, or at most 2 years. In addition, stable liquid formulations include liquid formulations that exhibit desirable features after storage at 25 °C for periods including 1 month, 3 months, and 6 months, or storage at 40 °C for periods including 1 month. Typical acceptable criteria for stability are as follows: typically, no more than about 10%, preferably no more than about 5%, of antibody monomer is degraded as measured by SEC-HPLC. The pharmaceutical antibody formulation is colorless, or clear to slightly opalescent, by visual analysis. The concentration, pH, and osmolality of the formulation have a change of no more than $\pm 10\%$. Typically, clippings of no more than about 10%, preferably no more than about 5%, are observed. Typically, aggregation of no more than about 10%, preferably no more than about 5%, is formed.

An antibody "retains its physical stability" in a pharmaceutical formulation if it shows no significant increase in aggregation, precipitation and/or denaturation upon visual inspection of color and/or clarity, or as determined by UV light scattering, size exclusion chromatography (SEC), and dynamic light scattering (DLS). Changes in protein conformation can be assessed by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed proteins. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

An antibody "retains its biological activity" in a pharmaceutical formulation if the biological activity of the antibody at a given time is within a predetermined range of the biological activity exhibited during the preparation of the pharmaceutical formulation. The biological activity of an antibody can be determined, for example, by an antigen-binding assay.

"Administrating", "giving", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administering", "giving", and "treating" can refer to, for example, therapeutic, pharmacokinetic, diagnostic, research, and experimental methods. The treatment of cells comprises contacting a reagent with cells and contacting the reagent with a fluid, where the fluid is in contact with the cells. "Administering", "giving", and "treating" also refer to treating, e.g., cells by reagents, diagnosis, binding compositions or by another cell *in vitro* and *ex vivo*. "Treating", when applied to humans, veterinary, or research subjects, refers to therapeutic treatment, preventive or prophylactic measures, and research and diagnostic applications.

"Treating" or "treatment" refers to administering a therapeutic agent, such as a therapeutic agent comprising any one of the antibodies of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective to alleviate one or more disease symptoms in the subject or population being treated, whether by inducing regression of such symptoms or inhibiting the development of such symptoms into any clinically measurable degree. The amount of therapeutic agent effective to alleviate any specific disease symptom (also referred to as "therapeutically effective amount") may vary depending on factors such as the disease state, the age, and the body weight of the subject, and the ability of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or products) may be ineffective in alleviating a disease symptom of interest in a certain subject, they shall alleviate the disease symptom of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method and dose of administration, and the severity of side effects. An effective amount may be the maximum dose or administration regimen to avoid significant side effects or toxic effects. The subject of the present disclosure may be an animal or a human subject.

The "subject" or "patient" in the present disclosure means a mammal, particularly a primate, and especially a human.

**The equipment and methods used in the detection are shown below:**

**Appearance:**

A visual method was used. The sample vial was wiped clean, and the color, clarity, and visible foreign matter of the sample were observed on a clarity tester under a white background and a black background at an illumination intensity of 1000-1500 lx.

Instrument for appearance examination: Jingtuo Instrument YB-2A clarity analyzer.

**SEC molecular exclusion chromatography:**

This is an analytical method that separates a solute based on the relative relationship between the pore size of the gel pores and the coil size of the polymer sample molecule. SEC monomer content percentage = A monomer/A total $\times$ 100% (A monomer represents the peak area of the main peak monomer in the sample, and A total represents the sum of all peak areas).

Instrument for SEC determination: Agilent 1260-Bio; chromatographic column: Waters, XBrige BEH200Å SEC (300 $\times$ 7.8 mm, 3.5 $\mu$m)

**NR-CE capillary gel electrophoresis:**

This is a type of electrophoresis in which a gel is moved into a capillary as a support medium and a method that achieves separation under a certain voltage based on the molecular weight of the sample.

Non-reduced CE purity percentage = A main peak/A total $\times$ 100% (A main peak represents the peak area of the main peak in the sample, and A total represents the sum of all peak areas).

Instrument for CE determination: Sciex model PA800 plus.

**icIEF imaged capillary isoelectric focusing electrophoresis:**

This is a technique for separation according to the difference of isoelectric points (pI) of proteins.

**icIEF** main peak content percentage = main peak area/total area $\times$ 100% (total area represents the sum of areas of acidic, main, and basic peaks).

Manufacturer of instrument for **icIEF** determination: Protein Simple, model: Muarice.

**Protein concentration determination:**

Instrument for protein concentration determination: ultraviolet-visible spectrophotometer; model: Nano Drop 2000; optical path length: 1 mm.

**Exemplary antibody pharmaceutical composition (formulation) preparation process**

Step 1: A PD-1/PVRIG/TIGIT-binding protein was mixed with the following formula amounts of excipients to prepare a stock solution of a formulation containing the PD-1/PVRIG/TIGIT-binding protein. The stock solution was filtered, subjected to in-process control sampling, and then analyzed for sterility. The stock solution was filtered through a 0.22 $\mu$m filter, and the filtrate was collected.

Step 2: The filling amount was adjusted to 1.15 mL, the filtrate was filled into 2 mL vials, stoppers were applied, and in-process control samplings were performed at the beginning, in the middle, and at the end of filling to detect the difference of filling volume.

Step 3: The capping machine was started, aluminum caps were put on, and capping was carried out.

Step 4: Visual inspection was performed to confirm that the products have no defects, such as inaccurate filling amount. Vial labels were printed and put on the vials; carton labels were printed, cartons were folded, packing was performed, and the carton labels were put on the cartons.

## II. Examples and Test Examples

The present disclosure is further illustrated in detail by the following examples. These examples are for illustrative purposes only and are not intended to limit the scope of the present disclosure.

Experimental methods without specific conditions indicated in the examples of the present disclosure were generally conducted under conventional conditions or conditions recommended by the manufacturer of the starting materials or commercial products. Reagents without specific origins indicated were commercially available conventional reagents.

## Examples

The preparation and purification methods for the PD-1/PVRIG/TIGIT-binding protein in the present application are described in Patent Application No. WO2023040945, which is incorporated herein by reference in its entirety.

## Example 1. Screening, Preparation, and Functional Verification of Anti-PD-1 Nanobodies

In this example, His-tagged human PD-1 was used as an immunizing antigen, and biotinylated human PD-1 and cynomolgus monkey PD-1 as screening antigens; the antigens were all purchased from AcroBiosystems.

## Example 1-1. Screening and Preparation of Anti-PD-1 Nanobodies

1. Sequences and preparation of immunizing and screening antigens

Table 2. PD-1 antigens for immunizing alpacas and screening

| Antigen | Cat. No. | Starting and ending amino acids | Accession No. |
|---|---|---|---|
| Human PD-1 protein (His Tag) | PD1-H5221 | Leu25-Gln167 | # NP_005009.2 |
| Biotinylated human PD-1 protein (Avitag -His Tag) | PD1-H82E4 | | # Q15116-1 |
| Biotinylated monkey PD-1 protein (Avitag -His Tag) | PD1-C82E6 | | # B0LAJ3 |

The sequence of human PD-1 protein (NP_005009.2) is shown below, with the sequence of the extracellular region underlined-it starts with the amino acid Leu25 and ends with the amino acid Gln167.

> Human PD-1 amino acid sequence

MQIPQAPWPVVWAVLQLGWRPGWF<u>LDSPDRPWNPPTFSPALLVVTEGDNATFT
CSFSNTSESFVLNWYRMSPSNQTDKLAAFPEDRSQPGQDCRFRVTQLPNGRDFH
MSVVRARRNDSGTYLCGAISLAPKAQIKESLRAELRVTERRAEVPTAHPSPSPRP
AGQFQ</u>TLVVGVVGGLLGSLVLLVWVLAVICSRAARGTIGARRTGQPLKEDPSAV
PVFSVDYGELDFQWREKTPEPPVPCVPEQTEYATIVFPSGMGTSSPARRGSADGP
RSAQPLRPEDGHCSWPL(SEQ ID NO: 1).

2. Alpaca immunization, nanobody yeast display library construction, and antibody screening

1) Alpaca immunization

Two healthy alpacas were immunized. On day 0, the first immunization was performed: 0.25 mg of human PD-1 antigen was well mixed with 1 mL of Freund's complete adjuvant (CFA), and the mixture was injected subcutaneously. On days 21, 42, and 63, 0.125 mg of human PD-1 antigen was well mixed with 1 mL of Freund's incomplete adjuvant (IFA), and the mixture was injected subcutaneously. 10 mL, 50 mL, and 50 mL of blood were collected on day 28, day 49, and day 70, respectively. The serum titer was measured, and alpaca peripheral lymphocytes up to the library construction standard were used to construct a nanobody yeast display library.

2) Yeast library construction

After the third and fourth immunizations of the 2 alpacas, 50 mL peripheral blood samples were taken. PBMCs were separated from the peripheral blood samples, and RNA was extracted from the PBMCs and reverse-transcribed to obtain total cDNA. The cDNA was used as a template for the first round of nested PCR. After two rounds of nested PCR amplification, the fragment products of the second round of nested PCR were ligated to a yeast library vector (the yeast library vector pYDN3), and the ligation products were introduced into yeast competent cells by electroporation. Colony PCR was used to verify the insertion rate and library diversity. The results of analysis based on the number of library transformants, the library insertion rate, and the library diversity and sequencing show that one alpaca's library capacity was $9.28 \times 10^7$ and the other alpaca's was $1.54 \times 10^8$.

3) Nanobody (VHH) screening

The yeast display system was screened for antibodies using flow cytometry (FACS) sorting, library screening, and identification. Two rounds of sorting were performed. In the first round, enrichment was performed using biotinylated human PD-1 protein (Avitag -His Tag) magnetic beads. In the second round, sorting was performed using a biotinylated monkey PD-1 protein (His, Avitag) flow cytometry sorter (flow cytometry antibody: mouse anti-HA tag Dylight 488; streptavidin Dylight 650). After the two rounds of sorting, monoclonal identification, sequencing, and sequence analysis were performed, and 18 unique VHH sequences that cross-bind to human and monkey PD-1 antigens were obtained. The sequence A17 among them is shown below.
> A17 variable region

EVQVVESGGGLVQPGGSLRLSCVASGLTFS<u>DYSMS</u>WYRQAPGKERELVA<u>IISGS</u>
<u>GVIAHYVDSVKG</u>RFTISRDNAKSTVYLQMVSLKPEDRGVYYCRA<u>VSDWDDY</u>W
GQGTQVTVSS(SEQ ID NO: 2).

Table 3. The CDRs of the anti-PD-1 nanobody (Kabat numbering scheme)

| No. | CDR sequences | |
|-----|------|---------------------------|
| A17 | CDR1 | DYSMS(SEQ ID NO:3) |
| | CDR2 | IISGSGVIAHYVDSVKG (SEQ ID NO:4) |
| | CDR3 | VSDWDDY (SEQ ID NO:5) |

The sequence described above was linked to a human IgG4 Fc (comprising a hinge region and S228P, according to the Eu numbering scheme) fragment to construct a VHH-Fc antibody. Plasmids were constructed and transiently transfected into HEK293 cells. The antibody was expressed and purified using a protein A column. The column was washed with PBS, and elution was performed with a 0.1 M glycine buffer, pH 2.5. The antibody was transferred to a pH 7.4 PBS buffer by dialysis. The sequences of the human IgG4 Fc (comprising a hinge region) and the VHH-Fc antibody based on A17 are as follows:

>hIgG4

ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGK
(SEQ ID NO: 6);

>A17_hIgG4

EVQVVESGGGLVQPGGSLRLSCVASGLTFS<u>DYSMS</u>WYRQAPGKERELVA<u>IISGS</u>
<u>GVIAHYVDSVKG</u>RFTISRDNAKSTVYLQMVSLKPEDRGVYYCRA<u>VSDWDDY</u>W
GQGTQVTVSSESKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVF
SCSVMHEALHNHYTQKSLSLSLGK
(SEQ ID NO: 7).

**Example 1-2. Verification of Affinity of Anti-PD-1 Nanobody for Antigen PD-1**

1. ELISA

The human PD-1 protein (his tag) was dissolved in PBS to form a 1 $\mu$g/mL solution, and the solution was added to a 96-well plate at 100 $\mu$L/well. The plate was coated with the antigen overnight at 4 °C. The plate was washed with a PBST (PBS + 0.05% tween20) solution three times. PBST/1% BSA solution was added, and the plate was incubated at 37 °C for 1 h for blocking. After three washes with PBST solution, different concentrations of the candidate PD-1 VHH-Fc antibodies (diluted with PBST/1% BSA solution) were added, and the plate was incubated at 37 °C for 1.5 h. The plate was washed with a PBST solution three times. 100 $\mu$L of HRP-conjugated anti-human IgG4 Fc secondary antibody (Thermo) solution was added, and the plate was incubated at 37 °C for 1 h. After three washes with PBST solution, TMB solution was added at 100 $\mu$L/well. After 5 min of reaction at room temperature, 50 $\mu$L of stop solution was added. Then 450 nM values were measured using a microplate reader, and $EC_{50}$ was calculated. According to the results shown in Table 4, A17_hIgG4 has relatively strong binding affinity for PD-1 antigen.

Table 4. The $EC_{50}$ values of anti-PD-1 nanobody measured by ELISA

| Antibody No. | $EC_{50}$ (nM) |
|---|---|
| A17_hIgG4 | 0.06099 |

2. FACS

To measure the ability of the anti-PD-1 nanobody to bind to PD-1 expressed on the cell membrane, different concentrations of the candidate anti-PD-1 VHH-Fc antibodies were added to $10^5$/well Jurkat cells overexpressing human PD-1 (Cat: J1250, Promega). The plate was incubated at room temperature for 20 min and then washed with PBS twice. 100 $\mu$L of anti-human IgG Fc fluorescent secondary antibody (Biolegend) was added. The plate was incubated at room temperature for 20 min and then washed with PBS twice, and the cells were resuspended in 250 $\mu$L of PBS. Fluorescence signals were detected by FACS, and $EC_{50}$ was calculated.

Pembrolizumab (purchased from Biointron) was used as a control. The results are shown in Table 5. The $EC_{50}$ value of A17_hIgG4 is 0.3568 nM, which is significantly better than those of the other antibodies obtained from the same screening (results not shown).

Table 5. The $EC_{50}$ values of anti-PD-1 nanobodies binding to cell surface PD-1 measured by FACS

| Antibody No. | $EC_{50}$ (nM) |
|---|---|
| A17_hIgG4 | 0.3568 |
| Pembrolizumab | 0.2169 |

**Example 1-3. Modification of Anti-PD-1 Nanobody Sequences**

1. Humanization and back mutation of anti-PD-1 nanobodies

Antibody humanization was performed by CDR-grafting. Through the website of IMGT or NCBI, the parent anti-PD-1 antibody was aligned to fully human germline genes in the IMGT or NCBI/igblast database, and the human germline gene IGHV 3-23 (IGHV3-23*01; IGHV3-23*04) that was highly homologous with the PD-1 nanobody was selected as a humanization template. CDRs were grafted, 4 key residues (Y37, E44, R45, and L47) in FR2 of the nanobody were kept, and the key core residues close to the CDRs and the Vernier zone residues that interact with the CDRs were back-mutated; humanized antibodies A17h1, A17h2, A17h3, and A6h1 were obtained.

> A17h1 variable region

EVQLQESGGGLVQPGGSLRLSCTASGLTFS<u>DYSMS</u>WYRQAPGKGRELVA<u>IISGSG</u>
<u>VIAHYVDS</u>VKGRFTISRDNSKNTVYLQMRSLTPEDRAVYYCRA<u>VSDWDDY</u>WG
QGTQVTVSS
(SEQ ID NO: 8);

> A17h2 variable region

EVQLVESGGGLVQPGGSLRLSCAASGLTFS<u>DYSMS</u>WVRQAPGKGLEWVA<u>IISGS</u>
<u>GVIAHYVDS</u>VKGRFTISRDNSKNTVYLQMNSLRAEDTAVYYCRA<u>VSDWDDY</u>W
GQGTQVTVSS(SEQ ID NO: 9);

> A17h3 variable region

EVQLVESGGGLVQPGGSLRLSCAASGLTFS<u>DYSMS</u>WVRQAPGKGLEWVA<u>IISGS</u>
<u>GVIAHYVDS</u>VKGRFTISRDNSKNTVYLQMNSLRAEDTAVYYCRA<u>VSDWDEY</u>W
GQGTQVTVSS(SEQ ID NO: 10).

2. TCE (T cell epitope)-removing, antibody deamidation-reducing, and antibody isomerization-reducing modifications

To reduce the potential immunogenic risk of the antibodies, TCE-removing modifications were performed. To improve the druggability of the antibodies, antibody deamidation and antibody isomerization site modifications were performed. The following sequences were obtained:

> A 17m01 variable region

EVQLQESGGGLVQPGGSLRLSCTASGLTFS<u>DYSMS</u>WYRQAPGKGRELVA<u>IISGSG</u>
<u>VIAHYVDS</u>VKGRFTISRDNAKNTVYLQMRSLTPEDRAVYYCRA<u>VSDWDDY</u>WG
QGTQVTVSS(SEQ ID NO: 11)

> A17m02 variable region

EVQLQESGGGLVQPGGSLRLSCTASGLTFS<u>DYSMS</u>WYRQAPGKGRELVA<u>IISGSG</u>
<u>VITHYVDS</u>VKGRFTISRDNSKNTVYLQMRSLTPEDRAVYYCRA<u>VSDWDDY</u>WG
QGTQVTVSS(SEQ ID NO: 12)

> A17m03 variable region

EVQLQESGGGLVQPGGSLRLSCTASGLTFS<u>DYSMS</u>WYRQAPGKGRELVA<u>IISGSG</u><u>GIAHYVDSVKG</u>RFTISRDNSKNTVYLQMRSLTPEDRAVYYCRA<u>VSDWDDY</u>WGQGTQVTVSS(SEQ ID NO: 13)

> A17m04 variable region

EVQLQESGGGLVQPGGSLRLSCTASGLTFS<u>DYSMS</u>WYRQAPGKGRELVA<u>IISGSG</u><u>VSAHYVDSVKG</u>RFTISRDNSKNTVYLQMRSLTPEDRAVYYCRA<u>VSDWDDY</u>WGQGTQVTVSS(SEQ ID NO: 14)

> A17m05 variable region

EVQLQESGGGLVQPGGSLRLSCTASGLTFS<u>DYSMS</u>WYRQAPGKGRELVA<u>IISGSG</u><u>VIAHYVDSVKG</u>RFTISRDNSKNTVYLQMRSLTPEDRAVYYCRA<u>VSDWEDY</u>WGQGTQVTVSS(SEQ ID NO: 15)

> A17m06 variable region

EVQLQESGGGLVQPGGSLRLSCTASGLTFS<u>DYSMS</u>WYRQAPGKGRELVA<u>IISGSG</u><u>VIAHYVDSVKG</u>RFTISRDNSKNTVYLQMRSLTPEDRAVYYCRA<u>VSDWDEY</u>WGQGTQVTVSS(SEQ ID NO: 16)

> A17m07 variable region

EVQLQESGGGLVQPGGSLRLSCTASGLTFS<u>DYSMS</u>WYRQAPGKGRELVA<u>IISGSG</u><u>VIAHYVDSVKG</u>RFTISRDNAKNTVYLQMRSLTPEDRAVYYCRA<u>VSDWEDY</u>WGQGTQVTVSS(SEQ ID NO: 17)

> A17m08 variable region

EVQLQESGGGLVQPGGSLRLSCTASGLTFS<u>DYSMS</u>WYRQAPGKGRELVA<u>IISGSG</u><u>VIAHYVDSVKG</u>RFTISRDNAKNTVYLQMRSLTPEDRAVYYCRA<u>VSDWDEY</u>WGQGTQVTVSS(SEQ ID NO: 18)

> A17m09 variable region

EVQLQESGGGLVQPGGSLRLSCTASGLTFS<u>DYSMS</u>WYRQAPGKGRELVA<u>IISGSG</u><u>VITHYVDSVKG</u>RFTISRDNAKNTVYLQMRSLTPEDRAVYYCRA<u>VSDWEDY</u>WGQGTQVTVSS(SEQ ID NO: 19)

> A17m10 variable region

EVQLQESGGGLVQPGGSLRLSCTASGLTFS<u>DYSMS</u>WYRQAPGKGRELVA<u>IISGSG</u><u>VITHYVDSVKG</u>RFTISRDNAKNTVYLQMRSLTPEDRAVYYCRA<u>VSDWDEY</u>WGQGTQVTVSS(SEQ ID NO: 20)

> A17m11 variable region

EVQLQESGGGLVQPGGSLRLSCTASGLTFS<u>DYSMS</u>WYRQAPGKGRELVA<u>IISGSG
GIAHYVDSVKG</u>RFTISRDNAKNTVYLQMRSLTPEDRAVYYCRA<u>VSDWEDY</u>WG
QGTQVTVSS(SEQ ID NO: 21)

> A17m12 variable region

EVQLQESGGGLVQPGGSLRLSCTASGLTFS<u>DYSMS</u>WYRQAPGKGRELVA<u>IISGSG
GIAHYVDSVKG</u>RFTISRDNAKNTVYLQMRSLTPEDRAVYYCRA<u>VSDWDEY</u>WG
QGTQVTVSS(SEQ ID NO: 22)

> A17m13 variable region

EVQLQESGGGLVQPGGSLRLSCTASGLTFS<u>DYSMS</u>WYRQAPGKGRELVA<u>IISGSG
VSAHYVDSVKG</u>RFTISRDNAKNTVYLQMRSLTPEDRAVYYCRA<u>VSDWEDY</u>WG
QGTQVTVSS(SEQ ID NO: 23)

> A17m14 variable region

EVQLQESGGGLVQPGGSLRLSCTASGLTFS<u>DYSMS</u>WYRQAPGKGRELVA<u>IISGSG
VSAHYVDSVKG</u>RFTISRDNAKNTVYLQMRSLTPEDRAVYYCRA<u>VSDWDEY</u>WG
QGTQVTVSS(SEQ ID NO: 24)

> A17m15 variable region

EVQLQESGGGLVQPGGSLRLSCTASGLTFS<u>DYSMS</u>WYRQAPGKGRELVA<u>IISGSG
GITHYVDSVKG</u>RFTISRDNAKNTVYLQMRSLTPEDRAVYYCRA<u>VSDWEDY</u>WG
QGTQVTVSS(SEQ ID NO: 25).

Moreover, A17m09 was selected, and the antibody sequence was further subjected to a germlining modification to improve the degree of humanization; the following sequences were obtained:

> A 17m0901 variable region

EVQLVESGGGLVQPGGSLRLSCAASGFTFS<u>DYSMS</u>WYRQAPGKGRELVA<u>IISGS
GVITHYVDSVKG</u>RFTISRDNAKNTLYLQMRSLRAEDTAVYYCRA<u>VSDWEDY</u>W
GQGTQVTVSS(SEQ ID NO: 26)

> A17m0902 variable region

EVQLVESGGGLVQPGGSLRLSCAASGLTFS<u>DYSMS</u>WYRQAPGKGRELVA<u>IISGS
GVITHYVDSVKG</u>RFTISRDNAKNTLYLQMRSLRAEDTAVYYCRA<u>VSDWEDY</u>W
GQGTQVTVSS(SEQ ID NO: 27)

> A17m0903 variable region

EVQLVESGGGGLVQPGGSLRLSCAASGLTFS<u>DYSMS</u>WYRQAPGKGRELVA<u>IISGS GVITHYVDSVKG</u>RFTISRDNAKNTVYLQMRSLRAEDTAVYYCRA<u>VSDWEDY</u>W GQGTQVTVSS(SEQ ID NO: 28)

> A17m0905 variable region

EVQLVESGGGGLVQPGGSLRLSCAASGLTFS<u>DYSMS</u>WYRQAPGKGRELVA<u>IISGS GVITHYVDSVKG</u>RFTISRDNAKNTVYLQMRSLRAEDRAVYYCRA<u>VSDWEDY</u>W GQGTQVTVSS(SEQ ID NO: 29).

That is, A17 of the present disclosure has the following general sequence:

CDR1: DYSMS (SEQ ID NO: 3)
CDR2: IISGSGX$_1$X$_2$X$_3$HYVDSVKG, wherein X$_1$ is selected from the group consisting of V and G; X$_2$ is selected from the group consisting of I and S; and X$_3$ is selected from the group consisting of T and A (SEQ ID NO: 30)
CDR3: VSDWX$_4$X$_5$Y, wherein X$_4$ is selected from the group consisting of D and E, and X$_5$ is selected from the group consisting of D and E (SEQ ID NO: 31).

Specifically, the CDR2 may be:

IISGSGVIAHYVDSVKG (SEQ ID NO: 4)
IISGSGVITHYVDSVKG (SEQ ID NO: 32)
IISGSGGIAHYVDSVKG (SEQ ID NO: 33)
IISGSGVSAHYVDSVKG (SEQ ID NO: 34)
IISGSGGITHYVDSVKG (SEQ ID NO: 79)

Specifically, the CDR3 may be:

VSDWDDY (SEQ ID NO: 5)
VSDWEDY (SEQ ID NO: 35)
VSDWDEY (SEQ ID NO: 36).

**Example 1-4. PD-1/PD-L1 Reporter Gene System Test of Blocking of PD-L1 binding to PD-1 by Anti-PD-1 Nanobodies**

The biological activity function of the anti-PD-1 antibodies at the cellular level can be measured using the PD-1/PD-L1 NFAT gene reporter system (Cat: J1250, Promega). The system consists of two genetically engineered cell lines: a PD-1-overexpressing effector cell Jurkat (containing NFAT reporter gene luciferase) and a PD-LI-overexpressing antigen-presenting cell CHO-K1 cell. When the two cells are co-cultured, the interaction of PD-1/PD-L1 inhibits TCR-mediated NFAT activation and thereby inhibits the expression of NFAT reporter gene luciferase. When the PD-1/PD-L1 interaction is disrupted, TCR activation induces luciferase expression through the NFAT pathway. By adding Bio-Glo reagent and using a luminometer to quantitatively measure fluorescence, the extent of effector cell activation and the biological activity of an antibody can be measured.

CHO-K1 cells overexpressing PD-L1 were diluted to $4 \times 10^5$/mL and added to a 96-well plate at 100 $\mu$L/well, and the plate was incubated overnight. After the medium was pipetted off, 40 $\mu$L of $1.25 \times 10^6$/mL PD-1 Jurkat cells was added rapidly, along with 40 $\mu$L of anti-PD-1 nanobody solutions (diluted with 1640 + 2% FBS solution) with different concentrations. The plate was incubated at 37 °C for 6 h and cooled to room temperature. Bright-glo reagent (Cat: E2620, promega) was added at 40 $\mu$L/well. The plate was shaken in the dark at 350 rpm for 5 min and left to stand in the dark at room temperature for 5 min. Then fluorescence values were measured using a microplate reader, and IC$_{50}$ was calculated.

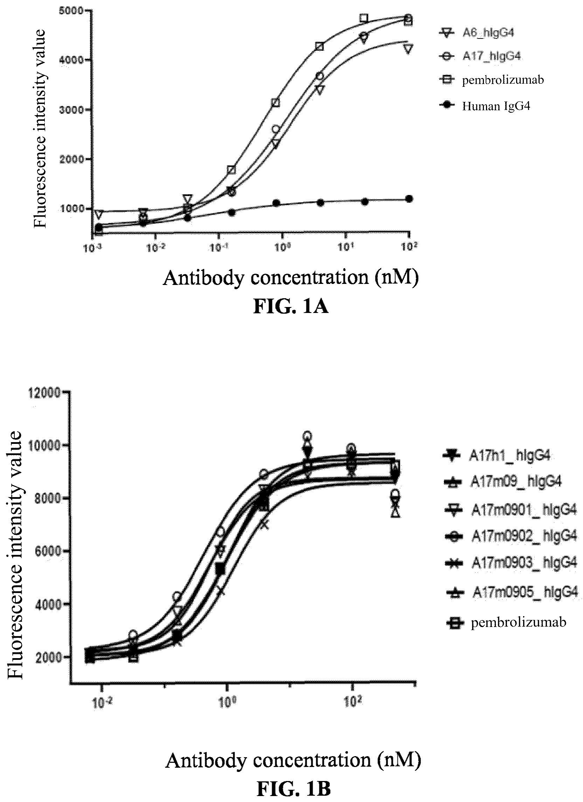

Pembrolizumab and hIgG4 isotype were used as controls. As shown in FIG. 1A and Table 6, the biologically functional activity IC$_{50}$ value of A17_hIgG4 is 1.199 nM, which is significantly better than those of the other antibodies obtained from the same screening in the present disclosure (for example, 7.62 for A3_hIgG4, and 3.208 for A13_hIgG4; the sequences of A3_hIgG4 and A13_hIgG4 are not shown). A6_hIgG4 was another antibody obtained by screening in the present application.

Table 6. The activity $IC_{50}$ values of the anti-PD-1 nanobodies, as measured using the PD-1/PD-L1 NFAT reporter gene system

| Antibody No. | $IC_{50}$ (nM) |
|---|---|
| A17_hIgG4 | 1.199 |
| Pembrolizumab | 0.5126 |
| hIgG4 | - |

Using the above method, the humanized anti-PD-1 nanobodies were functionally verified. Referring to FIG. 1B and Table 7, the $IC_{50}$ activity of A17h1_hIgG4 is similar to that of the positive antibody pembrolizumab. Among the candidate molecules with modified sequences, the antibodies A17m0901_hIgG4 and A17m0902_hIgG4 have biological activity $IC_{50}$ values of 0.5307 nM and 0.4352 nM, respectively, which are better than that of pembrolizumab.

Table 7. The activity $IC_{50}$ of the modified anti-PD-1 nanobodies, as measured using the PD-1/PD-L1 NFAT reporter gene system

| Antibody No. | $IC_{50}$ (nM) |
|---|---|
| A17h1_hIgG4 | 1.039 |
| A17m09_hIgG4 | 1.083 |
| A17m0901_hIgG4 | 0.5307 |
| A17m0902_hIgG4 | 0.4352 |
| A17m0903_hIgG4 | 1.284 |
| A17m0905_hIgG4 | 0.5588 |
| Pembrolizumab | 0.9756 |

## Example 1-5. Verification of Binding Affinity of Modified Anti-PD-1 Nanobodies for Antigen PD-1

The affinities of the anti-PD-1 nanobodies for PD-1 antigen were measured with a Biacore 8k (GE Healthcare) instrument using surface plasmon resonance (SPR). In the experiment, a CM5 sensor chip was used, and an HBS-EP + buffer (10 mM HEPES, 150 mM NaCl, 3 mM EDTA, 0.05% surfactant P20) was used as a mobile phase. A 30 μg/mL solution of anti-human IgG (Fc) antibody was prepared in a 10 mM sodium acetate buffer (pH 5.0), and the Immobilization program was selected to automatically perform anti-human IgG (Fc) antibody channel amino coupling immobilization. The test antibodies were separately diluted in an HBS-EP + buffer as ligands and captured using the anti-human IgG (Fc) antibody on the chip channels. The human or cynomolgus monkey PD-1 antigen protein (Sino Biological, 10377-H08H; 90311-C08H) was used as an analyte, serially diluted 2-fold with HBS-EP + buffer solution, and made to flow through the experimental and reference channels at a flow rate of 30 μL/min. Binding was allowed for 1 min and dissociation for 15 min. The regeneration buffer 10 mM Glycine pH 1.5 (GE Healthcare, BR-1003-54) was made to flow at a flow rate of 10 μL/min for 30 s. The binding rate Ka, the dissociation rate Kd, and the dissociation constant (i.e., affinity $K_D$) were calculated. The results are shown in Tables 8 and 9.

Table 8. SPR affinity data for the anti-PD-1 nanobodies binding to human PD-1 antigen

| Antibody No. | ka (l/Ms) | Kd (l/s) | Ko (M) |
|---|---|---|---|
| A17h1_hIgG4 | 4.30E+05 | 8.68E-04 | 2.02E-09 |
| A17m09_hIgG4 | 4.58E+05 | 1.63E-03 | 3.56E-09 |
| A17m0901_hIgG4 | 2.97E+05 | 1.92E-03 | 6.44E-09 |
| A17m0902_hIgG4 | 2.91E+05 | 1.77E-03 | 6.09E-09 |
| Pembrolizumab | 1.25E+06 | 3.89E-03 | 3.11E-09 |

Table 9. SPR affinity data for the anti-PD-1 nanobodies binding to cynomolgus monkey PD-1 antigen

| Antibody No. | ka (1/Ms) | Kd (1/s) | $K_D$ (M) |
|---|---|---|---|
| A17h1_hIgG4 | 4.44E+05 | 8.07E-04 | 1.82E-09 |
| A17m09_hIgG4 | 4.81E+05 | 8.43E-04 | 1.75E-09 |
| A17m0901_hIgG4 | 3.40E+05 | 1.13E-03 | 3.32E-09 |
| A17m0902_hIgG4 | 3.28E+05 | 1.08E-03 | 3.28E-09 |
| Pembrolizumab | 1.31E+06 | 1.15E-03 | 8.82E-10 |

The results of binding to human PD-1 antigen show that the humanized antibody A17h1_hIgG4 and the subsequently modified, optimized antibodies A17m09_hIgG4, A17m0901_hIgG4, and A17m0902_hIgG4 have similar $K_D$ values to pembrolizumab. A17h1_hIgG4, A17m09_hIgG4, A17m0901_hIgG4, and A17m0902_hIgG4 all showed smaller dissociation rates than pembrolizumab. The antibodies A17h1_hIgG4, A17m09_hIgG4, A17m0901_hIgG4, and A 17m0902_hIgG4 cross-bound to monkey PD-1, and the affinities of the antibodies for human and monkey PD-1 are similar.

**Example 1-6. DC:T Mix Lymphocyte Reaction (MLR) Test of *In Vitro* Efficacy of Modified Anti-PD-1 Nanobodies**

Co-culture of mature dendritic cells (DCs) with allogeneic T cells can activate T cells and promote cytokine secretion by T cells. This process is inhibited by PD-1/PD-L1 signals, and an anti-PD-1 antibody can be used to remove the inhibition and promote T cell activation. Thus, a DC:T mix lymphocyte reaction experiment can be used to test the *in vitro* efficacy of the anti-PD-1 antibody.

Monocytes were sorted from fresh peripheral blood (PBMCs) of a healthy person using a sorting kit (cat: 19359, stemcell). After seven days of culture using a DC induction kit (cat: 10985, stemcell), the monocytes were induced to differentiate into mature DCs. Then allogeneic T cells were sorted from fresh PBMCs of another healthy person using a sorting kit (cat: 17951, stemcell). 5,000 mature DCs were co-cultured with 50,000 allogeneic T cells, and a candidate antibody was added at a corresponding concentration. After six days of co-culture, the cell supernatant was collected and assayed for IFN-γ concentration using a Cisbio-HTRF IFN-γ assay kit (Cat: 62HIFNGPEH, Perkinelmer), and $EC_{50}$ was calculated.

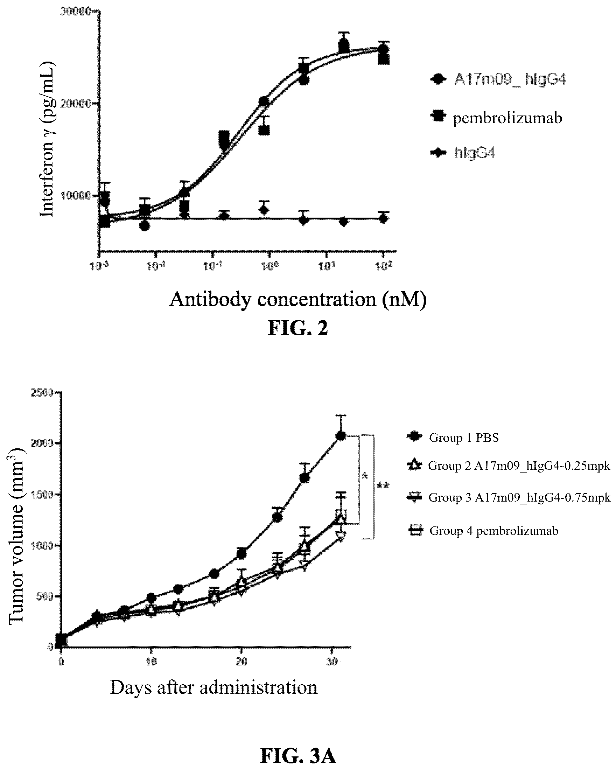

As shown in FIG. 2, A17m09_hIgG4 exhibited substantially the same *in vitro* efficacy as pembrolizumab in the MLR experiment. The $EC_{50}$ of A17m09_hIgG4 is 0.2931 nM, and the $EC_{50}$ of pembrolizumab is 0.3271 nM.

**Example 1-7. Inhibition of Tumor Growth in Colon Cancer Mouse Model by Anti-PD-1 Nanobodies**

In this example, the *in vivo* anti-tumor efficacy of the anti-PD-1 antibodies was verified using an MC38 tumor model of humanized PD-1 C57BL/6 mice.

The MC38 mouse colon cancer cell line was cultured with DMEM medium (10% FBS), and $5 \times 10^5$ MC38 cells were subcutaneously inoculated into humanized PD-1 C57BL/6 female mice (GemPharmatech). When the mean tumor volume of the mice reached about 80 mm$^3$, the mice were randomized into groups of 8 and administered intraperitoneal injections of antibodies or controls. Tumor volume and body weight were measured twice a week, and data were recorded. The grouping and administration regimen for the experiment are shown in Table 10. Given that the molecular weight of A17m09_hIgG4 is about 75 kDa, which is only half of the normal IgG molecular weight, its dose was set to be half of that of pembrolizumab to achieve an equimolar dose.

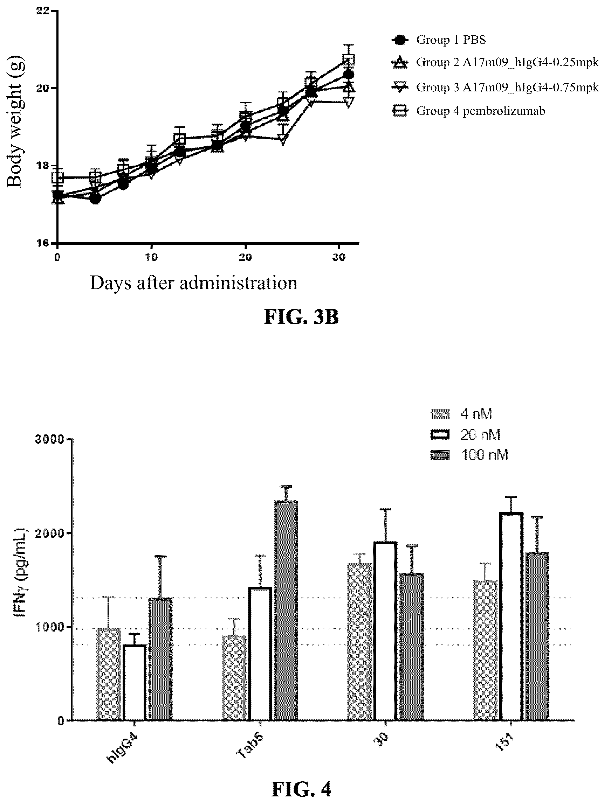

As shown in FIGs. 3A and 3B, A17m09_hIgG4 exhibited the same *in vivo* anti-tumor efficacy as pembrolizumab at the same equimolar dose. Tumor volumes and tumor inhibition rates are shown in Table 11.

Table 10. The grouping and administration regimen for the mouse experiment

| Group | Drug or control | Dose (mg/kg) | Route of administration | Frequency of administration |
|---|---|---|---|---|
| Group 1 | PBS | -- | i.p. | BIW*7 |
| Group 2 | A17m09 hIgG4 | 0.25 | | |
| Group 3 | A17m09 hIgG4 | 0.75 | | |
| Group 4 | Pembrolizumab | 0.5 | | |

Table 11. Mouse tumor volumes and tumor growth inhibition rates

| Group | Drug or control | Tumor volume/mm$^3$ (Mean ± SEM) | | Tumor inhibition rate | P value |
|---|---|---|---|---|---|
| | | Day 0 | Day 31 | Day 31 | |
| Group 1 | PBS | 79±4 | 2074±198 | / | / |
| Group 2 | A17m09_hIgG4(0.25 mg/kg) | 79±4 | 1261±260 | 39% | 0.0263 |
| Group 3 | A17m09_hIgG4(0.75 mg/kg) | 78±3 | 1078±186 | 48% | 0.0026 |
| Group 4 | Pembrolizumab(0.5 mg/kg) | 78±3 | 1300±174 | 37% | 0.0109 |
| (Note: i.p.: intraperitoneal injection; BIW*7: 2 times weekly, 7 injections in total; *P < 0.05, **P < 0.01, and ***P < 0.001 are thought to indicate significant differences compared to the control group) | | | | | |

**Example 2. Anti-PVRIG/TIGIT Bispecific Antibodies**

In this example, the his-tagged human PVRIG (h-PVRIG-his) recombinant protein, mouse IgG2a Fc-tagged human PVRIG (h-PVRIG-mIgG2a Fc) recombinant protein, and human IgG1 Fc-tagged mouse PVRIG (m-PVRIG-hIgG1 Fc) were purchased from Acrobiosystems. The his-tagged PVRL2 was purchased from AcroBiosystem (PV2-H52E2).

**Example 2-1. Screening and Preparation of Anti-PVRIG Nanobodies**

1. Sequences and preparation of immunizing and screening antigens

Table 12. The amino acid sequences of recombinant proteins

| Name | Start and end of amino acid sequence | Genbank accession No. |
|---|---|---|
| h-PVRIG-his | Thr41-Asp171 | Q6DKI7-1 |
| h-PVRIG-mIgG2a Fc | Thr41-Asp171 | Q6DKI7-1 |
| m-PVRIG-hIgGl Fc | Ser35-Asp165 | A0A1B0GS01-1 |

The sequence of the his-tagged cynomolgus monkey PVRIG (cyno-PVRIG-his) recombinant protein is as follows:

TPEVWVQVQMEATELSSFTVHCGFLGPGSISLVTVSWGGPDGAGGTKLAVLHP
ELGTRQWAPARQARWETQSSISLALEDSGASSPFANTTFCCKFASFPEGSWESCG
SLPPSSDPGLSAPPTPVPILRADHHHHHH (SEQ ID NO: 37).

2. Alpaca immunization, nanobody phage display library construction, and antibody screening

Referring to the method of Example 1-1, alpacas were immunized with a his-tagged human PVRIG recombinant protein (h-PVRIG-his). PBMCs were separated from camel peripheral blood on day 56, and RNA was extracted and reverse-transcribed into cDNA to construct a phage library of anti-human PVRIG nanobodies. After 3 rounds of screening, 400 clones were sequenced. The variable region sequences of 2 of them are shown below, and the CDRs are shown in Table 13.
> The variable region of 151

HVQLVESGGGSVQAGGSLRLSCVASASGFT<u>YRPYCMA</u>WFRQAPGKEREAVA<u>GI
DIFGGTTYADSVKG</u>RFTASRDNAGFSLFLQMNDLKPEDTAMYYCAA<u>GDSPDGR
CPPLGQGLNY</u>WGQGTQVTVSS (SEQ ID NO: 38).

Table 13. The CDRs of the anti-PVRIG nanobody (Kabat numbering scheme)

| No. | CDR sequences | |
|---|---|---|
| 151 | CDR1 | YRPYCMA(SEQ ID NO:39) |
| | CDR2 | GIDIFGGTTYADSVKG(SEQ ID NO:40) |
| | CDR3 | GDSPDGRCPPLGQGLNY(SEQ ID NO:41) |

The above antibody variable regions were linked to a human IgG4 heavy chain Fc region comprising a hinge region and mutations S228P, F234A, L235A, and K447A (Eu naming system) to construct a full-length antibody.

>hIgG4 Fc(S228P/F234A/L235A/K447A)

ESKYGPPCPPCPAPEAAGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGA
(SEQ ID NO: 42).

> The full length of 151

HVQLVESGGGSVQAGGSLRLSCVASASGFTYRPYCMAWFRQAPGKEREAVAGI
DIFGGTTYADSVKGRFTASRDNAGFSLFLQMNDLKPEDTAMYYCAAGDSPDGR
CPPLGQGLNYWGQGTQVTVSSESKYGPPCPPCPAPEAAGGPSVFLFPPKPKDTL
MISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLYSRLTVD
KSRWQEGNVFSCSVMHEALHNHYTQKSLSLSLGA(SEQ ID NO: 43).

The anti-PVRIG antibody CPA.7.021 shown in WO2016134333 was obtained by screening a phage library of antibodies. It is of the IgG1 subtype and can bind well to human PVRIG, but it does not bind to cynomolgus monkey PVRIG. The heavy and light chain variable regions of CPA.7.021 were linked to the human IgG4 heavy chain constant region (with mutations S228P, F234A, L235A, and K447A) and the human Kappa light chain constant region, respectively, to construct a positive antibody Tab5.

> The full-length heavy chain of Tab5

EVQLVESGGGVVKPGGSLRLSCAASGFTFGTSSMNWVRQAPGKGLEWVAVISF
DGTEIHYADSVKGRFTISRDNSKSTVFLQMNSLRPDDTALYYCAKGSGNIYFYS
GMDVWGQGTTVTVSSASTKGPSVFPLAPCSRSTSESTAALGCLVKDYFPEPVTV
SWNSGALTSGVHTFPAVLQSSGLYSLSSVVTVPSSSLGTKTYTCNVDHKPSNTK
VDKRVESKYGPPCPPCPAPEAAGGPSVFLFPPKPKDTLMISRTPEVTCVVVDVSQ
EDPEVQFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKGFYPS
DIAVEWESNGQPENNYKTTPPVLDSDGSFFLYSRLTVDKSRWQEGNVFSCSVMH
EALHNHYTQKSLSLSLGA(SEQ ID NO: 44)

> The full-length light chain of Tab5

DIQMTQSPSTLSASVGDRVTITCRAGQSISGWLAWFQQKPGKAPNLLIYETSTLE
SGVPSRFSGSGSGTEYTLTISSLQPDDFATYYCQQYYSYPLTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC      (SEQ
ID NO: 45).

Nucleic acid sequences for expressing the above amino acid sequences were cloned into the pcDNA3.1 expression vector, and the antibodies were expressed and purified by conventional methods. It was verified that the antibodies of interest were obtained.

**Example 2-2. Verification of Affinity of Anti-PVRIG Nanobodies for Antigen PVRIG**

1. ELISA

A his-tagged PVRIG recombinant protein was directly used for coating. After an antibody was added, a secondary antibody (an HRP-conjugated anti-primary antibody Fc antibody) and the HRP substrate TMB were added to test the binding activity of the antibody to the antigen.

A 96-well plate was coated with 1 $\mu$g/mL human, cynomolgus monkey, or mouse PVRIG protein at 100 $\mu$L/well and incubated overnight at 4 °C. The plate was washed three times with the wash buffer. A blocking solution (PBS + 0.05% Tween20 + 1% BSA) was added at 300 $\mu$L/well, and the plate was incubated at room temperature for 1 h. The plate was washed three times with the wash buffer. Dilutions of the test anti-PVRIG antibodies were added at 100 $\mu$L/well. The plate was incubated at 37 °C for 1 h. The plate was washed three times with the wash buffer. The HRP-labeled anti-human IgG secondary antibody (Sigma, A8667) was added at 100 $\mu$L per well. The plate was incubated at 37 °C for 1 h. The plate was washed three times with the wash buffer. TMB was added at 100 $\mu$L/well, and the plate was incubated in the dark for 15 min. 0.16 M sulfuric acid was added at 50 mL/well. OD450 values were measured using a Thermo M$\mu$LtiSkanFc microplate reader, and the $EC_{50}$ values of the anti-PVRIG antibodies binding to the PVRIG recombinant proteins were calculated. All the antibodies in Table 14 showed relatively strong abilities to bind to the human or cynomolgus monkey PVRIG recombinant protein, but they did not bind to the mouse PVRIG recombinant protein.

Table 14. The results of the experiment on the binding of anti-PVRIG antibodies to PVRIG recombinant proteins of different species

| Antibody No. | Human PVRIG-his ELISA $EC_{50}$ (nM) | Monkey PVRIG-his ELISA $EC_{50}$ (nM) |
|---|---|---|
| 151 | 2.15 | 2.43 |
| Tab5 | 2.86 | No binding |
| hIgG4 | No binding | No binding |

2. FACS assay

A stably transfected HEK293 cell strain expressing the human or cynomolgus monkey PVRIG gene was prepared. The cell strain was inoculated into a 96-well plate at $2 \times 10^5$ cells/well. The plate was centrifuged at 300 g for 5 min, and the supernatant was removed. 100 $\mu$L of test antibody was added, and the plate was incubated at 4 °C for 1 h. The plate was centrifuged, and the supernatant was removed. The plate was washed 3 times with 200 $\mu$L of wash buffer (PBS + 2% FBS), and 100 $\mu$L of an Alexa Fluor 488-labeled anti-human IgG secondary antibody (Invitrogen, A-11013) diluted at 1:500 was added. The plate was incubated at 4 °C for 1 h. The plate was centrifuged, and the supernatant was removed. The plate was washed 3 times with 200 $\mu$L of wash buffer (PBS + 2% FBS). The cells were resuspended in 100 $\mu$L of PBS and tested on a flow cytometer (BD FACS Calibur or BD FACS Canto_ II). All the antibodies showed relatively strong abilities to bind to human or cynomolgus monkey PVRIG expressed on the cell surfaces, and their binding abilities were significantly stronger than that of the positive antibody Tab5, which did not even bind to cynomolgus monkey PVRIG at all.

Table 15. The results of the experiment on the binding of anti-PVRIG antibodies to PVRIG of different species on cells

| Antibody No. | Human PVRIG FACS $EC_{50}$ (nM) | Monkey PVRIG FACS $EC_{50}$ (nM) |
|---|---|---|
| 151 | 0.01 | 2.23 |
| Tab5 | 2.13 | No binding |
| hIgG4 | No binding | No binding |

3. Fortebio assay

A Protein A biosensor (Fortebio, #18-5010) was immersed in 200 μL of KB buffer (PBS, pH 7.4, 0.02% tween-20, 0.1% BSA) for 60 s as a wetting treatment. Then an anti-PVRIG antibody was diluted to 10 μg/mL with KB buffer, and the sensor was immersed in 200 μL of the solution until the reading was 1.2 nm. The sensor was immersed in KB buffer for 100 s to remove excess antibody. His-tagged human PVRIG was serially diluted 2-fold to 64 nM-4 nM with KB buffer. The sensor was placed in the solution for 300 seconds of binding and then in KB buffer for 600 seconds of dissociation. Fitting was performed using a dynamic 1:1 binding mode. The affinities of anti-PVRIG antibodies for human PVRIG are shown in Table 16.

The results show that all the antibodies tested have high affinities for human PVRIG.

Table 16. The affinities of anti-PVRIG antibodies for human PVRIG

| Antibody No. | Kon (1/Ms) | Koff (1/s) | KD (M) |
|---|---|---|---|
| 151 | 2.61E+05 | 5.22E-05 | 2.00E-10 |
| Tab5 | 7.37E+05 | 1.61E-05 | 2.19E-10 |

**Example 2-3. Functional and Activity Verification of Anti-PVRIG Nanobodies**

1. Blocking of PVRIG binding to PVRL2 by anti-PVRIG nanobodies

A 96-well plate was coated with 1 μg/mL human PVRIG recombinant protein (h-PVRIG-mIgG2a Fc) at 100 μL/well and incubated overnight at 4 °C. The plate was washed three times with the wash buffer. A blocking solution was added at 300 μL/well, and the plate was incubated at room temperature for 1 h. The plate was washed three times with the wash buffer. 50 μL of diluted test anti-PVRIG antibody and 50 μL of his-tagged ligand PVRL2 were added to each well, and the plate was incubated at 37 °C for 1 h. The plate was washed three times with the wash buffer. An HRP-labeled anti-his-tagged secondary antibody (Genscrpit) diluted at 1:2000 was added at 100 μL/well. The plate was incubated at 37 °C for 1 h. The plate was washed three times with the wash buffer. TMB was added at 100 μL/well, and the plate was incubated in the dark for 15 min. 0.16 M sulfuric acid was added at 50 μL/well. OD values at 450 nm were measured using a Thermo MμLtiSkanFc microplate reader, and the $IC_{50}$ values of the anti-PVRIG antibodies blocking the binding of PVRIG to PVRL2 were calculated.

The results in Table 17 show that all the antibodies tested can greatly inhibit the binding of human PVRIG to human PVRL2.

Table 17. The blocking of human PVRIG/PVRL2 binding by antibodies

| Antibody No. | ELISA $IC_{50}$ (nM) |
|---|---|
| 151 | 0.37 |
| Tab5 | 1.16 |
| hIgG4 | No blocking |

2. Activity of anti-PVRIG nanobodies in reporter gene cells

Firstly, a plvx-OS8 (G418 resistance) plasmid was constructed and transfected into 293F cells, and G418 screening was performed. The expression of OS8 by clone cells was tested using a flow cytometer, and meanwhile the activation of Jurkat cells by OS8 was tested. Clones with moderate activation were selected, yielding a 293F-OS8 cell strain. A plvx-PVRL2 plasmid was constructed and used to infect 293F-OS8 cells, and clones with the highest level of PVRL2 expression were obtained by screening using a flow cytometer, thereby yielding a 293F-OS8-PVRL2 cell strain.

Secondly, a plvx-NFAT-Luc (Hygromycin resistance) was constructed and packaged into a lentivirus. The lentivirus was used to infect Jurkat E6.1 cells. Hygromycin was added, and clones with resistance were obtained by screening. The clones were stimulated with OKT3, and clones with moderate Luciferase signals were obtained by screening, yielding a Jurkat-NFAT-Luc cell line. A plvx-PVRIG (Puromycin resistance) vector was constructed and packaged into a lentivirus, and the lentivirus was used to infect Jurkat-NFAT-Luc cells. Clones with the highest level of PVRIG expression were obtained by screening using a flow cytometer, thereby yielding a Jurkat-NFAT-Luc-PVRIG cell strain.

1E4 Jurkat-NFAT-Luc-PVRIG cells were incubated with a test antibody at 37 °C for 20 min. 1E5 293F-OS8-PVRL2 cells were added, and the mixture was incubated at 37 °C for 5 h. The mixture was centrifuged, and the supernatant was removed. Luciferase buffer (Promega, E6130) was added to lyse the cells, and the fluorescence value was measured. $EC_{50}$ values were calculated and used to evaluate the *in vitro* cell activity of the anti-PVRIG antibodies. The experimental results are shown in Table 18.

The results show that all the antibodies tested have relatively strong abilities to activate luciferase in Jurkat cells, and their activity was at least 10 or more times that of the positive antibody, indicating that these antibodies can bind to PVRIG and block the binding of PVRL2 to PVRIG.

Table 18. The results of the experiment on the activity of anti-PVRIG antibodies in reporter gene cells

| Antibody No. | PVRIG reporter gene cell activity $EC_{50}$ (nM) |
| --- | --- |
| 151 | 0.04 |
| Tab5 | 0.74 |
| hIgG4 | No binding |

3. NK cell killing experiment for anti-PVRIG nanobodies

PVRIG is expressed on NK cells, while PVRL2 is expressed in many tumor cells (including K562 cells). By blocking the binding of PVRL2 to PVRIG, the anti-PVRIG antibodies can relieve the inhibition of the activity of NK cells by tumor cells.

Human malignant non-Hodgkin lymphoma NK92 cells were added to a 96-well plate at 50 $\mu$L ($1 \times 10^5$ cells in total)/well. 50 $\mu$L of 20 nM or 100 nM test antibody was added, and the plate was incubated at 37 °C for 30 min. The plate was washed twice with wash buffer, and the cells were resuspended to a density of $2 \times 10^5$/mL. Human chronic myeloid leukemia K562 cells were added at 50 $\mu$L ($1 \times 10^4$ cells in total)/well, making a ratio of the number of NK92 cells to the number of K562 cells of 10:1. The plate was incubated at 37 °C for 4 h. The killing activity was measured using the CytoTox-Glo cytotoxicity system (Promega, G9292). First, 50 $\mu$L of AAF-Glo reagent was added. The mixture was incubated at room temperature for 15 min, and the fluorescence of K562 cells killed by NK92 cells was measured. Then 50 $\mu$L of lysis buffer was added. The mixture was incubated at room temperature for 15 min to lyse the cells, and the fluorescence of all the cells was measured. Three control groups were prepared, including a sample containing only the culture medium (control group 1), a sample containing only NK92 cells (control group 2), and a sample containing only K562 cells (control group 3), and they were subjected to the same procedure.

The killing activity was calculated according to the following formula:

$$\text{killing activity (\%)} = \{[(R - BG) - (T - BG) - (E - BG)] / [(TL - BGL) - (T - BG)]\} \times 100;$$

where R represents the fluorescence value after addition of AAF-Glo; BG represents the fluorescence value of control group 1 after addition of AAF-Glo; E represents the fluorescence value of control group 2 after addition of AAF-Glo; and T represents the fluorescence value of control group 3 after addition of AAF-Glo; TL represents the fluorescence value of control group 3 after addition of lysis buffer, and BGL represents the fluorescence value of control group 1 after addition of lysis buffer.

According to the experimental results shown in Table 19, all the anti-PVRIG antibodies tested can significantly activate NK92 cells and kill K562 cells.

Table 19. The NK cell killing experiment for anti-PVRIG antibodies

| Antibody No. | Killing activity (%) (mean ± standard deviation) | |
|---|---|---|
| | 20 nM antibody | 100 nM antibody |
| 151 | 35±3 | 33±2 |
| Tab5 | 32±2 | 32±3 |
| hIgG4 | 22±1 | 22±2 |

4. Mixed lymphocyte reaction (MLR) experiment for anti-PVRIG nanobodies

PVRIG is expressed on T cells, while PVRL2 is expressed on DCs. By blocking the binding of PVRL2 to PVRIG, anti-PVRIG antibodies can eliminate the inhibition of T cells by DCs and activate T cells.

PBMCs were isolated from peripheral blood collected from a first individual and cultured in RPMI 1640 medium containing 10% FBS. GM-CSF (Peprotech, 300-03-100UG) and IL-4 (Peprotech, 200-04-100UG) were both added at a final concentration of 50 ng/mL, and a cytokine-containing fresh medium was added every 2-3 days. After 6 days of culture, 1 μg/mL LPS (Sigma, L2880-25MG) was added, and the mixture was incubated for 24 h. DCs obtained by differentiation and maturation were collected. PBMCs were isolated from peripheral blood collected from a second individual, and CD3$^+$ T cells were isolated from the PBMCs using the EasySep human CD3$^+$ T cell isolation kit (Stemcell, 17952). The density of the CD3$^+$ T cells and DCs was adjusted, and $1 \times 10^5$ CD3$^+$ T cells and $2 \times 10^4$ DCs were added to each well. The test antibodies were added, and the plate was incubated at 37 °C for 120 h. The supernatants were taken and assayed for IFNγ content using an ELISA kit (R&D, DY202).

As shown in Table 20 and FIG. 4, all of the anti-PVRIG antibodies tested can significantly activate the secretion of IFNγ by T cells, compared to the control antibody IgG4. Moreover, at low doses (such as 4 nM and 20 nM), the antibody 151 of the present disclosure outperformed the positive control Tab5. Antibody 30 was another antibody obtained by screening in the present application.

Table 20. The mixed lymphocyte reaction IFNγ secretion levels for anti-PVRIG antibodies

| Antibody No. | IFNγ (pg/mL) (mean ± standard deviation) | | |
|---|---|---|---|
| | 4 nM antibody | 20 nM antibody | 100 nM antibody |
| 151 | 1498±175 | 2224±162 | 1798±373 |
| Tab5 | 912±173 | 1425±330 | 2349±148 |
| hIgG4 | 984±335 | 814±112 | 1309±437 |

**Example 2-4. Modification of Sequences of Anti-PVRIG Nanobodies**

By homologous modeling of the three-dimensional structures of selected anti-PVRIG antibody molecules and comparison of the sequences of the anti-PVRIG antibodies with the GermLine database of antibodies, a highly homologous human germline template IGHV3-7 *01 was obtained. The CDRs were grafted into the corresponding human template. After the graft, the three-dimensional structures of the nanobodies were simulated again and analyzed. By back mutation of the embedded residues, the residues directly interacting with the CDRs, and the residues greatly influencing the conformation of the variable regions and optimization of the chemically unstable amino acid residues in the CDRs, a range of humanized nanobodies were generated. The human germline templates and humanized antibody heavy chain variable region sequences for the nanobodies are shown below.

> The variable region of 151H2

EVQLVESGGGLVQPGGSLRLSCAASGFTYRPYCMAWFRQAPGKGLEAVAGIDIF
GGTTYADSVKGRFTISRDNAKNSLYLQMNSLRAEDTAVYYCAAGDSPDGRCPP
LGQGLNYWGQGTMVTVSS (SEQ ID NO: 46)

> The variable region of 151H4

EVQLVESGGGLVQPGGSLRLSCVASASGFT<u>YRPYCMA</u>WFRQAPGKGLEAVA<u>GI DIFGGTTYADSVKG</u>RFTISRDNAKNSLYLQMNSLRAEDTAVYYCAA<u>GDSPDGRC PPLGQGLNY</u>WGQGTMVTVSS (SEQ ID NO: 47)

> The variable region of 151H7

HVQLVESGGGLVQPGGSLRLSCVASASGFT<u>YRPYCMA</u>WFRQAPGKEREAVA<u>GI DIFGGTTYADSVKG</u>RFTASRDNAGFSLYLQMNSLRAEDTAVYYCAA<u>GDSPDGR CPPLGQGLNY</u>WGQGTMVTVSS (SEQ ID NO: 48)

> The variable region of 151H8

EVQLVESGGGLVQPGGSLRLSCVASASGFT<u>YRPYCMA</u>WFRQAPGKGLEAVA<u>GI DIFGGTTYADSVKG</u>RFTISRDNAGFSLYLQMNSLRAEDTAVYYCAA<u>GDSPDGRC PPLGQGLNY</u>WGQGTMVTVSS (SEQ ID NO: 49)

> The variable region of 151H9

HVQLVESGGGLVQPGGSLRLSCVASASGFT<u>YRPYCMA</u>WFRQAPGKGLEAVA<u>GI DIFGGTTYADSVKG</u>RFTASRDNAGFSLYLQMNSLRAEDTAVYYCAA<u>GDSPDGR CPPLGQGLNY</u>WGQGTMVTVSS (SEQ ID NO: 50).

The heavy chain variable regions of the humanized antibodies described above were linked to the heavy chain Fc region of human IgG4 to construct full-length anti-PVRIG antibodies. The heavy chain Fc region comprises a hinge region and mutations S228P/F234A/L235A/K447A or mutations S228P/K447A. The antibodies were expressed and purified by conventional methods. It was verified that the antibodies of interest were obtained.

**Example 2-5. Activity and Functional Verification of Humanized Anti-PVRIG Antibodies**

1. Binding of humanized anti-PVRIG antibodies to cells expressing PVRIG

The binding of the anti-PVRIG antibodies to human or cynomolgus monkey PVRIG was measured by FACS using the method of Example 2-2. The experimental results are shown in Table 21.

Table 21. The results of the FACS experiment on the binding of anti-PVRIG nanobodies to PVRIG of different species

| Antibody No. | Human PVRIG FACS $EC_{50}$ (nM) | Monkey PVRIG FACS $EC_{50}$ (nM) |
|---|---|---|
| 151H4 | 0.240 | 0.035 |
| 151H7 | 0.002 | 0.467 |
| 151H8 | 0.006 | N.T. |
| 151H9 | 0.004 | 3.942 |
| Tab5 | 0.160 | No binding |
| hIgG4 | No binding | No binding |
| (Note: N.T., not tested) | | |

2. Assay for affinities of humanized anti-PVRIG antibodies for PVRIG

The affinities of the humanized anti-PVRIG antibodies for human PVRIG protein were measured using the Fortebio assay method of Example 2-2. As shown in Table 22, all the antibodies have high affinities for human PVRIG protein.

Table 22. The affinities of humanized anti-PVRIG antibodies for human PVRIG

| Antibody No. | Kon (1/Ms) | Koff (1/s) | $K_D$ (M) |
|---|---|---|---|
| 151H7 | 1.57E+05 | 1.88E-04 | 1.20E-09 |

3. Activity of humanized anti-PVRIG antibodies in reporter gene cells

The activity of the humanized anti-PVRIG antibodies in reporter gene cells was measured using the method of Example 2-3. The experimental results are shown in Table 23. All the antibodies listed in the Table have the ability to activate Jurkat cells.

Table 23. The activity of humanized anti-PVRIG antibodies in reporter gene cells

| Antibody No. | PVRIG reporter gene cell activity $EC_{50}$ (nM) |
|---|---|
| 151H7 | 0.038 |
| 151H8 | 0.058 |
| Tab5 | 1.380 |
| hIgG4 | No activation |

4. Abilities of humanized anti-PVRIG antibodies to activate NK cell killing

The abilities of the humanized anti-PVRIG antibodies to activate NK cells were measured using the method of Example 2-3. The experimental results are shown in Table 24. The results show that all the antibodies tested exhibited significant abilities to activate NK cells and promote the killing of the target cell K562 by NK cells.

Table 24. The NK cell killing experiment for humanized anti-PVRIG antibodies

| Antibody No. | Killing activity (%) (mean $\pm$ standard deviation) | | |
|---|---|---|---|
| | 4 nM antibody | 20 nM antibody | 100 nM antibody |
| 151 | 20.9$\pm$1.5 | 22.9$\pm$1.5 | 24.2$\pm$2.3 |
| 151H7 | 16.9$\pm$0.8 | 21.0$\pm$0.4 | 18.4$\pm$0.9 |
| hIgG4 | 11.2$\pm$0.4 | 11.7$\pm$2.1 | 9.2$\pm$0.6 |
| PBS | 6.6$\pm$1.1 | | |

**Example 2-6. Preparation of Anti-PVRIG/TIGIT Bispecific Antibodies**

To investigate the effect of differently constructed anti-PVRIG/TIGIT bispecific antibodies on antibody function, the anti-PVRIG nanobody 151 was linked to the N-terminus or C-terminus of the heavy or light chain of the anti-TIGIT antibody 1708 by a linker of GGGGSGGGGS (SEQ ID NO: 78). Four anti-PVRIG/TIGIT bispecific antibodies were formed and named 1708-151-1, 1708-151-2, 1708-151-3 and 1708-151-4, which corresponded to the cases where 151 was linked to the heavy chain N-terminal, heavy chain C-terminal, light chain N-terminal and light chain C-terminal of 1708, respectively. The anti-TIGIT antibody 1708 is of the human IgG4 subtype and comprises the mutation S228P (Eu naming system). The sequences of the anti-TIGIT antibody 1708 and its bispecific antibodies formed with 151 are shown in Table 25 below. The sequences of the anti-TIGIT antibody are shown in Tables 26 and 27. The TIGIT antibody has been disclosed in WO2019062832A, which is incorporated herein by reference in its entirety.

Table 25. The sequences of the first and second polypeptide chains of anti-PVRIG/TIGIT bispecific antibodies

| Antibody No. | Amino acid sequences of full-length heavy/light chains | |
|---|---|---|
| 1708 (anti-TIGI T anti-body) | First poly-peptide chain | EVQLVQSGAEVKKPGASVKVSCKASGYTFTNYW MHWVRQAPGQGLEWMGRIDPDSTGSKYNEKFKT RVTMTRDTSTSTVYMELSSLRSEDTAVYYCAREG AYGYYFDYWGQGTLVTVSSASTKGPSVFPLAPCS RSTSESTAALGCLVKDYFPEPVTVSWNSGALTSGV HTFPAVLQSSGLYSLSSVVTVPSSSLGTKTYTCNV DHKPSNTKVDKRVESKYGPPCPPCPAPEFLGGPSV FLFPPKPKDTLMISRTPEVTCVVVDVSQEDPEVQF NWYVDGVEVHNAKTKPREEQFNSTYRVVSVLTV LHQDWLNGKEYKCKVSNKGLPSSIEKTISKAKGQ PREPQVYTLPPSQEEMTKNQVSLTCLVKGFYPSDI AVEWESNGQPENNYKTTPPVLDSDGSFFLYSRLTV DKSRWQEGNVFSCSVMHEALHNHYTQKSLSLSL GK (SEQ ID NO: 51) |
| | Second polypeptide chain | DIQMTQSPSSLSASVGDRVTITCRASENIYSYLAWY QQKPGKSPKLLIYNARTLAEGVPSRFSGSGSGTDF TLTISSLQPEDFATYYCQYHSGSPLPFGGGTKVEIK RTVAAPSVFIFPPSDEQLKSGTASVVCLLNNFYPRE AKVQWKVDNALQSGNSQESVTEQDSKDSTYSLSS TLTLSKADYEKHKVYACEVTHQGLSSPVTKSFNRG EC(SEQ ID NO: 52) |
| 1708-151-1 (antibody 151 linked to N-terminus of heavy chain of 1708) | First poly-peptide chain | HVQLVESGGGSVQAGGSLRLSCVASASGFTYRPYC MAWFRQAPGKEREAVAGIDIFGGTTYADSVKGRFT ASRDNAGFSLFLQMNDLKPEDTAMYYCAAGDSPD GRCPPLGQGLNYWGQGTQVTVSS<u>GGGGSGGGGS</u> EVQLVQSGAEVKKPGASVKVSCKASGYTFTNYW MHWVRQAPGQGLEWMGRIDPDSTGSKYNEKFKT RVTMTRDTSTSTVYMELSSLRSEDTAVYYCAREGA YGYYFDYWGQGTLVTVSSASTKGPSVFPLAPCSRS TSESTAALGCLVKDYFPEPVTVSWNSGALTSGVHT FPAVLQSSGLYSLSSVVTVPSSSLGTKTYTCNVDHK PSNTKVDKRVESKYGPPCPPCPAPEFLGGPSVFLFP PKPKDTLMISRTPEVTCVVVDVSQEDPEVQFNWY VDGVEVHNAKTKPREEQFNSTYRVVSVLTVLHQD WLNGKEYKCKVSNKGLPSSIEKTISKAKGQPREPQ VYTLPPSQEEMTKNQVSLTCLVKGFYPSDIAVEWE SNGQPENNYKTTPPVLDSDGSFFLYSRLTVDKSRW QEGNVFSCSVMHEALHNHYTQKSLSLSLGK (SEQ ID NO: 53) |
| | Second polypeptide chain | Same as the light chain of 1708 (SEQ ID NO: 52) |

(continued)

| Antibody No. | Amino acid sequences of full-length heavy/light chains | |
|---|---|---|
| 1708-151-2 (151 linked to C terminus of heavy chain of 1708) | First poly-peptide chain | EVQLVQSGAEVKKPGASVKVSCKASGYTFTNYW MHWVRQAPGQGLEWMGRIDPDSTGSKYNEKFKT RVTMTRDTSTSTVYMELSSLRSEDTAVYYCAREGA YGYYFDYWGQGTLVTVSSASTKGPSVFPLAPCSRS TSESTAALGCLVKDYFPEPVTVSWNSGALTSGVHT FPAVLQSSGLYSLSSVVTVPSSSLGTKTYTCNVDHK PSNTKVDKRVESKYGPPCPPCPAPEFLGGPSVFLFP PKPKDTLMISRTPEVTCVVVDVSQEDPEVQFNWY VDGVEVHNAKTKPREEQFNSTYRVVSVLTVLHQD WLNGKEYKCKVSNKGLPSSIEKTISKAKGQPREPQ VYTLPPSQEEMTKNQVSLTCLVKGFYPSDIAVEWE SNGQPENNYKTTPPVLDSDGSFFLYSRLTVDKSRW QEGNVFSCSVMHEALHNHYTQKSLSLSLGK<u>GGGG SGGGGS</u>HVQLVESGGGSVQAGGSLRLSCVASASG FTYRPYCMAWFRQAPGKEREAVAGIDIFGGTTYAD SVKGRFTASRDNAGFSLFLQMNDLKPEDTAMYYC AAGDSPDGRCPPLGQGLNYWGQGTQVTVSS (SEQ ID NO: 54) |
| | Second polypeptide chain | Same as the light chain of 1708 (SEQ ID NO: 52) |
| 1708-151-3 (151 linked to N-terminus of light chain of 1708) | First polypeptide chain | Same as the heavy chain of 1708 (SEQ ID NO: 51) |
| | Second polypeptide chain | HVQLVESGGGSVQAGGSLRLSCVASASGFTYRPYC MAWFRQAPGKEREAVAGIDIFGGTTYADSVKGRFT ASRDNAGFSLFLQMNDLKPEDTAMYYCAAGDSPD GRCPPLGQGLNYWGQGTQVTVSS<u>GGGGSGGGGS</u> DIQMTQSPSSLSASVGDRVTITCRASENIYSYLAWY QQKPGKSPKLLIYNARTLAEGVPSRFSGSGSGTDF TLTISSLQPEDFATYYCQYHSGSPLPFGGGTKVEIK RTVAAPSVFIFPPSDEQLKSGTASVVCLLNNFYPRE AKVQWKVDNALQSGNSQESVTEQDSKDSTYSLSS TLTLSKADYEKHKVYACEVTHQGLSSPVTKSFNRG EC(SEQ ID NO: 55) |

(continued)

| Antibody No. | Amino acid sequences of full-length heavy/light chains | |
|---|---|---|
| 1708-151-4 (151 linked to C-terminus of light chain of 1708) | First polypeptide chain | Same as the heavy chain of 1708 (SEQ ID NO: 51) |
| | Second polypeptide chain | DIQMTQSPSSLSASVGDRVTITCRASENIYSYLAWY QQKPGKSPKLLIYNARTLAEGVPSRFSGSGSGTDF TLTISSLQPEDFATYYCQYHSGSPLPFGGGTKVEIK RTVAAPSVFIFPPSDEQLKSGTASVVCLLNNFYPRE AKVQWKVDNALQSGNSQESVTEQDSKDSTYSLSS TLTLSKADYEKHKVYACEVTHQGLSSPVTKSFNRG EC<u>GGGGSGGGGS</u>HVQLVESGGGSVQAGGSLRLSC VASASGFTYRPYCMAWFRQAPGKEREAVAGIDIFG GTTYADSVKGRFTASRDNAGFSLFLQMNDLKPED TAMYYCAAGDSPDGRCPPLGQGLNYWGQGTQVT VSS (SEQ ID NO: 56) |

Table 26. The sequences of the heavy and light chain CDRs of the anti-TIGIT antibody (Kabat numbering scheme)

| Antibody | Heavy chain | | Light chain | |
|---|---|---|---|---|
| 1708 | HCDR1 | NYWMH (SEQ ID NO:57) | LCDR1 | RASENIYSYLA (SEQ ID NO:60) |
| | HCDR2 | RIDPDSTGSKYNEKFKT (SEQ ID NO:58) | LCDR2 | NARTLAE (SEQ ID NO:61) |
| | HCDR3 | EGAYGYYFDY (SEQ ID NO:59) | LCDR3 | QYHSGSPLP (SEQ ID NO:62) |

Table 27. The sequences of the heavy chain VHs and light chain VLs of the anti-TIGIT antibody

| Antibody | Sequences of heavy chain VHs and light chain VLs |
|---|---|
| 1708-VH1 | EVQLVQSGAEVKKPGASVKVSCKASGYTFTNYWMHWVRQAPGQ GLEWMGRIDPDSTGSKYNEKFKTRVTMTRDTSTSTVYMELSSLRS EDTAVYYCAREGAYGYYFDYWGQGTLVTVSS(SEQ ID NO:63) |
| 1708-VH2 | EVQLVQSGAEVKKPGASVKVSCKASGYTFTNYWMHWVRQAPGQ GLEWMGRIDPDSTGSKYNEKFKTRVTMTVDTSTSTVYMELSSLRS EDTAVYYCAREGAYGYYFDYWGQGTLVTVSS(SEQ ID NO:64) |
| 1708-VH3 | EVQLVQSGAEVKKPGASVKVSCKASGYTFTNYWMHWVRQAPGQ GLEWIGRIDPDSTGSKYNEKFKTRVTMTVDTSTSTAYMELSSLRSE DTAVYYCAREGAYGYYFDYWGQGTLVTVSS(SEQ ID NO:65) |
| 1708-VL1 | DIQMTQSPSSLSASVGDRVTITCRASENIYSYLAWYQQKPGKAPKL LIYNARTLAEGVPSRFSGSGSGTDFTLTISSLQPEDFATYYCQYHSG SPLPFGGGTKVEIK(SEQ ID NO:66) |
| 1708-VL2 | DIQMTQSPSSLSASVGDRVTITCRASENIYSYLAWYQQKPGKSPKL LIYNARTLAEGVPSRFSGSGSGTDFTLTISSLQPEDFATYYCQYHSG SPLPFGGGTKVEIK(SEQ ID NO:67) |

Different humanized anti-PVRIG antibody variable regions (151H7 and 151H8) were linked to the N-terminus of the heavy chain of the anti-TIGIT antibody 1708; that is, bispecific antibodies were constructed using a bispecific antibody structure similar to 1708-151-1. Linker sequences are double-underlined in the sequences below.

> The first polypeptide chain of 1708-151H7

HVQLVESGGGLVQPGGSLRLSCVASASGFTYRPYCMAWFRQAPGKEREAVAGI
DIFGGTTYADSVKGRFTASRDNAGFSLYLQMNSLRAEDTAVYYCAAGDSPDGR
CPPLGQGLNYWGQGTMVTVSS<u>GGGGSGGGGS</u>EVQLVQSGAEVKKPGASVKVS
CKASGYTFTNYWMHWVRQAPGQGLEWMGRIDPDSTGSKYNEKFKTRVTMTR
DTSTSTVYMELSSLRSEDTAVYYCAREGAYGYYFDYWGQGTLVTVSSASTKGP
SVFPLAPCSRSTSESTAALGCLVKDYFPEPVTVSWNSGALTSGVHTFPAVLQSSG
LYSLSSVVTVPSSSLGTKTYTCNVDHKPSNTKVDKRVESKYGPPCPPCPAPEFLG
GPSVFLFPPKPKDTLMISRTPEVTCVVVDVSQEDPEVQFNWYVDGVEVHNAKT
KPREEQFNSTYRVVSVLTVLHQDWLNGKEYKCKVSNKGLPSSIEKTISKAKGQP
REPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGK (SEQ
ID NO: 68)

> The first polypeptide chain of 1708-151H8

EVQLVESGGGLVQPGGSLRLSCVASASGFTYRPYCMAWFRQAPGKGLEAVAGID
IFGGTTYADSVKGRFTISRDNAGFSLYLQMNSLRAEDTAVYYCAAGDSPDGRCP
PLGQGLNYWGQGTMVTVSS<u>GGGGSGGGGS</u>EVQLVQSGAEVKKPGASVKVSC
KASGYTFTNYWMHWVRQAPGQGLEWMGRIDPDSTGSKYNEKFKTRVTMTRD
TSTSTVYMELSSLRSEDTAVYYCAREGAYGYYFDYWGQGTLVTVSSASTKGPS
VFPLAPCSRSTSESTAALGCLVKDYFPEPVTVSWNSGALTSGVHTFPAVLQSSGL
YSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTK
PREEQFNSTYRVVSVLTVLHQDWLNGKEYKCKVSNKGLPSSIEKTISKAKGQPR
EPQVYTLPPSQEEMTKNQVSLTCLVKGFYPSDIAVEWESNGQPENNYKTTPPVL
DSDGSFFLYSRLTVDKSRWQEGNVFSCSVMHEALHNHYTQKSLSLSLGK    (SEQ

ID NO: 69).

The second polypeptide chains of 1708-30H2, 1708-151H7, and 1708-151H8 are all identical to the light chain of 1708 (SEQ ID NO: 52).

Transient transfection and antibody expression and purification were performed by conventional methods. It was verified that the full-length anti-PVRIG/TIGIT bispecific antibodies of the present disclosure were obtained, and they all showed good expression levels and purity.

**Example 2-7. Activity and Functional Verification of Anti-PVRIG/TIGIT Bispecific Antibodies**

1. Binding of bispecific antibodies to human PVRIG and blocking of ligand PVRL2

Experiments were conducted using the methods of Examples 2-2 and 2-3. The results show that there was no statistically significant difference in the binding of the bispecific antibodies of different configurations-1708-151-1, 1708-151-2, 1708-151-3, and 1708-151-4-to human PVRIG recombinant protein and cells overexpressing human PVRIG, and in their blocking of PVRL2 binding to PVRIG.

2. Binding of bispecific antibodies to human TIGIT and blocking of ligand PVR

Experiments were conducted using the methods of Examples 2-2 and 2-3 (the corresponding receptor and ligand were changed to human TIGIT and human PVR), and the results are shown in Table 28. The results show that there was no statistically significant difference in the binding of the bispecific antibodies of different configurations-1708-151-1, 1708-151-2, 1708-151-3, and 1708-151-4-and the anti-TIGIT antibody to human TIGIT recombinant protein and cells overexpressing human TIGIT, and in their blocking of TIGIT binding to its ligand PVR.

It can be seen that whether the anti-PVRIG antibody was linked to the N-terminus or C-terminus of the heavy or light chain of the anti-TIGIT antibody, the binding to PVRIG and TIGIT and the blocking of the ligand were both maintained, and good expression levels and purity were shown.

3. Binding of humanized anti-PVRIG/TIGIT bispecific antibodies to PVRIG and TIGIT and blocking of corresponding ligands

The binding of the humanized anti-PVRIG/TIGIT bispecific antibodies to human and cynomolgus monkey PVRIG and their blocking of the ligand for human PVRIG were measured using the methods of Examples 2-2 and 2-3. The results are shown in Table 28. The results show that all the bispecific antibodies can bind to human PVRIG and block the binding of PVRIG to PVRL2.

Table 28. The binding of humanized bispecific antibodies to PVRIG and their blocking of the ligand

| Antibody No. | $EC_{50}$ (nM) of binding to human PVRIG recombinant protein | $EC_{50}$ (nM) of binding to cells overexpressing human PVRIG | $IC_{50}$ (nM) of blocking binding of human PVRIG to human PVRL2 |
|---|---|---|---|
| 1708-151H7 | 0.679 | 0.043 | 0.598 |
| 1708-151H8 | 0.390 | 0.094 | 0.654 |
| 1708 | No binding | No binding | Not tested |
| Tab5 | 1.407 | 0.789 | 0.964 |
| hIgG4 | No binding | No binding | No blocking |

Using methods similar to those of Examples 2-2 and 2-3, the binding of the humanized anti-PVRIG/TIGIT bispecific antibodies to human and cynomolgus monkey TIGIT and their blocking of human TIGIT binding to the ligand were measured. In the methods, TIGIT was used in place of PVRIG protein, and PVR was used in place of PVRL2. The results are shown in Table 29. The results show that all the bispecific antibodies can bind to human TIGIT and block the binding of TIGIT to PVR.

Table 29. The binding of humanized bispecific antibodies to TIGIT and their blocking of the ligand

| Antibody No. | $EC_{50}$ (nM) of binding to human TIGIT recombinant protein | $EC_{50}$ (nM) of binding to cells overexpressing human TIGIT | $IC_{50}$ (nM) of blocking binding of human TIGIT to human PVR |
|---|---|---|---|
| 1708-151H7 | 0.160 | 0.012 | 0.773 |
| 1708-151H8 | 0.184 | 0.006 | 1.087 |
| 1708 | 0.133 | 0.0027 | 0.779 |
| Tab5 | No binding | No binding | Not tested |
| hIgG4 | No binding | No binding | No blocking |

The affinities of the bispecific antibodies for human PVRIG and human TIGIT were measured using Biacore. A humanized bispecific antibody was captured on a Protein A biosensor chip (GE lifesciences, 29127557) of a Biacore instrument (Biacore X100, GE), and then a series of concentrations of human PVRIG antigen (AcroBiosystem, PVG-H52H4) or human TIGIT antigen (AcroBiosystem, TIT-H52H3) were made to flow over the surface of the chip to obtain a binding and dissociation curve. The results are shown in Table 30.

Table 30. The affinities of humanized bispecific antibody for human PVRIG and human TIGIT

| Antibody No. | Antigen | kon (1/Ms) | koff (1/s) | $K_D$ (M) |
|---|---|---|---|---|
| 1708-151H7 | Human PVRIG | 6.06E+06 | 7.57E-04 | 1.25E-10 |
| 1708-151H7 | Human TIGIT | 1.40E+06 | 1.16E-04 | 8.28E-11 |

4. Mixed lymphocyte reaction (MLR) experiment for anti-PVRIG/TIGIT bispecific antibody

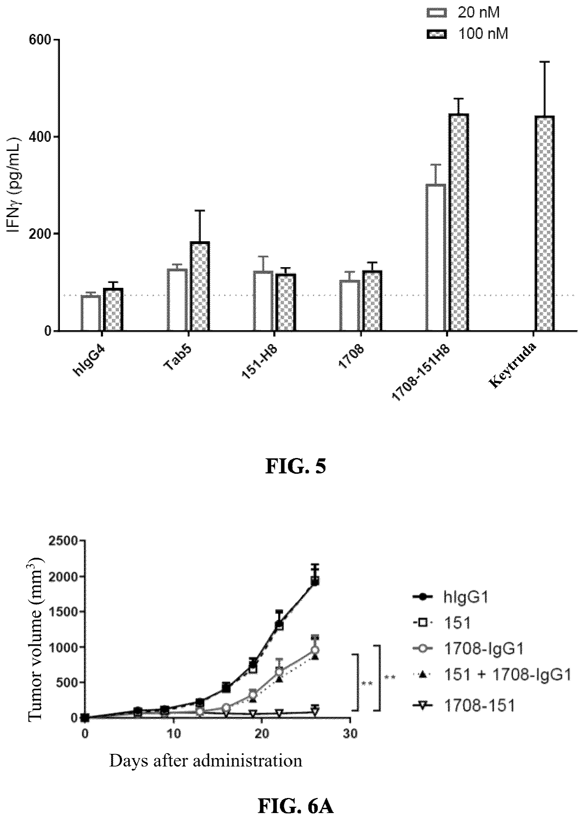

The ability of the humanized anti-PVRIG/TIGIT bispecific antibody to activate T cells was tested using the method in part 4 of Example 2-3. The experimental results are shown in FIG. 5 and Table 31. The results show that the humanized anti-PVRIG/TIGIT bispecific antibody 1708-151H8 has a significant ability to activate T cells and promote the secretion of IFNγ by T cells. Importantly, the activity of the bispecific antibody is stronger than that of the anti-PVRIG antibody 151H8 alone or the anti-TIGIT antibody 1708 alone.

Table 31. The mixed lymphocyte reaction IFNγ secretion levels for the humanized bispecific antibodies

| Antibody No. | IFNγ (pg/mL) (mean $\pm$ standard deviation) | |
|---|---|---|
| | 20 nM antibody | 100 nM antibody |
| hIgG4 | 74$\pm$5 | 89$\pm$12 |
| 151H8 | 124$\pm$29 | 118$\pm$11 |
| 1708 | 106$\pm$16 | 125$\pm$16 |
| 1708-151H8 | 303$\pm$40 | 448$\pm$40 |
| Tab5 | 128$\pm$8.9 | 185$\pm$63 |
| Pembrolizumab | Not tested | 444$\pm$111 |

**Example 2-8. Evaluation of Anti-Tumor Effects of Anti-PVRIG/TIGIT Bispecific Antibodies in Mouse Subcutaneous Xenograft Tumor Model of Human Melanoma A375 Mixed with Human PBMCs**

To further investigate the role of bispecific antibody subtypes in animal efficacy, animal efficacy tests were performed. The heavy chain variable region of 1708-IgG1 was identical to that of 1708 except that the heavy chain constant region subtype was changed to IgG1; the full-length light chain of 1708-IgG1 and the second polypeptide chain of 1708-151-IgG1 were identical to the light chain of 1708.

NCG mice, female, aged 4-8 weeks, weighing about 18-22 g, purchased from Jiangsu GemPharmatech Co., Ltd. All the NCG mice were kept in an SPF animal house with an IVC constant-temperature and constant-pressure system.

A375 cells were cultured in DMEM medium containing 10% fetal bovine serum (FBS). A375 cells in the exponential growth phase were collected and resuspended in HBSS to a concentration appropriate for subcutaneous tumor inoculation into NCG mice. The A375 cells used for co-culture were treated with Mitomycin C for 2 h and washed three times with PBS. Peripheral blood was collected from a normal human, and human PBMCs were isolated by density gradient centrifugation and counted. The PBMCs were then resuspended in RPMI1640 medium (containing IL2 and 10% FBS) to a concentration of $3 \times 10^6$ cells/mL and co-cultured with the Mitomycin C-treated A375 cells. After 6 days of co-culture, PBMCs were harvested, and meanwhile freshly digested A375 cells were harvested. Each mouse was inoculated with $5 \times 10^5$ PBMCs and $4 \times 10^6$ A375 cells; the inoculation volume was 0.2 mL/mouse (containing 50% Matrigel); the cells were inoculated subcutaneously into the right side of each female NCG mouse. The mice were randomized into groups according to body weight and dosed. The method of administration, the doses, and the route of administration are detailed in Table 32. The day when the grouping and administration were performed was day 0. Given that the molecular weights of the anti-PVRIG antibody and anti-TIGIT antibody are different, these doses ensured that the anti-PVRIG antibody and anti-TIGIT antibody had the same initial molar concentration.

Table 32. The administration regimen

| Group | Treatment group | N | Dose (mg/kg) | Administration regimen (route of administration) |
|---|---|---|---|---|
| 1 | hIgG1 | 7 | 30 | |

(continued)

| Group | Treatment group | N | Dose (mg/kg) | Administration regimen (route of administration) |
|---|---|---|---|---|
| 2 | 151-IgG4 | 7 | 16.1 | Q2D (i.p) |
| 3 | 1708-IgG1 | 7 | 30 | |
| 4 | 151-IgG4+1708-IgG1 | 7 | 16.1+30 | |
| 5 | 1708-151-IgG4 | 7 | 35.8 | |
| (Note: N: the number of animals used; i.p.: intraperitoneal injection; Q2D: once every two days; administration volume: adjusted depending on the body weight of tumor-bearing mice (0.1 mL/10 g)) | | | | |

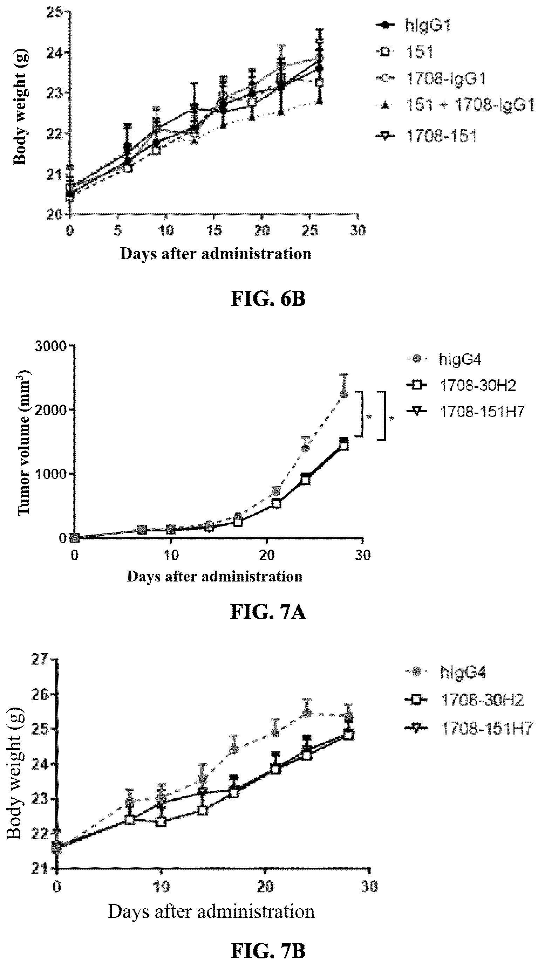

After the start of administration, the tumor volume and body weight of the mice were measured 2 times a week. The experimental results are shown in Table 33 and FIGs. 6A and 6B.

Table 33. The anti-tumor effects of the anti-PVRIG/TIGIT bispecific antibodies in the human A375 tumor mouse model

| Group | Tumor volume on day 0 ($mm^3$) | Tumor volume on day 26 ($mm^3$) (mean $\pm$ standard deviation) | TGI(%) | T/C(%) | p value |
|---|---|---|---|---|---|
| 1 | 0 | 1913.62$\pm$188.23 | -- | -- | -- |
| 2 | 0 | 1942.70$\pm$223.36 | -1.52 | 101.52 | 0.916 |
| 3 | 0 | 958.83$\pm$204.39 | 49.89 | 50.11 | <0.001*** |
| 4 | 0 | 876.21$\pm$243.70 | 54.21 | 45.79 | <0.001*** |
| 5 | 0 | 79.99$\pm$36.57 | 95.82 | 4.18 | <0.001*** |
| (Note: *$P < 0.05$, **$P < 0.01$, and ***$P < 0.001$ are thought to indicate significant differences compared to the control group (hIgG1)) | | | | | |

At the end of the experiment (day 26 after administration), the anti-PVRIG antibody 151-IgG4 single-drug group showed no significant difference from the control group. The anti-TIGIT antibody 1708-IgG1 single-drug group and the anti-PVRIG antibody 151-IgG4 and anti-TIGIT antibody 1708-IgG1 combination group showed reductions in tumor volume. The 1708-151-IgG4 bispecific antibody group could even achieve complete inhibition of tumor growth, showing significant differences from the other groups (see FIG. 6A).

The mice were randomized into groups according to body weight and dosed. The method of administration, the doses, and the route of administration are detailed in Table 34. The day when the grouping and administration were performed was day 0.

Table 34. Administration regimen

| Group | Treatment group | N | Dose (mg/kg) | Administration regimen (route of administration) |
|---|---|---|---|---|
| 1 | hIgG4 | 7 | 35.8 | Q2D (i.p) |
| 3 | 1708-151H7-IgG4 | 7 | 12 | |
| (Note: N: the number of animals used; i.p.: intraperitoneal injection; Q2D: once every two days; administration volume: adjusted depending on the body weight of tumor-bearing mice (0.1 mL/10 g)) | | | | |

After the start of administration, the body weight and tumor volume of the mice were measured 2 times a week. The experimental results are shown in Table 35 and FIGs. 7A-7B.

Table 35. The anti-tumor effects of the anti-PVRIG/TIGIT bispecific antibodies in the human A375 tumor mouse model

| Group | Tumor volume on day 0 ($mm^3$) | Tumor volume on day 28 ($mm^3$) (mean $\pm$ standard deviation) | TGI(%) | T/C(%) | p value |
|---|---|---|---|---|---|
| 1 | 0 | 2239.26$\pm$322.87 | -- | -- | -- |

(continued)

| Group | Tumor volume on day 0 (mm$^3$) | Tumor volume on day 28 (mm$^3$) (mean $\pm$ standard deviation) | TGI(%) | T/C(%) | p value |
|---|---|---|---|---|---|
| 2 | 0 | 1468.96$\pm$67.07 | 34.40 | 65.60 | <0.05* |
| (Note: *P < 0.05, **P < 0.01, and ***P < 0.001 are thought to indicate significant differences compared to the control group (hIgG1)) | | | | | |

At the end of the experiment (day 28 after administration), the 1708-151H7 bispecific antibody group could achieve effective inhibition of tumor growth at low doses, showing significant differences from the control group (see FIGs. 7A and 7B). 1708-30H2 was another antibody obtained by screening in the present application.

**Example 3. Anti-PD-1/PVRIG/TIGIT Trispecific Antibodies**

**Example 3-1. Design and Preparation of Anti-PD-1/PVRIG/TIGIT Trispecific Antibodies**

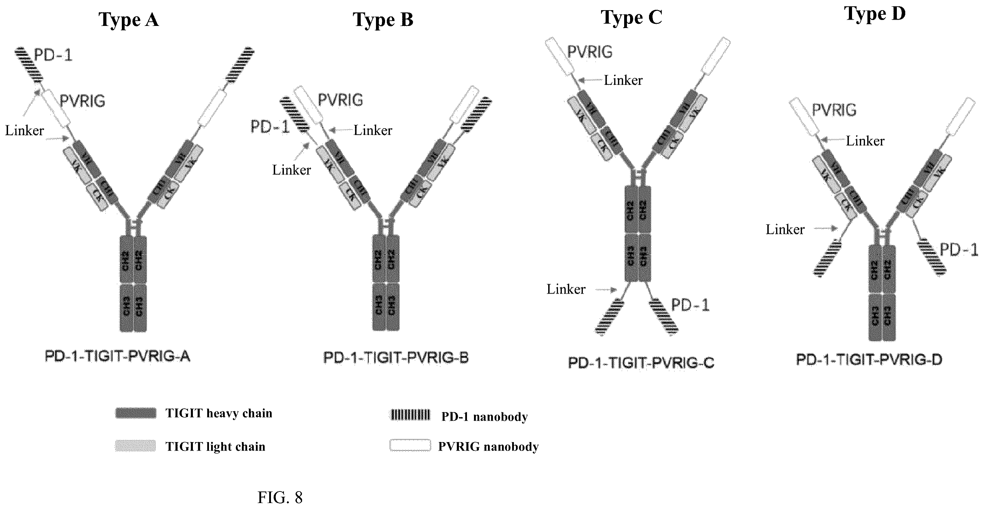

There are four types of trispecific antibodies: types A, B, C, and D, which are schematically shown in FIG. 8.
Type A: Anti-PD-1 nanobodies are linked to anti-PVRIG nanobodies by linkers (e.g., GGGGSGGGGS), and then the anti-PVRIG nanobodies are linked to the N-termini of the heavy chains of an anti-TIGIT antibody by linkers (A of FIG. 8).
Type B: Anti-PVRIG antibodies are linked to the N-termini of the heavy chains of a TIGIT IgG molecule by linkers, and anti-PD-1 antibodies are linked to the N-termini of the light chains of the anti-TIGIT antibody by linkers (B of FIG. 8).
Type C: Anti-PVRIG antibodies are linked to the N-termini of the heavy chains of a TIGIT IgG molecule by linkers. Anti-PD-1 antibodies are linked to the C-termini of the heavy chains of the anti-TIGIT antibody by linkers (GGGGSGGGGS) (C of FIG. 8).
Type D: Anti-PVRIG antibodies are linked to the N-termini of the heavy chains of a TIGIT IgG molecule by linkers. Anti-PD-1 antibodies are linked to the C-termini of the light chains of the anti-TIGIT antibody by linkers (D of FIG. 8).
The names of the trispecific antibodies in the present disclosure are shown in Table 36. For example, A17m0902-1708-151H7-A represents that the clones of the anti-PD-1, TIGIT, and PVRIG antibodies used are A17m0902, 1708, and 151H7, respectively, and the trispecific antibody is of type A.

Table 36. The names of anti-PD-1/PVRIG/TIGIT trispecific antibodies

| Antibody type name | Antibody No. |
|---|---|
| | Anti-PVRIG antibody being 151H7 |
| PD-1/PVRIG/TIGIT-A | A17m0902-1708-151H7-A |
| PD-1/PVRIG/TIGIT-B | A17m0902-1708-151H7-B |
| PD-1/PVRIG/TIGIT-C | A17m0902-1708-151H7-C |
| PD-1/PVRIG/TIGIT-D | A17m0902-1708-151H7-D |
| PD-1/PVRIG/TIGIT-A | A17h1-1708-151H7-A |
| PD-1/PVRIG/TIGIT-B | A17h1-1708-151H7-B |
| PD-1/PVRIG/TIGIT-C | A17h1-1708-151H7-C |
| PD-1/PVRIG/TIGIT-D | A17h1-1708-151H7-D |

The A17h1-related molecules in Table 36 were all obtained on the basis of their corresponding A17m0902-related molecules by replacing the A17m0902 (SEQ ID NO: 27) sequence with the A17h1 (SEQ ID NO: 8) sequence.
The sequences are as follows (linkers underlined, Fc or light chain Cκ italicized):

> The first polypeptide chain of A17m0902-1708-151H7-A

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW
GQGTQVTVSS<u>GGGGSGGGGS</u>HVQLVESGGGLVQPGGSLRLSCVASASGFTYRP
YCMAWFRQAPGKEREAVAGIDIFGGTTYADSVKGRFTASRDNAGFSLYLQMNS
LRAEDTAVYYCAAGDSPDGRCPPLGQGLNYWGQGTMVTVSS<u>GGGGSGGGGS</u>E
VQLVQSGAEVKKPGASVKVSCKASGYTFTNYWMHWVRQAPGQGLEWMGRID
PDSTGSKYNEKFKTRVTMTRDTSTSTVYMELSSLRSEDTAVYYCAREGAYGYY
FDYWGQGTLVTVSS*ASTKGPSVFPLAPCSRSTSESTAALGCLVKDYFPEPVTVSWNS*
*GALTSGVHTFPAVLQSSGLYSLSSVVTVPSSSLGTKTYTCNVDHKPSNTKVDKRVESKY*
*GPPCPPCPAPEFLGGPSVFLFPPKPKDTLMISRTPEVTCVVVDVSQEDPEVQFNWYV*
*DGVEVHNAKTKPREEQFNSTYRVVSVLTVLHQDWLNGKEYKCKVSNKGLPSSIEKTI*
*SKAKGQPREPQVYTLPPSQEEMTKNQVSLTCLVKGFYPSDIAVEWESNGQPENNYKT*
*TPPVLDSDGSFFLYSRLTVDKSRWQEGNVFSCSVMHEALHNHYTQKSLSLSLGK.*
(SEQ ID NO: 70)

The second polypeptide chain of A17m0902-1708-151H7-A is set forth in SEQ ID NO: 52.
The first polypeptide chain of A17m0902-1708-151H7-B is set forth in SEQ ID NO: 68.

> The second polypeptide chain of A17m0902-1708-151H7-B

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW
GQGTQVTVSS<u>GGGGSGGGGS</u>DIQMTQSPSSLSASVGDRVTITCRASENIYSYLA
WYQQKPGKSPKLLIYNARTLAEGVPSRFSGSGSGTDFTLTISSLQPEDFATYYCQ
YHSGSPLPFGGGTKVEIK*RTVAAPSVFIFPPSDEQLKSGTASVVCLLNNFYPREAKVQ*
*WKVDNALQSGNSQESVTEQDSKDSTYSLSSTLTLSKADYEKHKVYACEVTHQGLSSPV*
*TKSFNRGEC (*SEQ ID NO: 71).

> The first polypeptide chain of A17m0902-1708-151H7-C

HVQLVESGGGLVQPGGSLRLSCVASASGFTYRPYCMAWFRQAPGKEREAVAGI
DIFGGTTYADSVKGRFTASRDNAGFSLYLQMNSLRAEDTAVYYCAAGDSPDGR
CPPLGQGLNYWGQGTMVTVSS<u>GGGGSGGGGS</u>EVQLVQSGAEVKKPGASVKVS
CKASGYTFTNYWMHWVRQAPGQGLEWMGRIDPDSTGSKYNEKFKTRVTMTR
DTSTSTVYMELSSLRSEDTAVYYCAREGAYGYYFDYWGQGTLVTVSS*ASTKGPS*
*VFPLAPCSRSTSESTAALGCLVKDYFPEPVTVSWNSGALTSGVHTFPAVLQSSGLYSLS*
*SVVTVPSSSLGTKTYTCNVDHKPSNTKVDKRVESKYGPPCPPCPAPEFLGGPSVFLFP*
*PKPKDTLMISRTPEVTCVVVDVSQEDPEVQFNWYVDGVEVHNAKTKPREEQFNSTY*
*RVVSVLTVLHQDWLNGKEYKCKVSNKGLPSSIEKTISKAKGQPREPQVYTLPPSQEE*
*MTKNQVSLTCLVKGFYPSDIAVEWESNGQPENNYKTTPPVLDSDGSFFLYSRLTVDK*
*SRWQEGNVFSCSVMHEALHNHYTQKSLSLSLGK*<u>GGGGSGGGGS</u>EVQLVESGGGLV
QPGGSLRLSCAASGLTFSDYSMSWYRQAPGKGRELVAIISGSGVITHYVDSVKG
RFTISRDNAKNTLYLQMRSLRAEDTAVYYCRAVSDWEDYWGQGTQVTVSS.
(SEQ ID NO: 72)

The second polypeptide chain of A17m0902-1708-151H7-C is set forth in SEQ ID NO: 52.
The first polypeptide chain of A17m0902-1708-151H7-D is set forth in SEQ ID NO: 68.

> The second polypeptide chain of A17m0902-1708-151H7-D

DIQMTQSPSSLSASVGDRVTITCRASENIYSYLAWYQQKPGKSPKLLIYNARTLA
EGVPSRFSGSGSGTDFTLTISSLQPEDFATYYCQYHSGSPLPFGGGTKVEIK*RTVAA*
*PSVFIFPPSDEQLKSGTASVVCLLNNFYPREAKVQWKVDNALQSGNSQESVTEQDSK*
*DSTYSLSSTLTLSKADYEKHKVYACEVTHQGLSSPVTKSFNRGEC*<u>GGGGSGGGGS</u>EV
QLVESGGGLVQPGGSLRLSCAASGLTFSDYSMSWYRQAPGKGRELVAIISGSGVI
THYVDSVKGRFTISRDNAKNTLYLQMRSLRAEDTAVYYCRAVSDWEDYWGQG
TQVTVSS(SEQ ID NO: 73).

After cell transfection, expression, and purification by conventional methods, the antibodies of interest can be obtained.

**Example 3-2. Binding Affinities of Anti-PD-1/PVRIG/TIGIT Trispecific Antibodies for Antigens**

ELISA assays were performed. TIGIT or PVRIG protein (his tag, Acrobiosystems, PVG-H52H4) was dissolved in PBS to 1 μg/mL, and the solution was added to a 96-well plate at 100 μL/well. The plate was coated with the antigen overnight at 4 °C. The plate was washed with PBST solution three times. PBST/1% BSA solution was added, and the plate was incubated at 37 °C for 1 h for blocking. After three washes with PBST solution, different concentrations of the trispecific antibodies (diluted with PBST/1% BSA solution) were added, and the plate was incubated at 37 °C for 1.5 h. The plate was washed with a PBST solution three times. 100 μL of HRP-conjugated anti-human IgG4 Fc secondary antibody (Thermo) solution was added, and the plate was incubated at 37 °C for 1 h. After three washes with PBST solution, TMB solution was added at 100 μL/well. After 5 min of reaction at room temperature, 50 μL of stop solution was added. OD450 values were measured using a microplate reader, and $EC_{50}$ was calculated.

1. Binding to TIGIT

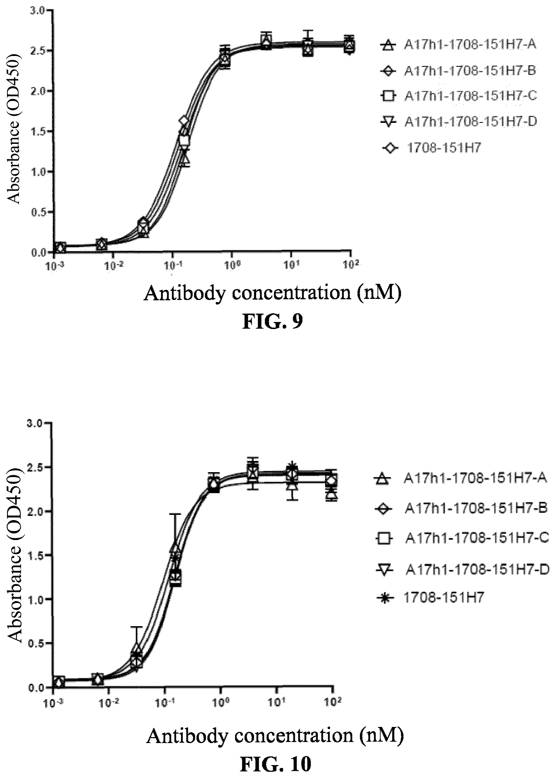

The A17h1-1708-151H7 trispecific antibodies of the four configurations can all form relatively strong binding to TIGIT, and their binding abilities are similar to each other and to that of the bispecific antibody 1708-151H7 to TIGIT. The results are shown in FIG. 9 and Table 37. Thus, all the trispecific antibody configurations do not affect their binding to TIGIT.

Table 37. The $EC_{50}$ values of trispecific antibodies binding to TIGIT antigen

| Antibody No. | $EC_{50}$ (nM) of binding to human TIGIT |
|---|---|
| 1708-151H7 | 0.1175 |
| A17h1-1708-151H7-A | 0.1859 |
| A17h1-1708-151H7-B | 0.1293 |
| A17h1-1708-151H7-C | 0.148 |
| A17h1-1708-151H7-D | 0.1624 |

2. Binding to PVRIG

The A17h1-1708-151H7 trispecific antibodies of the four configurations can all form relatively strong binding to PVRIG, and their binding abilities are similar to each other and to that of the bispecific antibody 1708-151H7 to PVRIG. See Table 38 and FIG. 10. Thus, all the trispecific antibody configurations do not affect their binding to PVRIG.

Table 38. The $EC_{50}$ values of trispecific antibodies binding to PVRIG antigen

| Antibody No. | $EC_{50}$ (nM) of binding to human PVRIG |
|---|---|
| 1708-151H7 | 0.1249 |
| A17h1-1708-151H7-A | 0.09519 |
| A17h1-1708-151H7-B | 0.1543 |
| A17h1-1708-151H7-C | 0.1596 |
| A17h1-1708-151H7-D | 0.1578 |

**Example 3-3. Optimization of PVRIG Sequences of Anti-PD-1/PVRIG/TIGIT Trispecific Antibodies**

In the design of the PD-1/PVRIG/TIGIT trispecific antibody A17m0902-1708-151H7-C, the first-position amino acid of the original PVRIG antibody sequence (151H7) is a histidine (the single-letter abbreviation for which is H). The histidine at the N-terminus of the trispecific antibody molecular sequence may undergo a pyridoxal phosphate modification and, following dephosphorylation, a pyridoxal modification in the actual antibody production process, and therefore the first-position histidine of the original PVRIG sequence needs to be mutated. Since glutamic acid (the single-letter abbreviation for which is E) and glutamine (the single-letter abbreviation for which is Q) are the most frequently occurring amino acids at the first positions of the nanobody sequences, H was mutated into E or Q. The mutated sequence of the PVRIG antibody 151H7 is as follows:

>151H7 H1E

**E**VQLVESGGGLVQPGGSLRLSCVASASGFTYRPYCMAWFRQAPGKEREAVAGID IFGGTTYADSVKGRFTASRDNAGFSLYLQMNSLRAEDTAVYYCAAGDSPDGRCP PLGQGLNYWGQGTMVTVSS(SEQ ID NO: 74).

>151H7 H1Q

**Q**VQLVESGGGLVQPGGSLRLSCVASASGFTYRPYCMAWFRQAPGKEREAVAGI DIFGGTTYADSVKGRFTASRDNAGFSLYLQMNSLRAEDTAVYYCAAGDSPDGR CPPLGQGLNYWGQGTMVTVSS(SEQ ID NO: 75).

The trispecific antibody molecule obtained by changing the first-position amino acid of the sequence of the PVRIG antibody (151H7) from H to E is named A17m0902-1708-151H7-01-C; the trispecific antibody molecule obtained by changing the first-position amino acid of the PVRIG (151H7) sequence from H to Q is named A17m0902-1708-151H7-02-C.

The molecules A17m0902-1708-151H7-01-C and A17m0902-1708-151H7-02-C are of type C-the anti-PVRIG antibodies are linked to the N-termini of the heavy chains of the anti-TIGIT antibody by linkers 1, and the anti-PD-1 antibodies are linked to the C-termini of the heavy chains of the anti-TIGIT antibody by linkers 2 (C of FIG. 8).

The polypeptide chain sequences of A17m0902-1708-151H7-01-C and A17m0902-1708-151H7-02-C are as follows:

> The first polypeptide chain of A17m0902-1708-151H7-01-C

EVQLVESGGGLVQPGGSLRLSCVASASGFTYRPYCMAWFRQAPGKEREAVAGID IFGGTTYADSVKGRFTASRDNAGFSLYLQMNSLRAEDTAVYYCAAGDSPDGRCP PLGQGLNYWGQGTMVTVSS<u>GGGGSGGGGS</u>EVQLVQSGAEVKKPGASVKVSC KASGYTFTNYWMHWVRQAPGQGLEWMGRIDPDSTGSKYNEKFKTRVTMTRD TSTSTVYMELSSLRSEDTAVYYCAREGAYGYYFDYWGQGTLVTVSS*ASTKGPSV FPLAPCSRSTSESTAALGCLVKDYFPEPVTVSWNSGALTSGVHTFPAVLQSSGLYSLSS VVTVPSSSLGTKTYTCNVDHKPSNTKVDKRVESKYGPPCPPCPAPEFLGGPSVFLFPP KPKDTLMISRTPEVTCVVVDVSQEDPEVQFNWYVDGVEVHNAKTKPREEQFNSTYR VVSVLTVLHQDWLNGKEYKCKVSNKGLPSSIEKTISKAKGQPREPQVYTLPPSQEEM TKNQVSLTCLVKGFYPSDIAVEWESNGQPENNYKTTPPVLDSDGSFFLYSRLTVDKSR WQEGNVFSCSVMHEALHNHYTQKSLSLSLGK*<u>GGGGSGGGGS</u>EVQLVESGGGLVQ

PGGSLRLSCAASGLTFSDYSMSWYRQAPGKGRELVAIISGSGVITHYVDSVKGRF TISRDNAKNTLYLQMRSLRAEDTAVYYCRAVSDWEDYWGQGTQVTVSS (SEQ ID NO: 76).

The second polypeptide chain of A17m0902-1708-151H7-01-C is set forth in SEQ ID NO: 52.

> The first polypeptide chain of A17m0902-1708-151H7-02-C

QVQLVESGGGLVQPGGSLRLSCVASASGFTYRPYCMAWFRQAPGKEREAVAGI DIFGGTTYADSVKGRFTASRDNAGFSLYLQMNSLRAEDTAVYYCAAGDSPDGR CPPLGQGLNYWGQGTMVTVSS<u>GGGGSGGGGS</u>EVQLVQSGAEVKKPGASVKVS CKASGYTFTNYWMHWVRQAPGQGLEWMGRIDPDSTGSKYNEKFKTRVTMTR DTSTSTVYMELSSLRSEDTAVYYCAREGAYGYYFDYWGQGTLVTVSS*ASTKGPS VFPLAPCSRSTSESTAALGCLVKDYFPEPVTVSWNSGALTSGVHTFPAVLQSSGLYSLS SVVTVPSSSLGTKTYTCNVDHKPSNTKVDKRVESKYGPPCPPCPAPEFLGGPSVFLFP PKPKDTLMISRTPEVTCVVVDVSQEDPEVQFNWYVDGVEVHNAKTKPREEQFNSTY RVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDK SRWQEGNVFSCSVMHEALHNHYTQKSLSLSLGK*<u>GGGGSGGGGS</u>EVQLVESGGGLV QPGGSLRLSCAASGLTFSDYSMSWYRQAPGKGRELVAIISGSGVITHYVDSVKG RFTISRDNAKNTLYLQMRSLRAEDTAVYYCRAVSDWEDYWGQGTQVTVSS (SEQ ID NO: 77).

The second polypeptide chain of A17m0902-1708-151H7-02-C is set forth in SEQ ID NO: 52.

**Example 3-4. Binding Affinities of Anti-PD-1/PVRIG/TIGIT Trispecific Antibodies with Optimized PVRIG Sequences for Antigens**

A stably transfected HEK293F cell strain expressing the human PVRIG gene, a stably transfected CHO-K1 cell strain expressing the human TIGIT gene, and Jurkat cells expressing human PD-1 were used. The cell strain was inoculated into a 96-well plate at $2 \times 10^5$ cells/well. The plate was centrifuged at 300 g for 5 min, and the supernatant was removed. 100 $\mu$L of test antibody was added, and the plate was incubated at 4 °C for 1 h. The plate was centrifuged, and the supernatant was removed. The plate was washed 3 times with 200 $\mu$L of wash buffer (PBS + 2% FBS), and 100 $\mu$L of an Alexa Fluor 488-labeled anti-human IgG secondary antibody (Invitrogen, A-11013) diluted at 1:500 was added. The plate was incubated at 4 °C for 1 h. The plate was centrifuged, and the supernatant was removed. The plate was washed 3 times with 200 $\mu$L of wash buffer (PBS + 2% FBS). The cells were resuspended in 100 $\mu$L of PBS and tested on a flow cytometer (BD FACS Calibur or BD FACS Canto_ II).

1. Binding to PVRIG

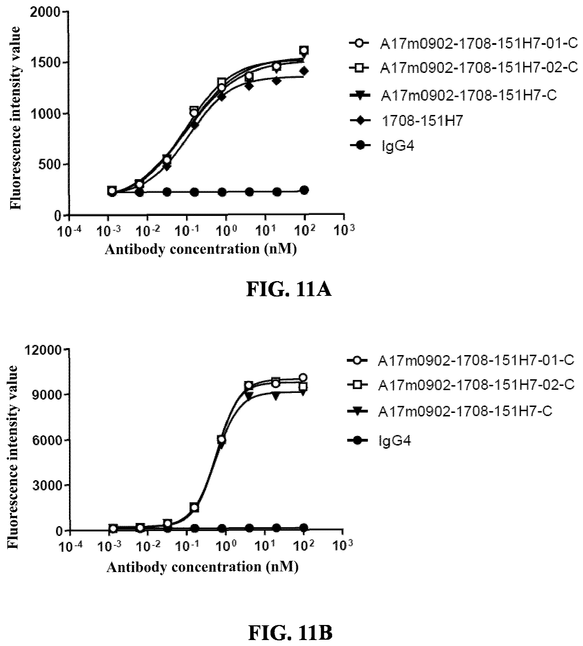

The two trispecific antibody molecules A17m0902-1708-151H7-01-C and A17m0902-1708-151H7-02-C obtained by mutation of the first-position amino acid of the PVRIG antibody showed relatively similar activity to the original trispecific antibody molecule A17m0902-1708-151H7-C and the original bispecific antibody molecule 1708-151H7 in binding to human PVRIG expressed on cell surfaces; their $EC_{50}$ values are similar, indicating that the mutation of the first-position amino acid of the PVRIG antibody did not affect the binding of the trispecific antibodies to PVRIG. The results are shown in Tables 39 and FIG. 11A.

Table 39. The $E_{MAX}$ and $EC_{50}$ of antibodies binding to cells expressing human PVRIG

| Antibody | EMAX | $EC_{50}$ (nM) |
| --- | --- | --- |
| A17m0902-1708-151H7-01-C | 1547 | 0.1048 |
| A17m0902-1708-151H7-02-C | 1521 | 0.09636 |
| A17m0902-1708-151H7-C | 1516 | 0.0944 |
| 1708-151H7 | 1357 | 0.1124 |

2. Binding to TIGIT

The two trispecific antibody molecules A17m0902-1708-151H7-01-C and A17m0902-1708-151H7-02-C showed relatively similar activity to the original trispecific antibody molecule A17m0902-1708-151H7-C in binding to human TIGIT expressed on cell surfaces; their EC50 values are similar, indicating that the mutation of the first-position amino acid of the PVRIG antibody did not affect the binding of the trispecific antibodies to TIGIT. The results are shown in Tables 40 and FIG. 11B.

Table 40. The $E_{MAX}$ and $EC_{50}$ of trispecific antibodies binding to cells expressing human TIGIT

| Antibody | EMAX | $EC_{50}$ (nM) |
| --- | --- | --- |
| A17m0902-1708-151H7-01-C | 9964 | 0.6008 |
| A17m0902-1708-151H7-02-C | 9747 | 0.586 |
| A17m0902-1708-151H7-C | 9105 | 0.5577 |

3. Binding to PD-1

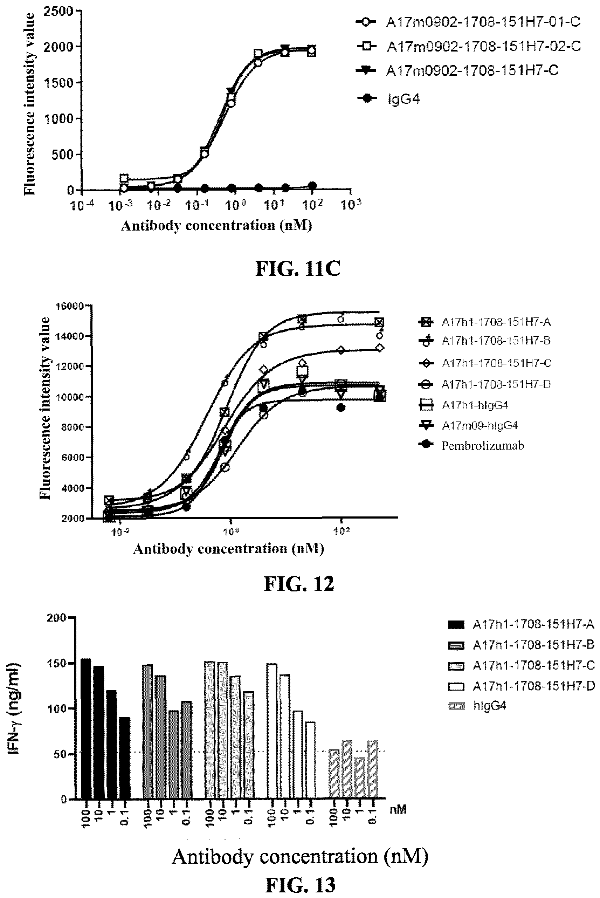

The two trispecific antibody molecules A17m0902-1708-151H7-01-C and A17m0902-1708-151H7-02-C showed similar activity to the original antibody A17m0902-1708-151H7-C in binding to human PD-1 expressed on cell surfaces; their EC50 values are similar, indicating that the mutation of the first-position amino acid of the PVRIG antibody did not affect the binding of the trispecific antibodies to PD-1. The results are shown in Tables 41 and FIG. 11C.

Table 41. The EMAX and $EC_{50}$ of trispecific antibodies binding to cells expressing human PD-1

| Antibody | EMAX | $EC_{50}$ (nM) |
|---|---|---|
| A17m0902-1708-151H7-01-C | 1956 | 0.5027 |
| A17m0902-1708-151H7-02-C | 1937 | 0.4806 |
| A17m0902-1708-151H7-C | 1974 | 0.4006 |

It can be concluded from the above binding activity results that the affinities of the two trispecific antibody molecules A17m0902-1708-151H7-01-C and A17m0902-1708-151H7-02-C obtained by mutation of the first-position amino acid of the PVRIG antibody for PVRIG, TIGIT, and PD-1 antigens expressed on cell surfaces are similar to those of the parent antibody A17m0902-1708-151H7-C; these mutations did not affect the binding of the trispecific antibodies to PVRIG, TIGIT, and PD-1.

## Example 3-5. PD-1/PD-L1 Reporter Gene System Test of Blocking of PD-1 by Anti-PD-1/PVRIG/TIGIT Trispecific Antibodies

A test was performed using the method of Example 1-4. As shown in FIG. 12, all the trispecific antibodies of the four configurations formed using A17h1 as a PD-1 end can block PD-1, and the reporter gene activity of the type B and type C trispecific antibodies is better.

## Example 3-6. MLR Tests of Activation of T Cells by Anti-PD-1/PVRIG/TIGIT Trispecific Antibodies

Cells from human subjects were subjected to a DC:T mix lymphocyte reaction experiment using the method of Example 1-6, and the results are shown in FIG. 13 and Table 42. The results show that the trispecific antibodies of 4 configurations can all effectively promote T cell activation, eliminate PD-1/PD-L1 inhibition, and promote cytokine secretion by T cells, and they all have relatively strong *in vitro* efficacy and activity.

Table 42. The MLR results for trispecific antibodies of the four configurations

| Antibody No. | Antibody concentration (nM) | | | |
|---|---|---|---|---|
| | 100 | 10 | 1 | 0.1 |
| A17h1-1708-151H7-A | 155.1 | 146.9 | 120.8 | 91.3 |
| A17h1-1708-151H7-B | 148.3 | 136.2 | 97.8 | 107.6 |
| A17h1-1708-151H7-C | 151.6 | 150.9 | 135.6 | 117.8 |
| A17h1-1708-151H7-D | 148.8 | 136.7 | 97.3 | 84.8 |
| hIgG4 | 54.2 | 64.5 | 45.9 | 64.5 |

## Example 3-7. NK92 Killing Test of Activation of NK Cells by Anti-PD-1/PVRIG/TIGIT Trispecific Antibodies

An NK cell killing experiment can be used to test the *in vitro* efficacy of the anti-PD-1/PVRIG/TIGIT trispecific antibodies. A cell killing experiment was conducted using NK92 cells as effector cells and K562 cells as target cells. First, NK92 cells were co-incubated with different concentrations of a candidate antibody at 37 °C for 30 min. Then, the NK92 cells pre-incubated with the antibody were mixed with CTV (Cat: C34557, Thermo)-labeled K562 cells at an effector-to-target ratio of 10:1, and the mixture was incubated at 37 °C in 1640 + 10% FBS + 10 ng/mL IL-2 for 4 h. After the mixture was centrifuged and the supernatant was removed, the cells were resuspended in a 10 μg/mL propidium iodide solution to stain dead cells. After 10 min of staining at room temperature, the proportion of dead cells (PI positive) among the CTV-labeled K562 cells was measured by FACS, and killing activity was calculated.

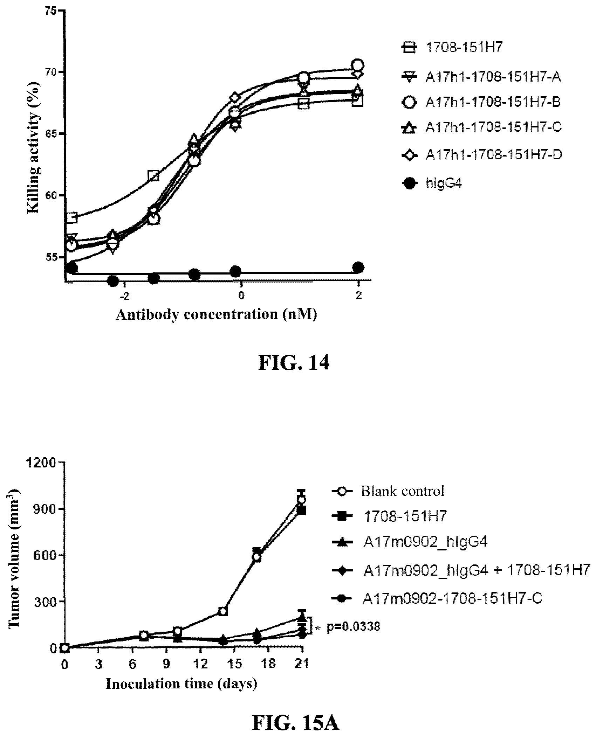

As shown in FIG. 14, the trispecific antibodies retained the anti-PVRIG/TIGIT bispecific antibodies' biological function of promoting NK cell killing, and the $IC_{50}$ values for all the configurations are similar. See Table 43.

Table 43. The NK cell killing function of trispecific antibodies of 4 configurations

| Antibody name | $EC_{50}$ (nM) |
|---|---|
| A17h1-1708-151H7 -A | 0.116 |

(continued)

| Antibody name | EC$_{50}$ (nM) |
|---|---|
| A17h1-1708-151H7 -B | 0.182 |
| A17h1-1708-151H7 -C | 0.07 |
| A17h1-1708-151H7 -D | 0.121 |
| 1708-151H7 | 0.066 |

**Example 3-8. Evaluation of Anti-Tumor Effect of Anti-PD-1/PVRIG/TIGIT Trispecific Antibody on Mouse Subcutaneous Xenograft Tumor Model of Human Melanoma A375 Mixed with Human PBMCs Responsive to PD-1 mAb**

In this example, the *in vivo* efficacy of the PD-1/PVRIG/TIGIT trispecific antibody on an *in vivo* tumor model of human melanoma A375 mixed with human PBMCs responsive to the PD-1 mAb was verified.

NCG mice, female, aged 4-8 weeks, weighing about 18-22 g (the mice were purchased from Jiangsu GemPharmatech Co., Ltd.). The mice were kept in an SPF animal house with an IVC constant-temperature and constant-pressure system.

Donor PBMCs responsive to the PD-1 mAb in the *in vivo* efficacy model were selected from frozen PBMCs and counted. The PBMCs were resuspended to a certain concentration in RPMI 1640 medium containing IL-2 and 10% FBS. The PBMCs were added to A375 cells which had been treated with Mitomycin C for 2 h. PBMCs were harvested after 5 days of co-culture with A375. A375 cells were cultured in DMEM medium containing 10% fetal bovine serum (FBS), and freshly digested A375 cells were harvested. Each mouse was inoculated with $5 \times 10^5$ PBMCs and $4 \times 10^6$ A375 cells; the inoculation volume was 0.2 mL/mouse (containing 50% Matrigel); the cells were inoculated subcutaneously into the right side of female NCG mice. The day of the inoculation was day 0, and on day 1, the mice were randomized into groups according to body weight and dosed.

Given that the molecular weight of A17m0902_hIgG4 is 76 kDa; the molecular weight of 1708-151H7 is 173.5 kDa; and the molecular weight of A17m0902-1708-151H7-C is 200 kDa, an equimolar administration regimen was designed. The administration regimen, the doses, and the route of administration are detailed in Table 44.

Table 44. The experimental groups of mice and the administration regimen

| Group | Drug or control | N | Dose (mg/kg) | Frequency of administration (route of administration) |
|---|---|---|---|---|
| Group 1 | Blank control | 7 | 10 | BIW*8 (i.p.) |
| Group 2 | 1708-151H7 | 7 | 11.5 | |
| Group 3 | A17m0902 hIgG4 | 7 | 5 | |
| Group 4 | 1708-151H7 + A17m0902-hIgG4 | 7 | 11.5+5 | |
| Group 5 | A17m0902-1708-151H7-C | 7 | 13.3 | |
| (Note: N: the number of animals used; i.p.: intraperitoneal injection; BIW*8: 2 times weekly, 8 injections in total) | | | | |

After the start of administration, the body weight and tumor volume of the mice were measured 2 times a week.

After day 21 of administration, the tumor growth inhibition rates (TGI) of the 1708-151H7 (11.5 mg/kg) treatment group, the A17m0902_hIgG4 (5 mg/kg) treatment group, the A17m0902-1708-151H7-C (13.3 mg/kg) treatment group, and the 1708-151H7 (11.5 mg/kg) + A17m0902_hIgG4 (5 mg/kg) treatment group were 7.05%, 79.91%, 91.63%, and 88.25%, respectively, as compared to the control group. The efficacy of the treatment group of the trispecific antibody A17m0902-1708-151H7-C is significantly better than that of the treatment group of the anti-PD-1 mAb A17m0902_hIgG4 and is better than the PVRIG/TIGIT bispecific antibody + PD-1 mAb combination group.

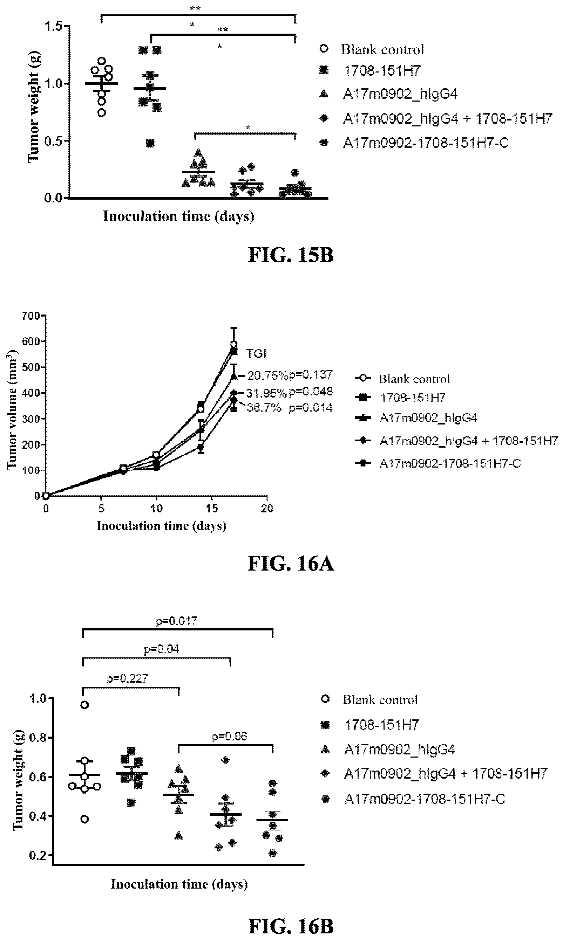

The experiment was ended after day 21 after administration. All the mice were euthanized, and their tumors were separated, weighed, and photographed. The tumor weight TGI was calculated, and it showed a similar trend to the tumor volume TGI. The results also show that the efficacy of the treatment group of the trispecific antibody A17m0902-1708-151H7-C is significantly better than that of the treatment group of the PD-1 mAb A17m0902_hIgG4 and is better than the PVRIG/TIGIT bispecific antibody + PD-1 mAb combination group. The experimental results are shown in Table 45 and FIGs. 15A and 15B.

Table 45. The anti-tumor effect of the anti-PD-1/PVRIG/TIGIT trispecific antibody in the human A375 tumor mouse model responsive to the PD-1 mAb

| Group | Drug | Tumor volume TGI (%) | Tumor weight TGI (%) |
|---|---|---|---|
| 1 | Blank control | / | / |
| 2 | 1708-151H7 | 7.05 | 3.84 |
| 3 | A17m0902 hIgG4 | 79.91 | 77.20 |
| 4 | A17m0902_hIgG4 + 1708-151H7 | 88.25 | 87.60 |
| 5 | A17m0902-1708-151H7-C | 91.63 | 91.50 |
| (Note: N: the number of animals used; i.p.: intraperitoneal injection; BIW: 2 times weekly) | | | |

**Example 3-9. Evaluation of Anti-Tumor Effect of Anti-PD-1/PVRIG/TIGIT Trispecific Antibody on Mouse Subcutaneous Xenograft Tumor Model of Human Melanoma A375 Mixed with Human PBMCs Non-Responsive to PD-1 mAb**

In this example, the *in vivo* efficacy of the anti-PD-1/PVRIG/TIGIT trispecific antibody on an *in vivo* tumor model of human melanoma A375 mixed with human PBMCs non-responsive to the anti-PD-1 mAb was verified.

NCG mice, female, aged 4-8 weeks, weighing about 18-22 g (purchased from Jiangsu GemPharmatech Co., Ltd.) were kept in an SPF animal house with an IVC constant-temperature and constant-pressure system.

Donor PBMCs non-responsive to the anti-PD-1 mAb in the *in vivo* efficacy model were selected from frozen PBMCs. The experimental protocol was the same as the method described in Example 3-8. The experimental groups of mice and the administration regimen are shown in Table 46.

Table 46. The experimental groups of mice and the administration regimen

| Group | Drug or control | N | Dose (mg/kg) | Frequency of administration (route of administration) |
|---|---|---|---|---|
| Group 1 | Blank control | 7 | 30 | BIW*8 (i.p.) |
| Group 2 | 1708-151H7 | 7 | 35.8 | |
| Group 3 | A17m0902_hIgG4 | 7 | 15.5 | |
| Group 4 | 1708-151H7 + A17m0902_hIgG4 | 7 | 35.8+15.5 | |
| Group 5 | A17m0902-1708-151H7 -C | 7 | 41.3 | |
| (Note: N: the number of animals used; i.p.: intraperitoneal injection; BIW*8: 2 times weekly, 8 injections in total) | | | | |

After the start of administration, the body weight and tumor volume of the mice were measured 2 times a week.

On day 17 after administration, the tumor growth inhibition rates (TGI%) of the experimental groups 1708-151H7, A17m0902_hIgG4, A17m0902-1708-151H7-C, and 1708-151H7 + A17m0902_hIgG4 were 4.53%, 20.75%, 36.70%, and 31.95%, respectively, as compared to the IgG control group, according to the measured average tumor volumes of the mice in these groups.

The experiment was ended after day 17 after administration. All the mice were euthanized, and their tumors were separated, weighed, and photographed. The tumor growth inhibition rates (tumor weight TGI%) of the experimental groups 1708-151H7, A17m0902_hIgG4, A17m0902-1708-151H7-C, and 1708-151H7 + A17m0902_hIgG4 were 0%, 16.8%, 38.2%, and 33.4%, respectively, as compared to the IgG control group, according to the measured tumor weights of the mice in these groups. The tumor weight TGI showed a similar trend to the tumor volume TGI. The experimental results are shown in Table 47 and FIGs. 16A and 16B.

Compared to the IgG control group, the A17m0902-1708-151H7-C treatment group and the 1708-151H7 + A17m0902_hIgG4 treatment group showed significant reductions in both tumor volume and weight (FIGs. 16A and 16B, P < 0.05), indicating significant tumor-inhibiting effects. During this experiment, all the mice did not exhibit significant weight losses or behavioral abnormalities, indicating that the mice well tolerated the test drugs under these experimental conditions.

Table 47. The anti-tumor effect of the anti-PD-1/PVRIG/TIGIT trispecific antibody in the human A375 tumor mouse model non-responsive to the PD-1 mAb

| Group | Drug | Tumor volume on day 0 (mm3) | Tumor volume on day 17 (mm3) (mean ± standard deviation) | Tumor volume TGI (%) | Tumor weight on day 17 (g) (mean ± standard deviation) | Tumor weight TGI (%) |
|---|---|---|---|---|---|---|
| 1 | Blank control | 0 | 588.39±62.69 | / | 0.611±0.068 | / |
| 2 | 1708-151H7 | 0 | 561.76±31.34 | 4.53 | 0.616±0.034 | 0 |
| 3 | A17m0902_hIg G4 | 0 | 466.28±44.23 | 20.75 | 0.508±0.043 | 16.8 |
| 4 | A17m0902_hIg G4+1708-151H 7 | 0 | 400.39±57.75 | 31.95* | 0.406±0.057 | 33.4 * |
| 5 | A17m0902-170 8-151H7-C | 0 | 372.45±40.80 | 36.7 * | 0.377±0.049 | 38.2 * |

(Note: *$P < 0.05$, **$P < 0.01$, and ***$P < 0.001$ are thought to indicate significant differences compared to the control group (hIgG1))

**Example 4. Buffer System, pH Value, and Excipient Screening for PD-1/PVRIG/TIGIT Trispecific Antibody Formulations**

The PD-1/PVRIG/TIGIT trispecific antibody used was the aforementioned A17m0902-1708-151H7-C, and the amino acid sequences of the first polypeptide chain and the second polypeptide chain thereof are SEQ ID NOs: 72 and 52, respectively. The following buffers were prepared, corresponding excipients and surfactants were added, and antibody formulations of the PD-1/PVRIG/TIGIT trispecific antibody at a concentration of 50 mg/mL were prepared for stability studies. The results of the experiment are shown in Table 48.

1) F1 10 mM citric acid-disodium citrate buffer pH 5.0, with 8% w/v sucrose and 0.04 mg/mL polysorbate 80 added;
2) F2 10 mM histidine-histidine hydrochloride buffer pH 5.5, with 8% w/v sucrose and 0.04 mg/mL polysorbate 80 added;
3) F3 10 mM histidine-acetic acid buffer pH 5.0, with 8% w/v sucrose and 0.04 mg/mL polysorbate 80 added;
4) F4 10 mM histidine-acetic acid buffer pH 5.5, with 8% w/v sucrose and 0.04 mg/mL polysorbate 80 added;
5) F5 10 mM histidine-acetic acid buffer pH 6.0, with 8% w/v sucrose and 0.04 mg/mL polysorbate 80 added;
6) F6 10 mM histidine-acetic acid buffer pH 5.0, with 8% w/v sucrose, 0.04 mg/mL polysorbate 80, and 50 mM arginine added;
7) F7 10 mM histidine-acetic acid buffer pH 5.5, with 8% w/v sucrose, 0.04 mg/mL polysorbate 80, and 50 mM arginine added.

Table 48. The screening results of the influencing factors (freeze-thaw and shaking)

| Detection item | Conditions | | F2 | F3 | F4 | F5 | F6 | F7 |
|---|---|---|---|---|---|---|---|---|
| Appearance | T0 | | Colorless, opalescent, no particles | Colorless, clear, no particles | Colorless, nearly clear, no particles | Colorless, opalescent, no particles | Colorless, significantly opalescent, no particles | Colorless, significantly opalescent, no particles |
| | 3 freeze-thaw cycles | | Colorless, opalescent, no particles | Colorless, clear, no particles | Colorless, nearly clear, no particles | Colorless, opalescent, no particles | Colorless, significantly opalescent, no particles | Colorless, significantly opalescent, no particles |
| | 5 freeze-thaw cycles | | Colorless, opalescent, no particles | Colorless, clear, no particles | Colorless, nearly clear, no particles | Colorless, opalescent, no particles | Colorless, significantly opalescent, no particles | Colorless, significantly opalescent, no particles |
| | Shaking-1 day | | Colorless, opalescent, no particles | Colorless, clear, no particles | Colorless, nearly clear, no particles | Colorless, opalescent, no particles | Colorless, significantly opalescent, no particles | Colorless, significantly opalescent, no particles |
| | Shaking-3 days | | Colorless, opalescent, no particles | Colorless, clear | Colorless, clear | Colorless, opalescent, no particles | Colorless, significantly opalescent, a small number of particles | Colorless, significantly opalescent, a small number of particles |
| SEC | TO | Aggregate% | 1.78 | 1.72 | 1.77 | 1.82 | 2.02 | 2.01 |
| | | Monomer% | 97.77 | 97.82 | 97.75 | 97.71 | 97.55 | 97.55 |
| | | Fragment% | 0.45 | 0.47 | 0.48 | 0.46 | 0.43 | 0.45 |
| | 5 freeze-thaw cycles | Aggregate% | 1.31 | 1.87 | 1.53 | 2.06 | 2.07 | 2.14 |
| | | Monomer% | 98.69 | 97.85 | 98.24 | 97.64 | 97.80 | 97.76 |
| | | Fragment% | 0.00 | 0.27 | 0.23 | 0.30 | 0.13 | 0.10 |
| | Shaking-3 days | Aggregate% | 1.89 | 1.95 | 2.09 | 2.20 | 2.31 | 2.41 |
| | | Monomer% | 97.80 | 97.64 | 97.53 | 97.42 | 97.35 | 97.26 |
| | | Fragment% | 0.31 | 0.40 | 0.38 | 0.38 | 0.33 | 0.34 |

(continued)

| Detection item | Conditions | | F2 | F3 | F4 | F5 | F6 | F7 |
|---|---|---|---|---|---|---|---|---|
| CEX | TO | Acidic peak% | 59.01 | 58.74 | 58.60 | 59.01 | 59.24 | 58.82 |
| | | Main peak% | 35.89 | 35.60 | 35.76 | 35.95 | 35.83 | 35.62 |
| | | Basic peak% | 5.10 | 5.66 | 5.64 | 5.03 | 4.93 | 5.56 |
| | 5 freeze-thaw cycles | Acidic peak% | 60.51 | 60.93 | 61.12 | 60.78 | 60.83 | 60.97 |
| | | Main peak% | 35.32 | 35.40 | 35.48 | 35.31 | 35.28 | 35.33 |
| | | Basic peak% | 4.17 | 3.68 | 3.40 | 3.91 | 3.89 | 3.70 |
| | Shaking-3 days | Acidic peak% | 61.42 | 59.14 | 59.12 | 60.52 | 59.02 | 59.25 |
| | | Main peak% | 33.67 | 35.67 | 35.21 | 33.97 | 35.68 | 35.70 |
| | | Basic peak% | 4.91 | 5.19 | 5.67 | 5.50 | 5.29 | 5.05 |
| NR-CE % | TO | | 97.24 | 97.26 | 97.25 | 97.38 | 97.28 | 97.47 |
| | 5 freeze-thaw cycles | | 96.42 | 96.61 | 96.08 | 96.55 | 96.11 | 96.63 |
| | Shaking-3 days | | 96.34 | 96.34 | 96.18 | 96.04 | 96.43 | 96.33 |

Table 49. Appearance results at 40 °C-1

| No. | Appearance | | |
|---|---|---|---|
| | T0 | 40 °C-2 W | 40 °C-4 W |
| F2 | Colorless, opalescent, no visible particles | Colorless, opalescent, no visible particles | Colorless, opalescent, no visible particles |
| F3 | Colorless, clear, no visible particles | Colorless, clear, no visible particles | Colorless, clear, no visible particles |
| F4 | Colorless, nearly clear, no visible particles | Colorless, clear, no visible particles | Colorless, clear, no visible particles |
| F5 | Colorless, opalescent, no visible particles | Colorless, opalescent, no visible particles | Colorless, opalescent, no visible particles |
| F6, | Colorless, significantly opalescent, no visible particles | Colorless, significantly opalescent, no visible particles | Colorless, significantly opalescent, no visible particles |
| F7 | Colorless, significantly opalescent, no visible particles | Colorless, significantly opalescent, no visible particles | Colorless, significantly opalescent, no visible particles |

Table 50. Purity screening results at 40 °C-2

| Detection item | Conditions | | F2 | F3 | F4 | F5 | F6 | F7 |
|---|---|---|---|---|---|---|---|---|
| SEC | TO | Aggregate% | 1.78 | 1.72 | 1.77 | 1.82 | 2.02 | 2.01 |
| | | Monomer% | 97.77 | 97.82 | 97.75 | 97.71 | 97.55 | 97.55 |
| | | Fragment% | 0.45 | 0.47 | 0.48 | 0.46 | 0.43 | 0.45 |
| | 40 °C-2 W | Aggregate% | 4.26 | 4.01 | 4.37 | 4.69 | 5.44 | 4.69 |
| | | Monomer% | 95.70 | 95.81 | 95.42 | 94.96 | 94.49 | 95.09 |
| | | Fragment% | 0.03 | 0.18 | 0.21 | 0.35 | 0.07 | 0.22 |
| | 40 °C-4 W | Aggregate% | 5.33 | 4.83 | 5.16 | 6.13 | 7.21 | 6.12 |
| | | Monomer% | 94.47 | 95.01 | 94.65 | 93.62 | 92.54 | 93.76 |
| | | Fragment% | 0.20 | 0.16 | 0.20 | 0.25 | 0.25 | 0.12 |
| CEX | TO | Acidic peak% | 59.01 | 58.74 | 58.60 | 59.01 | 59.24 | 58.82 |
| | | Main peak% | 35.89 | 35.60 | 35.76 | 35.95 | 35.83 | 35.62 |
| | | Basic peak% | 5.10 | 5.66 | 5.64 | 5.03 | 4.93 | 5.56 |
| | 40 °C-2 W | Acidic peak% | 60.42 | 59.52 | 60.17 | 61.09 | 56.30 | 59.00 |
| | | Main peak% | 29.76 | 27.71 | 28.75 | 28.86 | 27.18 | 28.03 |
| | | Basic peak% | 9.82 | 12.77 | 11.08 | 10.05 | 16.52 | 12.97 |
| | 40 °C-4 W | Acidic peak% | 58.04 | 58.16 | 58.93 | 60.26 | 52.90 | 57.37 |
| | | Main peak% | 30.59 | 29.68 | 29.85 | 29.46 | 29.35 | 30.36 |
| | | Basic peak% | 11.37 | 12.16 | 11.23 | 10.29 | 17.75 | 12.27 |
| NR-CE % | TO | | 97.24 | 97.26 | 97.25 | 97.38 | 97.28 | 97.47 |
| | 40 °C-2 W | | 93.84 | 94.39 | 94.30 | 93.00 | 94.07 | 93.75 |
| | 40 °C-4 W | | 91.81 | 91.95 | 92.02 | 91.13 | 90.11 | 91.09 |

Experimental results:

Formula F1 showed significant turbidity, and the protein was unstable in the citric acid system.

The results of the freeze-thaw and shaking experiments (Table 48) show that: after shaking for 3 days, formulas F3 (histidine-acetate buffer system pH 5.0) and F4 (histidine-acetate buffer system pH 5.5) were in a non-opalescent and clear state, and other formulas showed opalescence from T0; all formulas showed good stability in appearance after repeated freeze-thaw; formulas F2-F7 showed no significant differences in SEC, CEX, and NR-CE detection results after shaking or repeated freeze-thaw, and exhibited relatively good stability.

High-temperature experimental results (Tables 49-50):

The appearance results show that: after storage at 40 °C for 4 weeks, formulas F2-F7 showed no significant changes in appearance.

The SEC results show that: after storage at a high temperature of 40 °C for 4 weeks, all formulas showed reductions in SEC purity, wherein the reductions in F2 (histidine hydrochloride system pH 5.5), F3 (histidine acetate buffer system pH 5.0), and F4 (histidine acetate buffer system pH 5.5) were less than those in formulas F5-F7.

The CEX results show that: after storage at 40 °C for 4 weeks, the main peaks of CEX of the formulas tended to decrease, and the difference among the formulas was small.

The NR-CE results show that: after storage at 40 °C for 4 weeks, the main peak purity of the formulas remained above 90%, and the difference among the formulas was small. In summary, in terms of the appearance, SEC, CEX, and NR-CE detection results of the formulations, formulas F3-F4 were slightly better than other formulas and exhibited better stability. Therefore, F3 (histidine-acetic acid buffer, pH 5.0) and F4 (histidine-acetic acid buffer, pH 5.5) were selected for the next round of protein concentration screening.

**Example 5. Screening for Concentrations of PD-1/PVRIG/TIGIT Trispecific Antibody**

The PD-1/PVRIG/TIGIT trispecific antibody used was the aforementioned A17m0902-1708-151H7-C, and the amino acid sequences of the first polypeptide chain and the second polypeptide chain thereof are SEQ ID NOs: 72 and 52, respectively. The 10 mM histidine-acetic acid pH 5.0 and pH 5.5 buffer systems were selected to prepare formulations containing 8% w/v sucrose, 0.4 mg/mL polysorbate 80, and different antibody concentrations of PD-1/PVRIG/TIGIT trispecific antibody:

1) F8 10 mM histidine-acetic acid pH 5.0, 50 mg/mL antibody;
2) F9 10 mM histidine-acetic acid pH 5.5, 50 mg/mL antibody;
3) F10 10 mM histidine-acetic acid pH 5.0, 100 mg/mL antibody.

Stability studies were performed.

Table 51. Shaking and freeze-thaw results of antibody concentration screening

| Detection item | Conditions | F8 | F9 | F10 |
|---|---|---|---|---|
| Appearance | TO | Colorless, clear, no particles | Colorless, slightly opalescent, no particles | Colorless, clear, no particles |
| | 3 freeze-thaw cycles | Colorless, clear, no particles | Colorless, slightly opalescent, no particles | Colorless, clear, no particles |
| | 5 freeze-thaw cycles | Colorless, clear, no particles | Colorless, slightly opalescent, no particles | Colorless, clear, no particles |
| | Shaking-1 day | Colorless, clear, no particles | Colorless, slightly opalescent, no particles | Colorless, clear, no particles |
| | Shaking-3 days | Colorless, clear, no particles | Colorless, slightly opalescent, no particles | Colorless, clear, no particles |

(continued)

| Detection item | Conditions | | F8 | F9 | F10 |
|---|---|---|---|---|---|
| SEC | TO | Aggregate% | 0.77 | 0.88 | 0.93 |
| | | Monomer% | 98.90 | 98.82 | 98.75 |
| | | Fragment% | 0.33 | 0.31 | 0.31 |
| | 5 freeze-thaw cycles | Aggregate% | 0.66 | 0.72 | 0.79 |
| | | Monomer% | 99.02 | 98.96 | 98.90 |
| | | Fragment% | 0.32 | 0.32 | 0.31 |
| | Shaking-3 days | Aggregate% | 0.85 | 0.93 | 1.15 |
| | | Monomer% | 98.84 | 98.71 | 98.59 |
| | | Fragment% | 0.32 | 0.36 | 0.26 |
| CEX | TO | Acidic peak% | 62.02 | 61.96 | 61.70 |
| | | Main peak% | 35.96 | 35.95 | 35.93 |
| | | Basic peak% | 2.02 | 2.08 | 2.36 |
| | 5 freeze-thaw cycles | Acidic peak% | 61.33 | 61.29 | 61.15 |
| | | Main peak% | 35.76 | 35.81 | 35.84 |
| | | Basic peak% | 2.91 | 2.91 | 3.01 |
| | Shaking-3 days | Acidic peak% | 60.85 | 61.00 | 60.57 |
| | | Main peak% | 35.47 | 35.67 | 35.44 |
| | | Basic peak% | 3.68 | 3.33 | 3.98 |
| NR-CE main peak% | TO | | 96.62 | 97.12 | 96.73 |
| | 5 freeze-thaw cycles | | 97.01 | 97.16 | 96.83 |
| | Shaking-3 days | | 96.82 | 96.98 | 96.87 |

Table 52. High-temperature experimental results of antibody concentration screening

| Detection item | Conditions | F8 | F9 | F10 |
|---|---|---|---|---|
| Appearance | TO | Colorless, clear, no par-ticles | Colorless, slightly opa-lescent, no particles | Colorless, clear, no particles |
| | 2-8 °C-4 W | Colorless, clear, no par-ticles | Colorless, slightly opa-lescent, no particles | Colorless, clear, no particles |
| | 25 °C-4 W | / | Colorless, slightly opalescent, no particles | Colorless, clear, no particles |
| | 40 °C-2 W | Colorless, slightly opalescent, no particles | Colorless, slightly opalescent, no particles | Colorless, clear, no particles |
| | 40 °C-4 W | Pale yellow, slightly opalescent, no particles | Pale yellow, slightly opalescent, no particles | Pale yellow, clear, no parti-cles |

(continued)

| Detection item | Conditions | | F8 | F9 | F10 |
|---|---|---|---|---|---|
| SEC | TO | Aggregate% | 0.77 | 0.88 | 0.93 |
| | | Monomer% | 98.90 | 98.82 | 98.75 |
| | | Fragment% | 0.33 | 0.31 | 0.31 |
| | 2-8 °C-2 W | Aggregate% | 1.17 | 1.29 | 1.39 |
| | | Monomer% | 98.25 | 98.26 | 98.27 |
| | | Fragment% | 0.58 | 0.45 | 0.35 |
| | 25 °C-4 W | Aggregate% | 2.17 | 2.55 | 2.79 |
| | | Monomer% | 97.42 | 97.05 | 96.71 |
| | | Fragment% | 0.41 | 0.41 | 0.49 |
| | 40 °C-2 W | Aggregate% | 2.56 | 2.69 | 3.99 |
| | | Monomer% | 97.27 | 97.12 | 95.52 |
| | | Fragment% | 0.17 | 0.19 | 0.49 |
| | 40 °C-4 W | Aggregate% | 4.90 | 5.61 | 8.11 |
| | | Monomer% | 94.68 | 94.05 | 91.23 |
| | | Fragment% | 0.42 | 0.34 | 0.66 |
| CEX | TO | Acidic peak% | 62.02 | 61.96 | 61.70 |
| | | Main peak% | 35.96 | 35.95 | 35.93 |
| | | Basic peak% | 2.02 | 2.08 | 2.36 |
| | 2-8 °C-4 W | Acidic peak% | 61.52 | 62.10 | 60.83 |
| | | Main peak% | 32.58 | 33.19 | 32.08 |
| | | Basic peak% | 5.90 | 4.71 | 7.09 |
| | 25 °C-4 W | Acidic peak% | 61.32 | 61.64 | 59.54 |
| | | Main peak% | 31.04 | 31.19 | 30.32 |
| | | Basic peak% | 7.64 | 7.17 | 10.14 |
| | 40 °C-2 W | Acidic peak% | 59.90 | 60.92 | 57.50 |
| | | Main peak% | 29.74 | 30.36 | 28.42 |
| | | Basic peak% | 10.37 | 8.73 | 14.09 |
| | 40 °C-4 W | Acidic peak% | 62.22 | 64.06 | 58.44 |
| | | Main peak% | 22.97 | 23.48 | 21.74 |
| | | Basic peak% | 14.81 | 12.47 | 19.82 |
| NR-CE main peak% | TO | | 96.62 | 97.12 | 96.73 |
| | 2-8 °C-4 W | | 97.66 | 97.27 | 96.88 |
| | 25 °C-4 W | | 96.68 | 96.47 | 95.83 |
| | 40 °C-2 W | | 94.55 | 94.53 | 93.06 |
| | 40 °C-4 W | | 93.89 | 93.52 | 91.49 |

Note: "/" indicates that there were not enough samples to observe the appearance, and thus there were no results.

Experimental results

The freeze-thaw and shaking experimental results (Table 51) show that: after shaking or freeze-thaw, the formulas F8-F10 showed good stability in appearance, and there was no significant difference in the SEC, CEX, and NR-CE detection results for each formula, exhibiting relatively good stability.

The high-temperature experimental results (Table 52) show that:

The appearance results show that: after storage at 40 °C for 4 weeks, the formulas with different antibody concentrations all exhibited pale yellow color and no visible particles; after storage at 2-8 °C and 25 °C for 4 weeks, the appearance was unchanged as compared to T0.

The SEC results show that: after storage at 40 °C for 4 weeks, the monomer content of each formula was significantly reduced, and in the acetic acid-histidine buffer system, the monomer content and the polymer content of F8 (pH 5.0) were slightly better than those of F9 (pH 5.5) at the same antibody concentration (50 mg/mL); as the formula concentration increased, the polymer content increased, but the monomer content of F10 (pH 5.0, with the antibody concentration of 100 mg/mL) was also about 91.23%, and the long-term stability data at 2-8 °C were good, which could support the development of high-concentration formulations.

The CEX results show that: after storage at 40 °C for 4 weeks, the main peaks of formulas F8-F10 reduced significantly, and the trends of the formulas were consistent; in the acetic acid-histidine pH 5.0 buffer system, the main peaks of F8 (with the antibody concentration of 50 mg/mL) and F10 (100 mg/mL) were 22.97% and 21.74%, respectively, and the antibody concentration had little effect on the reduction of the main peak of CEX.

The NR-CE results show that: after storage at 40 °C for 4 weeks, formula F10 (100 mg/mL) showed the greatest reduction, but the main peak was also 91.49%; other formulas also showed slight reductions in purity, and the reductions were comparable. The main peaks of the three formulas at 2-8 °C and 25 °C after storage for 4 weeks were not greatly different from those at point T0.

Combining shaking, repeated freeze-thaw, and high-temperature experiments, it can be seen that the antibody has good stability in the acetic acid-histidine buffer system, and also has relatively good stability at pH 5.0 when the concentration is developed to 100 mg/mL.

**Example 6. Screening for pH Value and Concentration of PD-1/PVRIG/TIGIT Trispecific Antibody**

The PD-1/PVRIG/TIGIT trispecific antibody used was the aforementioned A17m0902-1708-151H7-01-C, and the amino acid sequences of the first polypeptide chain and the second polypeptide chain thereof are SEQ ID NOs: 76 and 52, respectively.

The 10 mM histidine-acetic acid pH 5.0 and pH 5.5 buffer systems were selected to prepare formulations containing 8% w/v sucrose, 0.8mg/mL polysorbate 80, and different antibody concentrations of PD-1/PVRIG/TIGIT trispecific antibody:

1) F11 10 mM histidine-acetic acid pH 5.0, 50 mg/mL antibody;

2) F12 10 mM histidine-acetic acid pH 5.0, 80 mg/mL antibody;

3) F13 10 mM histidine-acetic acid pH 5.0, 100 mg/mL antibody;

4) F14 10 mM histidine-acetic acid pH 5.5, 50 mg/mL antibody;

5) F15 10 mM histidine-acetic acid pH 5.5, 100 mg/mL antibody.

Stability studies, including high temperature (40 °C), 25 °C, 2-8 °C, freeze-thaw, and shaking, were performed.

Table 53. Shaking and freeze-thaw results of antibody pH value and concentration screening

| Detection item | Conditions | | F11 | F12 | F13 | F14 | F15 |
|---|---|---|---|---|---|---|---|
| Appearance | TO | | Pale yellow, slightly opalescent, no visible particles | Pale yellow, slightly opalescent, no visible particles | Pale yellow, slightly opalescent, no visible particles | Pale yellow, opalescent, no visible particles | Pale yellow, opalescent, no visible particles |
| | 3 freeze-thaw cycles | | Pale yellow, slightly opalescent, no visible particles | Pale yellow, slightly opalescent, no visible particles | Pale yellow, slightly opalescent, no visible particles | Pale yellow, opalescent, no visible particles | Pale yellow, opalescent, no visible particles |
| | 5 freeze-thaw cycles | | Pale yellow, slightly opalescent, no visible particles | Pale yellow, slightly opalescent, no visible particles | Pale yellow, slightly opalescent, no visible particles | Pale yellow, opalescent, no visible particles | Pale yellow, opalescent, no visible particles |
| | Shaking-1 day | | Pale yellow, slightly opalescent, no visible particles | Pale yellow, slightly opalescent, no visible particles | Pale yellow, slightly opalescent, no visible particles | Pale yellow, opalescent, no visible particles | Pale yellow, opalescent, no visible particles |
| | Shaking-3 days | | Pale yellow, slightly opalescent, no visible particles | Pale yellow, slightly opalescent, no visible particles | Pale yellow, slightly opalescent, no visible particles | Pale yellow, opalescent, no visible particles | Pale yellow, opalescent, no visible particles |
| SEC | TO | Aggregate% | 2.53 | 2.63 | 2.72 | 2.70 | 2.86 |
| | | Monomer% | 97.47 | 97.37 | 97.28 | 97.30 | 97.14 |
| | | Fragment% | 0.00 | 0.00 | 0.00 | 0.00 | 0.00 |
| | 5 freeze-thaw cycles | Aggregate% | 2.60 | 2.84 | 2.96 | 2.83 | 3.10 |
| | | Monomer% | 97.40 | 97.16 | 97.04 | 97.17 | 96.90 |
| | | Fragment% | 0.00 | 0.00 | 0.00 | 0.00 | 0.00 |
| | Shaking-3 days | Aggregate% | 2.98 | 3.48 | 3.70 | 3.28 | 3.84 |
| | | Monomer% | 97.02 | 96.52 | 96.30 | 96.72 | 96.16 |
| | | Fragment% | 0.00 | 0.00 | 0.00 | 0.00 | 0.00 |
| CEX | TO | Acidic peak% | 31.70 | 31.65 | 31.87 | 31.83 | 31.89 |
| | | Main peak% | 62.36 | 62.55 | 62.35 | 62.45 | 62.08 |
| | | Basic peak% | 5.93 | 5.80 | 5.78 | 5.72 | 6.03 |
| | 5 freeze-thaw cycles | Acidic peak% | 31.68 | 31.65 | 31.86 | 31.80 | 31.77 |
| | | Main peak% | 62.50 | 62.56 | 62.31 | 62.55 | 62.55 |
| | | Basic peak% | 5.81 | 5.78 | 5.82 | 5.66 | 5.68 |
| | Shaking-3 days | Acidic peak% | 29.63 | 29.55 | 29.57 | 30.01 | 29.82 |
| | | Main peak% | 62.85 | 63.06 | 62.80 | 63.61 | 63.63 |
| | | Basic peak% | 7.52 | 7.39 | 7.63 | 6.37 | 6.55 |

(continued)

| Detection item | Conditions | F11 | F12 | F13 | F14 | F15 |
|---|---|---|---|---|---|---|
| NR-CE main peak% | TO | 98.21 | 98.13 | 98.20 | 98.25 | 98.28 |
| | 5 freeze-thaw cycles | 96.54 | 97.31 | 96.76 | 97.41 | 96.78 |
| | Shaking-3 days | 97.44 | 96.49 | 96.52 | 96.55 | 96.30 |

Table 54. Shaking and freeze-thaw results of antibody pH value and concentration screening

| Detection item | Conditions | | F11 | F12 | F13 | F14 | F15 |
|---|---|---|---|---|---|---|---|
| Appearance | TO | | Pale yellow, slightly opalescent, no visible particles | Pale yellow, slightly opalescent, no visible particles | Pale yellow, slightly opalescent, no visible particles | Pale yellow, opalescent, no visible particles | Pale yellow, opalescent, no visible particles |
| | 2-8 °C-2 W | | Pale yellow, slightly opalescent, no visible particles | Pale yellow, slightly opalescent, no visible particles | Pale yellow, slightly opalescent, no visible particles | Pale yellow, opalescent, no visible particles | Pale yellow, opalescent, no visible particles |
| | 25 °C-2 W | | Pale yellow, slightly opalescent, no visible particles | Pale yellow, slightly opalescent, no visible particles | Pale yellow, slightly opalescent, no visible particles | Pale yellow, opalescent, no visible particles | Pale yellow, opalescent, no visible particles |
| | 40 °C-2 W | | Pale yellow, slightly opalescent, no visible particles | Pale yellow, with some increase in opalescence, no visible particles | Pale yellow, with some increase in opalescence, no visible particles | Pale yellow, with some increase in opalescence, no visible particles | Pale yellow, with some increase in opalescence, no visible particles |
| SEC | TO | Aggregate% | 2.53 | 2.63 | 2.72 | 2.70 | 2.86 |
| | | Monomer% | 97.47 | 97.37 | 97.28 | 97.30 | 97.14 |
| | | Fragment% | 0.00 | 0.00 | 0.00 | 0.00 | 0.00 |
| | 2-8 °C-2 W | Aggregate% | 2.63 | 2.84 | 2.95 | 2.83 | 3.08 |
| | | Monomer% | 97.37 | 97.16 | 97.05 | 97.17 | 96.92 |
| | | Fragment% | 0.00 | 0.00 | 0.00 | 0.00 | 0.00 |
| | 25 °C-2 W | Aggregate% | 2.90 | 3.37 | 3.54 | 3.17 | 3.74 |
| | | Monomer% | 97.10 | 96.63 | 96.46 | 96.83 | 96.26 |
| | | Fragment% | 0.00 | 0.00 | 0.00 | 0.00 | 0.00 |
| | 40 °C-2 W | Aggregate% | 3.80 | 4.86 | 5.33 | 4.20 | 5.45 |
| | | Monomer% | 96.03 | 94.97 | 94.48 | 95.66 | 94.41 |
| | | Fragment% | 0.17 | 0.17 | 0.19 | 0.13 | 0.13 |

(continued)

| Detection item | Conditions | | F11 | F12 | F13 | F14 | F15 |
|---|---|---|---|---|---|---|---|
| CEX | TO | Acidic peak% | 31.70 | 31.65 | 31.87 | 31.83 | 31.89 |
| | | Main peak% | 62.36 | 62.55 | 62.35 | 62.45 | 62.08 |
| | | Basic peak% | 5.93 | 5.80 | 5.78 | 5.72 | 6.03 |
| | 2-8 °C-2 W | Acidic peak% | 31.59 | 31.45 | 31.48 | 31.56 | 31.59 |
| | | Main peak% | 63.10 | 62.73 | 62.63 | 62.73 | 62.62 |
| | | Basic peak% | 5.30 | 5.82 | 5.89 | 5.70 | 5.79 |
| | 25°C-2W | Acidic peak% | 29.86 | 29.61 | 29.56 | 30.09 | 29.70 |
| | | Main peak% | 63.01 | 63.61 | 63.32 | 63.57 | 63.63 |
| | | Basic peak% | 7.13 | 6.79 | 7.12 | 6.34 | 6.67 |
| | 40 °C-2 W | Acidic peak% | 28.55 | 28.65 | 28.17 | 30.86 | 30.76 |
| | | Main peak% | 59.58 | 60.50 | 59.32 | 60.66 | 60.73 |
| | | Basic peak% | 11.87 | 10.85 | 12.51 | 8.48 | 8.51 |
| NR-CE main peak% | TO | | 98.21 | 98.13 | 98.20 | 98.25 | 98.28 |
| | 2-8 °C-2 W | | 97.44 | 97.46 | 96.60 | 97.23 | 97.64 |
| | 25 °C-2 W | | 96.78 | 96.40 | 96.95 | 96.69 | 96.73 |
| | 40 °C-2 W | | 95.26 | 94.41 | 94.76 | 94.40 | 93.78 |

**Experimental results:** The freeze-thaw and shaking experimental results (Table 53) show that: after shaking or freeze-thaw, the formulas F11-F15 showed good stability in appearance, and there was no significant difference in the SEC, CEX, and NR-CE detection results for each formula, exhibiting relatively good stability.

The high-temperature experimental results (Table 54) show that:

The appearance results show that: after storage at 40 °C for 2 weeks, all the formulas with different antibody concentrations exhibited no visible particles, and the opalescence of F12-F15 was enhanced; after storage at 2-8 °C and 25 °C for 2 weeks, each formula had no change in appearance as compared to T0.

The SEC results show that: after storage at 40 °C for 2 weeks, the monomer content of each formula was significantly reduced, and in the acetic acid-histidine buffer system, the monomer content and the polymer content of F11 (pH 5.0) were slightly better than those of F14 (pH 5.5) at the same antibody concentration (50 mg/mL); as the formula concentration increased, the polymer content increased.

The CEX results show that: after storage at 40 °C for 2 weeks, the main peaks of formulas F11-F15 reduced significantly, and the trends of the formulas were consistent; in the acetic acid-histidine pH 5.0 buffer system, the main peaks of F11 (with the antibody concentration of 50 mg/mL) and F13 (100 mg/mL) were 59.58% and 59.32%, respectively, and the antibody concentration had little effect on the reduction of the main peak of CEX.

The NR-CE results show that: after storage at 40 °C for 2 weeks, the main peaks of the formulas all reduced, and the reductions were comparable. The main peaks of the five formulas at 2-8 °C and 25 °C after storage for 2 weeks were not greatly different from those at point T0.

Combining the data from the shaking, repeated freeze-thaw, and high-temperature experiments for 2 W, it can be seen that the antibody has good stability in the acetic acid-histidine buffer system, and the purity data at pH 5.0 and pH 5.5 are within the acceptable range; as the concentration increases, the polymers increase slightly but within the acceptable range.

**Claims**

1. A pharmaceutical composition, comprising a PD-1/PVRIG/TIGIT-binding protein and a buffer, wherein the PD-1/PVRIG/TIGIT-binding protein comprises:

    a first antigen-binding domain that specifically binds to PD-1,
    a second antigen-binding domain that specifically binds to PVRIG, and
    a third antigen-binding domain that specifically binds to TIGIT,
    wherein the first antigen-binding domain comprises or is an immunoglobulin single variable domain comprising:

       a CDR1, a CDR2, and a CDR3 from the amino acid sequence set forth in any one of SEQ ID NOs: 27, 2, 8-10, 11-26, and 28-29; the CDR1, CDR2, and CDR3 are defined according to the Kabat, IMGT, Chothia, AbM, or Contact numbering scheme;
       preferably, the amino acid sequences of the CDR1, CDR2, and CDR3 of the immunoglobulin single variable domain are set forth in SEQ ID NOs: 3, 30, and 31, or SEQ ID NOs: 3, 32, and 35, respectively;
       the buffer is selected from the group consisting of a citrate buffer and a histidine salt buffer, preferably a histidine salt buffer, more preferably a histidine-hydrochloride buffer or a histidine-acetate buffer, and most preferably a histidine-histidine hydrochloride buffer or a histidine-acetic acid buffer.

2. The pharmaceutical composition according to claim 1, wherein the PD-1/PVRIG/TIGIT-binding protein is an anti-PD-1/PVRIG/TIGIT trispecific antibody.

3. The PD-1/PVRIG/TIGIT-binding protein according to claim 1 or 2, wherein:

    (B) the second antigen-binding domain comprises an immunoglobulin single variable domain, wherein the immunoglobulin single variable domain comprises: a CDR1, a CDR2, and a CDR3 from the amino acid sequence set forth in any one of SEQ ID NOs: 74-75, 38, and 46-50; the CDR1, CDR2, and CDR3 are defined according to the Kabat, IMGT, Chothia, AbM, or Contact numbering scheme;
    preferably, the amino acid sequences of the CDR1, CDR2, and CDR3 are set forth in SEQ ID NOs: 39, 40, and 41, respectively;
    and/or,
    (C) the third antigen-binding domain comprises a heavy chain variable region (VH) and a light chain variable region (VL), wherein the VH comprises a HCDR1, a HCDR2, and a HCDR3, and the amino acid sequences of the HCDR1, HCDR2, and HCDR3 are set forth in SEQ ID NOs: 57, 58, and 59, respectively; the VL comprises a LCDR1, a LCDR2, and a LCDR3, and the amino acid sequences of the LCDR1, LCDR2, and LCDR3 are set forth in SEQ ID NOs: 60, 61, and 62, respectively.

4. The PD-1/PVRIG/TIGIT-binding protein according to any one of claims 1 to 3, wherein:

(A) the first antigen-binding domain comprises the amino acid sequence set forth in any one of SEQ ID NOs: 27, 2, 8-10, 11-26, and 28-29 or an amino acid sequence having at least 90% sequence identity thereto, preferably SEQ ID NO: 27 or an amino acid sequence having at least 90% sequence identity thereto; and/or,
(B) the second antigen-binding domain comprises the amino acid sequence set forth in any one of SEQ ID NOs: 74-75, 38, and 46-50 or an amino acid sequence having at least 90% sequence identity thereto, preferably the amino acid sequence set forth in any one of SEQ ID NOs: 74 and 48 or an amino acid sequence having at least 90% sequence identity thereto; and/or,
(C) the third antigen-binding domain comprises a VH and a VL, wherein:

the VH comprises the amino acid sequence set forth in any one of SEQ ID NOs: 63-65 or an amino acid sequence having at least 90% sequence identity thereto, and the VL comprises the amino acid sequence set forth in SEQ ID NO: 67 or 66 or an amino acid sequence having at least 90% sequence identity thereto; preferably, the VH comprises SEQ ID NO: 63 or an amino acid sequence having at least 90% sequence identity thereto, and the VL comprises SEQ ID NO: 67 or an amino acid sequence having at least 90% sequence identity thereto.

5. The PD-1/PVRIG/TIGIT-binding protein according to claim 4, wherein the third antigen-binding domain comprises a heavy chain (HC) and a light chain (LC); preferably, the HC sequence is the amino acid sequence set forth in SEQ ID NO: 51 or has at least 90% sequence identity thereto, and the LC sequence is the amino acid sequence set forth in SEQ ID NO: 52 or has at least 90% sequence identity thereto.

6. The PD-1/PVRIG/TIGIT-binding protein according to any one of claims 1 to 5, comprising:

a first polypeptide chain, and
a second polypeptide chain,
wherein in order from N-terminus to C-terminus:

(I) the first polypeptide chain is represented by the following structure: [the first antigen-binding domain]-[linker 1]a-[the second antigen-binding domain]-[linker 2]b-[the VH of the third antigen-binding domain]-CH1-Fc, and
the second polypeptide chain is represented by the following structure: [the VL of the third antigen-binding domain]-Cκ; or
(II) the first polypeptide chain is represented by the following structure: [the second antigen-binding domain]-[linker 2]b-[the VH of the third antigen-binding domain]-CH1-Fc, and
the second polypeptide chain is represented by the following structure: [the first antigen-binding domain]-[linker 3]c-[the VL of the third antigen-binding domain]-Cκ; or
(III) the first polypeptide chain is represented by the following structure: [the second antigen-binding domain]-[linker 2]b-[the VH of the third antigen-binding domain]-CH1-Fc-[linker 4]d-[the first antigen-binding domain], and
the second polypeptide chain is represented by the following structure: [the VL of the third antigen-binding domain]-Cκ; or
(IV) the first polypeptide chain is represented by the following structure: [the second antigen-binding domain]-[linker 2]b-[the VH of the third antigen-binding domain]-CH1-Fc, and the second polypeptide chain is represented by the following structure: [the VL of the third antigen-binding domain]-Cκ-[linker 5]e-[the first antigen-binding domain];

wherein - represents a peptide bond;
the linker 1, linker 2, linker 3, linker 4, and linker 5 may be identical or different; a, b, c, d, and e are optionally independently 0 or 1;
preferably, the linker 1, linker 2, linker 3, linker 4, and linker 5 are independently $(G_xS)_y$, wherein x is selected from the group consisting of integers of 1-5, and y is selected from the group consisting of integers of 1-6; more preferably, the linker 1, linker 2, linker 3, linker 4, and linker 5 are independently linkers represented by any one of $(G_4S)_2$, $(G_4S)_3$, and $(G_4S)_4$.

7. The PD-1/PVRIG/TIGIT-binding protein according to any one of claims 1 to 6, comprising a first polypeptide chain and a second polypeptide chain, wherein the first polypeptide chain and the second polypeptide chain are selected from the group consisting of any one of the following groups:

the first and second polypeptide chains comprise the amino acid sequences set forth in SEQ ID NOs: 76 and 52, respectively;
the first and second polypeptide chains comprise the amino acid sequences set forth in SEQ ID NOs: 77 and 52, respectively;
the first and second polypeptide chains comprise the amino acid sequences set forth in SEQ ID NOs: 70 and 52, respectively;
the first and second polypeptide chains comprise the amino acid sequences set forth in SEQ ID NOs: 68 and 71, respectively;
the first and second polypeptide chains comprise the amino acid sequences set forth in SEQ ID NOs: 72 and 52, respectively;
the first and second polypeptide chains comprise the amino acid sequences set forth in SEQ ID NOs: 68 and 73, respectively;
and a combination of amino acid sequences having at least 90% sequence identity to the first and second polypeptide chains, respectively;
preferably, the PD-1/PVRIG/TIGIT-binding protein has two identical first polypeptide chains and two identical second polypeptide chains.

8. The pharmaceutical composition according to any one of claims 1 to 7, further comprising a surfactant, wherein preferably, the surfactant is a polysorbate; more preferably, the surfactant is polysorbate 80 or polysorbate 20; and most preferably, the surfactant is polysorbate 80.

9. The pharmaceutical composition according to any one of claims 1 to 8, further comprising a sugar, wherein preferably, the sugar is sucrose.

10. The pharmaceutical composition according to any one of claims 1 to 9, further comprising an additional amino acid or a pharmaceutically acceptable salt thereof, preferably arginine or a pharmaceutically acceptable salt thereof, and more preferably arginine-hydrochloric acid.

11. The pharmaceutical composition according to any one of claims 1 to 10, wherein the pharmaceutical composition has a pH of 3.5-7, preferably 4-6.5, more preferably 4.2-6.2, and most preferably 4.5-6.

12. The pharmaceutical composition according to any one of claims 1 to 11, wherein the PD-1/PVRIG/TIGIT-binding protein is at a concentration of 0.01 mg/mL-500 mg/mL, preferably 0.1 mg/mL-400 mg/mL, more preferably 0.5 mg/mL-200 mg/mL, and most preferably 1 mg/mL-150 mg/mL.

13. The pharmaceutical composition according to any one of claims 1 to 12, wherein the buffer is at a concentration of 0.1 mM-50 mM, preferably 0.5 mM-40 mM, more preferably 1 mM-30 mM, and most preferably 5 mM-20 mM.

14. The pharmaceutical composition according to any one of claims 8 to 13, wherein the surfactant is at a concentration of 0.01 mg/mL-10 mg/mL, preferably 0.05 mg/mL-5 mg/mL, more preferably 0.1 mg/mL-3 mg/mL, and most preferably 0.2 mg/mL-2 mg/mL.

15. The pharmaceutical composition according to any one of claims 9 to 14, wherein the sugar is at a concentration of 0.1% w/v-20% w/v, preferably 1% w/v-15% w/v, more preferably 3% w/v-12% w/v, and most preferably 5% w/v-10% w/v.

16. The pharmaceutical composition according to any one of claims 10 to 15, wherein the additional amino acid or the pharmaceutically acceptable salt thereof is at a concentration of 1 mM-200 mM, preferably 5 mM-170 mM, more preferably 10 mM-150 mM, and most preferably 15 mM-120 mM.

**17.** A pharmaceutical composition, comprising:

the PD-1/PVRIG/TIGIT-binding protein according to any one of claims 1 to 7;
a histidine salt buffer, preferably a histidine hydrochloride buffer or a histidine acetate buffer, and more preferably a histidine-histidine hydrochloride buffer or a histidine-acetic acid buffer;
polysorbate;
sucrose;
wherein optionally, the composition further comprises arginine or a pharmaceutically acceptable salt thereof.

**18.** The pharmaceutical composition according to claim 17, comprising any one of the following groups 1)-13):

1) 0.01 mg/mL-500 mg/mL said PD-1/PVRIG/TIGIT-binding protein according to any one of claims 1 to 7;

0.1 mM-50 mM histidine salt buffer, preferably a histidine hydrochloride buffer or a histidine acetate buffer, and more preferably a histidine-histidine hydrochloride buffer or a histidine-acetic acid buffer;
0.01 mg/mL-10 mg/mL polysorbate;
0.1% w/v-20% w/v sucrose;
wherein optionally, the pharmaceutical composition further comprises 1 mM-200 mM arginine or a pharmaceutically acceptable salt thereof;
and the pharmaceutical composition has a pH of 3.5-7;

2) 0.1 mg/mL-400 mg/mL said PD-1/PVRIG/TIGIT-binding protein according to any one of claims 1 to 7;

0.5 mM-40 mM histidine salt buffer, preferably a histidine hydrochloride buffer or a histidine acetate buffer, and more preferably a histidine-histidine hydrochloride buffer or a histidine-acetic acid buffer;
0.05 mg/mL-5 mg/mL polysorbate;
1% w/v-15% w/v sucrose;
wherein optionally, the pharmaceutical composition further comprises 5 mM-170 mM arginine or a pharmaceutically acceptable salt thereof;
and the pharmaceutical composition has a pH of 4-6.5;

3) 0.5 mg/mL-200 mg/mL said PD-1/PVRIG/TIGIT-binding protein according to any one of claims 1 to 7;

1 mM-30 mM histidine salt buffer, preferably a histidine hydrochloride buffer or a histidine acetate buffer, and more preferably a histidine-histidine hydrochloride buffer or a histidine-acetic acid buffer;
0.1 mg/mL-3 mg/mL polysorbate;
3% w/v-12% w/v sucrose;
wherein optionally, the pharmaceutical composition further comprises 10 mM-150 mM arginine or a pharmaceutically acceptable salt thereof;
and the pharmaceutical composition has a pH of 4.2-6.2;

4) 1 mg/mL-150 mg/mL said PD-1/PVRIG/TIGIT-binding protein according to any one of claims 1 to 7;

5 mM-20 mM histidine salt buffer, preferably a histidine hydrochloride buffer or a histidine acetate buffer, and more preferably a histidine-histidine hydrochloride buffer or a histidine-acetic acid buffer;
0.2 mg/mL-2 mg/mL polysorbate;
5% w/v-10% w/v sucrose;
wherein optionally, the pharmaceutical composition further comprises 15 mM-120 mM arginine or a pharmaceutically acceptable salt thereof;
and the pharmaceutical composition has a pH of 4.5-6;

5) 1 mg/mL-150 mg/mL said PD-1/PVRIG/TIGIT-binding protein according to any one of claims 1 to 7;

about 10 mM histidine hydrochloride buffer or histidine acetate buffer, preferably a histidine-histidine hydrochloride buffer or a histidine-acetic acid buffer;
0.2 mg/mL-1 mg/mL polysorbate 80;
about 8% w/v sucrose;
wherein the pharmaceutical composition has a pH of 4.5-6;

6) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL said PD-1/PVRIG/TIGIT-binding protein according to any one of claims 1 to 7;

    about 10 mM histidine hydrochloride buffer, preferably a histidine-histidine hydrochloride buffer;
    about 0.4 mg/mL polysorbate 80;
    about 8% w/v sucrose;
    wherein the pharmaceutical composition has a pH of about 5;

7) about 10 mg/mL, about 20 mg/mL, about 25 mg/mL, about 50 mg/mL, about 80 mg/mL, about 100 mg/mL, about 120 mg/mL, 130 mg/mL, or about 150 mg/mL said PD-1/PVRIG/TIGIT-binding protein according to any one of claims 1 to 7;

    about 10 mM histidine hydrochloride buffer, preferably a histidine-histidine hydrochloride buffer;
    about 0.4 mg/mL polysorbate 80;
    about 8% w/v sucrose;
    wherein the pharmaceutical composition has a pH of about 5.5;

8) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL said PD-1/PVRIG/TIGIT-binding protein according to any one of claims 1 to 7;

    about 10 mM histidine hydrochloride buffer, preferably a histidine-histidine hydrochloride buffer;
    about 0.8 mg/mL polysorbate 80;
    about 8% w/v sucrose;
    wherein the pharmaceutical composition has a pH of about 5;

9) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL said PD-1/PVRIG/TIGIT-binding protein according to any one of claims 1 to 7;

    about 10 mM histidine hydrochloride buffer, preferably a histidine-histidine hydrochloride buffer;
    about 0.8 mg/mL polysorbate 80;
    about 8% w/v sucrose;
    wherein the pharmaceutical composition has a pH of about 5.5;

10) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL said PD-1/PVRIG/TIGIT-binding protein according to any one of claims 1 to 7;

    about 10 mM histidine-acetic acid buffer;
    about 0.4 mg/mL polysorbate 80;
    about 8% w/v sucrose;
    wherein the pharmaceutical composition has a pH of about 5;

11) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL said PD-1/PVRIG/TIGIT-binding protein according to any one of claims 1 to 7;

    about 10 mM histidine-acetic acid buffer;
    about 0.4 mg/mL polysorbate 80;
    about 8% w/v sucrose;
    wherein the pharmaceutical composition has a pH of about 5.5;

12) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL said PD-1/PVRIG/TIGIT-binding protein according to any one of claims 1 to 7;

about 10 mM histidine-acetic acid buffer;
about 0.8 mg/mL polysorbate 80;
about 8% w/v sucrose;
wherein the pharmaceutical composition has a pH of about 5;

13) about 10 mg/mL, about 20 mg/mL, about 25 mg/mL, about 50 mg/mL, about 80 mg/mL, about 100 mg/mL, about 120 mg/mL, about 130 mg/mL, or about 150 mg/mL said PD-1/PVRIG/TIGIT-binding protein according to any one of claims 1 to 7;

about 10 mM histidine-acetic acid buffer;
about 0.8 mg/mL polysorbate 80;
about 8% w/v sucrose;
wherein the pharmaceutical composition has a pH of about 5.5.

19. A freeze-dried formulation, wherein the freeze-dried formulation is capable of forming the pharmaceutical composition according to any one of claims 1 to 18 upon reconstitution, or the freeze-dried formulation is obtained by lyophilizing the pharmaceutical composition according to any one of claims 1 to 18.

20. A reconstituted solution, wherein the reconstituted solution is prepared by reconstituting the freeze-dried formulation according to claim 19.

21. An article of manufacture, comprising a container containing the pharmaceutical composition according to any one of claims 1 to 18, the freeze-dried formulation according to claim 19, or the reconstituted solution according to claim 20.

22. Use of the pharmaceutical composition according to any one of claims 1 to 19, the freeze-dried formulation according to claim 19, or the reconstituted solution according to claim 20 in the manufacture of a medicament for treating cancer, wherein preferably, the cancer is selected from the group consisting of lung cancer, prostate cancer, breast cancer, head and neck cancer, esophageal cancer, gastric cancer, colon cancer, colorectal cancer, bladder cancer, cervical cancer, uterine cancer, ovarian cancer, liver cancer, melanoma, renal cancer, squamous cell carcinoma, and hematological cancer.

**FIG. 1A**

**FIG. 1B**

**FIG. 2**

**FIG. 3A**

**FIG. 3B**

**FIG. 4**

**FIG. 5**

**FIG. 6A**

**FIG. 6B**

**FIG. 7A**

**FIG. 7B**

Type A                Type B                Type C                Type D

PD-1-TIGIT-PVRIG-A    PD-1-TIGIT-PVRIG-B    PD-1-TIGIT-PVRIG-C    PD-1-TIGIT-PVRIG-D

TIGIT heavy chain          PD-1 nanobody

TIGIT light chain          PVRIG nanobody

FIG. 8

FIG. 9

FIG. 10

**FIG. 11A**

**FIG. 11B**

**FIG. 11C**

**FIG. 12**

**FIG. 13**

**FIG. 14**

**FIG. 15A**

**FIG. 15B**

**FIG. 16A**

**FIG. 16B**

## INTERNATIONAL SEARCH REPORT

| International application No. |
|---|
| **PCT/CN2024/081815** |

**A.    CLASSIFICATION OF SUBJECT MATTER**

A61K 39/395(2006.01)i;  C07K 16/28(2006.01)i;  C07K 19/00(2006.01)i;  C12N 15/13(2006.01)i;  C12N 15/62(2006.01)i;  A61P 35/00(2006.01)i

According to International Patent Classification (IPC) or to both national classification and IPC

**B.    FIELDS SEARCHED**

Minimum documentation searched (classification system followed by classification symbols)

IPC：  A61K C07K C12N A61P

Documentation searched other than minimum documentation to the extent that such documents are included in the fields searched

Electronic data base consulted during the international search (name of data base and, where practicable, search terms used)

CNABS; CNTXT; CJFD; ENTXTC; VEN; ENTXT; CNKI; 万方, WANFANG; 百度学术, BAIDU; 必应, Bing; ISI Web of Science; PubMed; NCBI; 中国专利生物序列检索系统, China Patents Biological Sequence Search System; STN; 上海迈晋生物医药科技有限公司, 三抗, 三特异性抗体, PD-1/PVRIG/TIGIT, PD-1, PDCD1, hPD-1, 程序性死亡1, A17, A17h1, A17m09, A17m0902, PVRIG, CD112R, 151H7, TIGIT, 1708, VHH, 重链抗体, 单域抗体, 纳米抗体, scFv, SHANGHAI MABGEN BIOTECH, Trispecific antibody, Programmed cell death-1, Poliovirus receptor-related Ig domain containing protein, T cell immunoglobulin and ITIM domain, Heavy chain antibody, Single domain antibody, Nanobody, 序列1-79, sequences 1-79

**C.    DOCUMENTS CONSIDERED TO BE RELEVANT**

| Category* | Citation of document, with indication, where appropriate, of the relevant passages | Relevant to claim No. |
|---|---|---|
| PX | WO 2023040945 A1 (JIANGSU HENGRUI PHARMACEUTICALS CO., LTD. et al.) 23 March 2023 (2023-03-23) <br> embodiments 1-3 | 1-22 |
| PA | WO 2023151661 A1 (JIANGSU HENGRUI PHARMACEUTICALS CO., LTD. et al.) 17 August 2023 (2023-08-17) <br> embodiments 1-3 | 1-22 |
| PA | WO 2023040940 A1 (JIANGSU HENGRUI PHARMACEUTICALS CO., LTD. et al.) 23 March 2023 (2023-03-23) <br> claims 1-28 | 1-22 |
| PA | WO 2023040935 A1 (JIANGSU HENGRUI PHARMACEUTICALS CO., LTD. et al.) 23 March 2023 (2023-03-23) <br> embodiments 16 and 18, and claims 1-16 | 1-22 |

☑ Further documents are listed in the continuation of Box C. ☑ See patent family annex.

| | |
|---|---|
| \*    Special categories of cited documents: <br> "A"  document defining the general state of the art which is not considered to be of particular relevance <br> "D"  document cited by the applicant in the international application <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search | Date of mailing of the international search report |
|---|---|
| **18 June 2024** | **24 June 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/081815**

### C.    DOCUMENTS CONSIDERED TO BE RELEVANT

| Category* | Citation of document, with indication, where appropriate, of the relevant passages | Relevant to claim No. |
|---|---|---|
| A | WO 2021180205 A1 (JIANGSU HENGRUI MEDICINE CO., LTD. et al.) 16 September 2021 (2021-09-16) embodiments 16 and 18, and table 28 | 1-22 |

Form PCT/ISA/210 (second sheet) (July 2022)

EP 4 681 729 A1

**INTERNATIONAL SEARCH REPORT**

International application No.

**PCT/CN2024/081815**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

| International application No. |
| --- |
| **PCT/CN2024/081815** |

| Patent document cited in search report | | | Publication date (day/month/year) | Patent family member(s) | | | Publication date (day/month/year) |
| --- | --- | --- | --- | --- | --- | --- | --- |
| WO | 2023040945 | A1 | 23 March 2023 | TW | 202328195 | A | 16 July 2023 |
| | | | | KR | 20240055080 | A | 26 April 2024 |
| | | | | CA | 3231320 | A1 | 23 March 2023 |
| | | | | CN | 117940453 | A | 26 April 2024 |
| WO | 2023151661 | A1 | 17 August 2023 | TW | 202342535 | A | 01 November 2023 |
| WO | 2023040940 | A1 | 23 March 2023 | TW | 202327649 | A | 16 July 2023 |
| WO | 2023040935 | A1 | 23 March 2023 | TW | 202317644 | A | 01 May 2023 |
| | | | | CA | 3231553 | A1 | 23 March 2023 |
| | | | | CN | 117858903 | A | 09 April 2024 |
| WO | 2021180205 | A1 | 16 September 2021 | TW | 202144417 | A | 01 December 2021 |
| | | | | US | 2024043530 | A1 | 08 February 2024 |
| | | | | EP | 4119162 | A1 | 18 January 2023 |
| | | | | EP | 4119162 | A4 | 23 August 2023 |
| | | | | JP | 2023516936 | A | 21 April 2023 |
| | | | | KR | 20220154140 | A | 21 November 2022 |
| | | | | CA | 3170025 | A1 | 16 September 2021 |
| | | | | CN | 115003333 | A | 02 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310250609
- WO 2023040945 A
- WO 2016134333 A
- WO 2016134335 A
- WO 2018033798 A
- WO 2018220446 A
- WO 2019079777 A
- WO 2019232484 A
- WO 2020018879 A
- WO 2021021837 A
- WO 2021097294 A
- WO 2021091605 A
- WO 2021113831 A
- WO 2019062832 A
- WO 2009126688 A
- WO 2014089113 A
- WO 2015009856 A
- WO 2015143343 A
- WO 2015174439 A
- WO 2016028656 A
- WO 2016106302 A
- WO 2017053748 A
- WO 2017030823 A
- US 20160176963 A
- US 20130251720 A
- WO 2009040562 A
- WO 2010035012 A
- WO 2005003169 A
- WO 2005003170 A
- WO 2005003171 A
- WO 9222583 A
- WO 05113605 A
- WO 9404678 A

**Non-patent literature cited in the description**

- **RILEY et al.** *Immunol. Rev*, 2009, vol. 29, 114-25
- **CHEN et al.** *Nat. Rev. Immunol.*, 2013, vol. 13, 227-42
- **DONG et al.** *Nat. Med.*, 1999, vol. 5, 1365-9
- **Y. ZHU et al.** *J Exp Med.*, 2016, vol. 213, 167-176
- **L. MARTINET et al.** *Cell Reports.*, 2015, vol. 11, 85-97
- **S. WHELAN et al.** *Cancer Immunol Res.*, 2019, vol. 7, 257-268
- **A. LEPLETIER et al.** *Clin Cancer Res.*, 2020, vol. 26, 3671-3681
- *J. biol. chem*, 1968, vol. 243, 3558
- **HOLLIGER** ; **HUDSON**. *Nature Biotech.*, 2005, vol. 23 (9), 1126-1136
- **ADAIR** ; **LAWSON**. *Drug Design Reviews-Online*, 2005, vol. 2 (3), 209-217
- **VERMA et al.** *Journal of Immunological Methods*, 1998, vol. 216, 165-181
- **ARAN F. LABRIJN et al.** *Nature Reviews Drug Discovery*, 2019, vol. 18, 585-608
- **CHEN S1 et al.** *J Immunol Res*, 11 February 2019, vol. 2019, 4516041
- **JOHNSON** ; **WU**. *Nucleic Acids Res.*, 2000, vol. 28, 214-8
- **CHOTHIA et al.** *J. Mol. Biol.*, 1986, vol. 196, 901-17
- **CHOTHIA et al.** *Nature*, 1989, vol. 342, 877-83
- **MARTIN et al.** *Proc Natl Acad Sci (USA)*, 1989, vol. 86, 9268-9272
- **SAMUDRALA et al.** Ab Initio Protein Structure Prediction Using a Combined Hierarchical Approach. *PROTEINS, Structure, Function and Genetics*, 1999, vol. 3, 194-198
- **MACCALLUM et al.** *J. Mol. Biol.*, 1996, vol. 5, 732-45
- **MAKABE et al.** *Journal of Biological Chemistry*, 2008, vol. 283, 1156-1166
- **HAMERS-CASTERMAN C ; ATARHOUCH T ; MUYLDERMANS S ; ROBINSON G ; HAMERS C ; SONGA EB ; BENDAHMAN N ; HAMERS R.** Naturally occurring antibodies devoid of light chains. *Nature*, 1993, vol. 363, 446-448
- **R. VAN DER LINDEN et al.** *Journal of Immunological Methods*, 2000, vol. 240, 185-195
- **LI et al.** *J Biol Chem.*, 2012, vol. 287, 13713-13721
- **DEFFAR et al.** *African Journal of Biotechnology*, 17 June 2009, vol. 8 (12), 2645-2652
- **KABAT, E. A. et al.** Sequences of Proteins of Immunological Interest. 1991
- **MARKS et al.** *Biotechnology*, 1992, vol. 10, 779-783

- **BARBAS et al.** *Proc. Nat. Acad. Sci, USA*, 1994, vol. 91, 3809-3813
- **SHIER et al.** *Gene*, 1995, vol. 169, 147-155
- **YELTON et al.** *Immunol.*, 1995, vol. 155, 1994-2004
- **JACKSON et al.** *J. Immunol.*, 1995, vol. 154 (7), 3310-9
- **HAWKINS et al.** *J. Mol. Biol.*, 1992, vol. 226 (3), 889896
- **KS JOHNSON** ; **RE HAWKINS**. Affinity maturation of antibodies using phage display. Oxford University Press, 1996
- **CACECI et al.** *Byte*, 1984, vol. 9, 340-362
- **WONG** ; **LOHMAN**. *Proc. Natl. Acad. Sci. USA*, 1993, vol. 90, 5428-5432